(19) Europäisches Patentamt
European Patent Office
Office européen des brevets

(11) **EP 2 800 476 B1**

(12) **EUROPEAN PATENT SPECIFICATION**

(45) Date of publication and mention
of the grant of the patent:
**24.04.2019 Bulletin 2019/17**

(21) Application number: **13733582.4**

(22) Date of filing: **04.01.2013**

(51) Int Cl.:
*A23K 40/20* (2016.01)          *A23K 20/168* (2016.01)
*A23K 20/189* (2016.01)          *A23K 10/14* (2016.01)
*A23K 50/75* (2016.01)          *A23K 50/80* (2016.01)
*A23K 50/60* (2016.01)          *A23K 40/25* (2016.01)
*A23K 50/30* (2016.01)          *C12N 9/16* (2006.01)
*A61K 38/46* (2006.01)

(86) International application number:
**PCT/CN2013/000004**

(87) International publication number:
**WO 2013/102430 (11.07.2013 Gazette 2013/28)**

(54) **METHOD OF FEEDING**

FÜTTERUNGSMETHODE

MODE D'ALIMENTATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.01.2012 GB 201200132
09.02.2012 US 201261596944 P
06.03.2012 GB 201203868
22.06.2012 GB 201211169
22.06.2012 GB 201211168
22.06.2012 GB 201211170
22.06.2012 GB 201211167
22.06.2012 GB 201211166**

(43) Date of publication of application:
**12.11.2014 Bulletin 2014/46**

(73) Proprietor: **DuPont Nutrition Biosciences ApS
1411 Copenhagen K (DK)**

(72) Inventors:
• **GILBERT, Ceinwen**
  **Rudloe, Corsham, Wiltshire SN13 0LY (GB)**
• **PLUMSTEAD, Peter**
  **Marlborough, Wiltshire SN84BT (GB)**
• **HAGEN, Klaus Schulze**
  **NL-3315 WS Dordrecht (NL)**
• **FROUEL, Stéphane**
  **F-75014 Paris (FR)**
• **PERON, Alexandre**
  **Singapore 117525 (CN)**

• **WU, Guangbing**
  **Guang Zhou, Guangdong 510060 (CN)**
• **YU, Shukun**
  **S-218 38 Bunke-flostrand (SE)**

(74) Representative: **D Young & Co LLP
120 Holborn
London EC1N 2DY (GB)**

(56) References cited:
**EP-A2- 2 675 287          WO-A2-2009/129489
WO-A2-2010/022532          WO-A2-2010/122532
WO-A2-2012/110778          CN-A- 101 063 113
CN-A- 101 381 709          CN-B- 101 381 709
US-A1- 2009 155 237**

• **Q.B. LEI ET AL: "Effect of reduced energy, protein
and entire substitution of inorganic phosphorus
by phytase on performance and bone
mineralisation of laying hens", BRITISH
POULTRY SCIENCE., vol. 52, no. 2, 1 April 2011
(2011-04-01), pages 202-213, XP055230237, GB
ISSN: 0007-1668, DOI:
10.1080/00071668.2011.562875**
• **J S UM ET AL: "Effects of Microbial Phytase
Supplementation on Egg Production, Eggshell
Quality, and Mineral Retention of Laying Hens
Fed Different Levels of Phosphorus", POULTRY
SCIENCE, vol. 78, no. 1, 1 January 1999
(1999-01-01), pages 75-79, XP055230354, US
ISSN: 0032-5791, DOI: 10.1093/ps/78.1.75**

SEQUENCE LISTING

<110> DuPont Nutrition Biosciences ApS

<120> Method of feeding

<130> P045968PCT

<150> GB 1200132.7
<151> 2012-01-05

<150> US 61/596,944
<151> 2012-02-09

<150> GB 1203868.3
<151> 2012-03-06

<150> GB 1211170.4
<151> 2012-06-22

<150> GB 1211168.8
<151> 2012-06-22

<150> GB 1211167.0
<151> 2012-06-22

<150> GB 1211169.6
<151> 2012-06-22

<150> GB 1211166.2
<151> 2012-06-22

<160> 6

<170> PatentIn version 3.5

<210> 1
<211> 413
<212> PRT
<213> Artificial Sequence

<220>
<223> Variant phytase BP17

<400> 1

```
Asn Asp Thr Pro Ala Ser Gly Tyr Gln Val Glu Lys Val Val Ile Leu
1               5                   10                  15


Ser Arg His Gly Val Arg Ala Pro Thr Lys Met Thr Gln Thr Met Arg
            20                  25                  30


Asp Val Thr Pro Asn Thr Trp Pro Glu Trp Pro Val Lys Leu Gly Tyr
            35                  40                  45


Ile Thr Pro Arg Gly Glu His Leu Ile Ser Leu Met Gly Gly Phe Tyr
        50                  55                  60


Arg Gln Lys Phe Gln Gln Gln Gly Ile Leu Ser Gln Gly Ser Cys Pro
65                  70                  75                  80
```

```
Thr Pro Asn Ser Ile Tyr Val Trp Thr Asp Val Ala Gln Arg Thr Leu
                85              90                      95

Lys Thr Gly Glu Ala Phe Leu Ala Gly Leu Ala Pro Gln Cys Gly Leu
            100             105                 110

Thr Ile His His Gln Gln Asn Leu Glu Lys Ala Asp Pro Leu Phe His
            115             120             125

Pro Val Lys Ala Gly Ile Cys Ser Met Asp Lys Thr Gln Val Gln Gln
    130             135             140

Ala Val Glu Lys Glu Ala Gln Thr Pro Ile Asp Asn Leu Asn Gln His
145             150             155             160

Tyr Ile Pro Ser Leu Ala Leu Met Asn Thr Thr Leu Asn Phe Ser Lys
            165             170             175

Ser Pro Trp Cys Gln Lys His Ser Ala Asp Lys Ser Cys Asp Leu Gly
            180             185             190

Leu Ser Met Pro Ser Lys Leu Ser Ile Lys Asp Asn Gly Asn Glu Val
        195             200             205

Ser Leu Asp Gly Ala Ile Gly Leu Ser Ser Thr Leu Ala Glu Ile Phe
    210             215             220

Leu Leu Glu Tyr Ala Gln Gly Met Pro Gln Ala Ala Trp Gly Asn Ile
225             230             235             240

His Ser Glu Gln Glu Trp Ala Leu Leu Leu Lys Leu His Asn Val Tyr
            245             250             255

Phe Asp Leu Met Glu Arg Thr Pro Tyr Ile Ala Arg His Lys Gly Thr
            260             265             270

Pro Leu Leu Gln Ala Ile Ser Asn Ala Leu Asn Pro Asn Ala Thr Glu
        275             280             285

Ser Lys Leu Pro Asp Ile Ser Pro Asp Asn Lys Ile Leu Phe Ile Ala
    290             295             300

Gly His Asp Thr Asn Ile Ala Asn Ile Ala Gly Met Leu Asn Met Arg
305             310             315             320

Trp Thr Leu Pro Gly Gln Pro Asp Asn Thr Pro Pro Gly Gly Ala Leu
            325             330             335
```

Val Phe Glu Arg Leu Ala Asp Lys Ser Gly Lys Gln Tyr Val Ser Val
340 345 350

Ser Met Val Tyr Gln Thr Leu Glu Gln Leu Arg Ser Gln Thr Pro Leu
355 360 365

Ser Leu Asn Gln Pro Ala Gly Ser Val Gln Leu Lys Ile Pro Gly Cys
370 375 380

Asn Asp Gln Thr Ala Glu Gly Tyr Cys Pro Leu Ser Thr Phe Thr Arg
385 390 395 400

Val Val Ser Gln Ser Val Glu Pro Gly Cys Gln Leu Gln
405 410

<210> 2
<211> 413
<212> PRT
<213> Artificial Sequence

<220>
<223> Variant phytase BP11

<400> 2

```
Asn Asp Thr Pro Ala Ser Gly Tyr Gln Val Glu Lys Val Val Ile Leu
1               5               10              15

Ser Arg His Gly Val Arg Ala Pro Thr Lys Met Thr Gln Thr Met Arg
            20              25              30

Asp Val Thr Pro Asn Thr Trp Pro Glu Trp Pro Val Lys Leu Gly Tyr
        35              40              45

Ile Thr Pro Arg Gly Glu His Leu Ile Ser Leu Met Gly Gly Phe Tyr
        50              55              60

Arg Gln Lys Phe Gln Gln Gln Gly Ile Leu Ser Gln Gly Ser Cys Pro
65              70              75              80

Thr Pro Asn Ser Ile Tyr Val Trp Thr Asp Val Asp Gln Arg Thr Leu
                85              90              95

Lys Thr Gly Glu Ala Phe Leu Ala Gly Leu Ala Pro Gln Cys Gly Leu
            100             105             110

Thr Ile His His Gln Gln Asn Leu Glu Lys Ala Asp Pro Leu Phe His
        115             120             125

Pro Val Lys Ala Gly Ile Cys Ser Met Asp Lys Thr Gln Val Gln Gln
        130             135             140
```

```
Ala Val Glu Lys Glu Ala Gln Thr Pro Ile Asp Asn Leu Asn Gln His
145             150             155             160

Tyr Ile Pro Ser Leu Ala Leu Met Asn Thr Thr Leu Asn Phe Ser Lys
                165             170             175

Ser Pro Trp Cys Gln Lys His Ser Ala Asp Lys Ser Cys Asp Leu Gly
                180             185             190

Leu Ser Met Pro Ser Lys Leu Ser Ile Lys Asp Asn Gly Asn Glu Val
            195             200             205

Ser Leu Asp Gly Ala Ile Gly Leu Ser Ser Thr Leu Ala Glu Ile Phe
        210             215             220

Leu Leu Glu Tyr Ala Gln Gly Met Pro Gln Ala Ala Trp Gly Asn Ile
225             230             235             240

His Ser Glu Gln Glu Trp Ala Leu Leu Leu Lys Leu His Asn Val Tyr
                245             250             255

Phe Asp Leu Met Glu Arg Thr Pro Tyr Ile Ala Arg His Lys Gly Thr
                260             265             270

Pro Leu Leu Gln Ala Ile Ser Asn Ala Leu Asn Pro Asn Ala Thr Glu
            275             280             285

Ser Lys Leu Pro Asp Ile Ser Pro Asp Asn Lys Ile Leu Phe Ile Ala
        290             295             300

Gly His Asp Thr Asn Ile Ala Asn Ile Ala Gly Met Leu Asn Met Arg
305             310             315             320

Trp Thr Leu Pro Gly Gln Pro Asp Asn Thr Pro Pro Gly Gly Ala Leu
            325             330             335

Val Phe Glu Arg Leu Ala Asp Lys Ser Gly Lys Gln Tyr Val Ser Val
            340             345             350

Ser Met Val Tyr Gln Thr Leu Glu Gln Leu Arg Ser Gln Thr Pro Leu
        355             360             365

Ser Leu Asn Gln Pro Ala Gly Ser Val Gln Leu Lys Ile Pro Gly Cys
        370             375             380

Asn Asp Gln Thr Ala Glu Gly Tyr Cys Pro Leu Ser Thr Phe Thr Arg
385             390             395             400
```

Val Val Ser Gln Ser Val Glu Pro Gly Cys Gln Leu Gln
              405                    410

<210> 3
<211> 413
<212> PRT
<213> Artificial Sequence

<220>
<223> Variant phytase BP111

<400> 3

```
Asn Asp Thr Pro Ala Ser Gly Tyr Gln Val Glu Lys Val Val Ile Leu
1               5                   10              15

Ser Arg His Gly Val Arg Ala Pro Thr Lys Met Thr Gln Thr Met Arg
            20              25              30

Asp Val Thr Pro Tyr Thr Trp Pro Glu Trp Pro Val Lys Leu Gly Tyr
            35              40              45

Ile Thr Pro Arg Gly Glu His Leu Ile Ser Leu Met Gly Gly Phe Tyr
        50              55              60

Arg Gln Lys Phe Gln Gln Gln Gly Ile Leu Pro Arg Gly Ser Cys Pro
65              70              75              80

Thr Pro Asn Ser Ile Tyr Val Trp Thr Asp Val Ala Gln Arg Thr Leu
            85              90              95

Lys Thr Gly Glu Ala Phe Leu Ala Gly Leu Ala Pro Gln Cys Gly Leu
            100             105             110

Thr Ile His His Gln Gln Asn Leu Glu Lys Ala Asp Pro Leu Phe His
        115             120             125

Pro Val Lys Ala Gly Ile Cys Ser Met Asp Lys Thr Gln Val Gln Gln
    130             135             140

Ala Val Glu Lys Glu Ala Gln Thr Pro Ile Asp Asn Leu Asn Gln Arg
145             150             155             160

Tyr Ile Pro Glu Leu Ala Leu Met Asn Thr Ile Leu Asn Phe Ser Lys
            165             170             175

Ser Pro Trp Cys Gln Lys His Ser Ala Asp Lys Pro Cys Asp Leu Ala
            180             185             190

Leu Ser Met Pro Ser Lys Leu Ser Ile Lys Asp Asn Gly Asn Glu Val
            195             200             205
```

```
Ser Leu Asp Gly Ala Ile Gly Leu Ser Ser Thr Leu Ala Glu Ile Phe
    210                 215             220

Leu Leu Glu Tyr Ala Gln Gly Met Pro Gln Val Ala Trp Gly Asn Ile
    225                 230             235                 240

His Ser Glu Gln Glu Trp Ala Leu Leu Leu Lys Leu His Asn Val Tyr
                245             250                 255

Phe Asp Leu Met Glu Arg Thr Pro Tyr Ile Ala Arg His Lys Gly Thr
            260             265             270

Pro Leu Leu Gln Ala Ile Ser Asn Ala Leu Asn Pro Asn Ala Thr Glu
    275                 280             285

Ser Lys Leu Pro Asp Ile Ser Pro Asp Asn Lys Ile Leu Phe Ile Ala
    290                 295             300

Gly His Asp Thr Asn Ile Ala Asn Ile Ala Gly Met Leu Asn Met Arg
305                 310             315                 320

Trp Thr Leu Pro Gly Gln Pro Asp Asn Thr Pro Pro Gly Gly Ala Leu
            325             330             335

Val Phe Glu Arg Leu Ala Asp Lys Ser Gly Lys Gln Tyr Val Ser Val
            340             345             350

Ser Met Val Tyr Gln Thr Leu Glu Gln Leu Arg Ser Gln Thr Pro Leu
    355                 360             365

Ser Leu Asn Gln Pro Pro Gly Ser Val Gln Leu Lys Ile Pro Gly Cys
    370             375             380

Asn Asp Gln Thr Ala Glu Gly Tyr Cys Pro Leu Ser Thr Phe Thr Arg
385             390             395             400

Val Val Ser Gln Ser Val Glu Pro Gly Cys Gln Leu Gln
            405             410
```

<210> 4
<211> 413
<212> PRT
<213> Buttiauxella sp.

<400> 4

```
Asn Asp Thr Pro Ala Ser Gly Tyr Gln Val Glu Lys Val Val Ile Leu
1               5                   10                  15

Ser Arg His Gly Val Arg Ala Pro Thr Lys Met Thr Gln Thr Met Arg
```

                          20                        25                        30

          Asp Val Thr Pro Asn Thr Trp Pro Glu Trp Pro Val Lys Leu Gly Tyr
                      35                  40                  45

          Ile Thr Pro Arg Gly Glu His Leu Ile Ser Leu Met Gly Gly Phe Tyr
                      50                  55                  60

          Arg Gln Lys Phe Gln Gln Gln Gly Ile Leu Ser Gln Gly Ser Cys Pro
          65                  70                  75                  80

          Thr Pro Asn Ser Ile Tyr Val Trp Ala Asp Val Asp Gln Arg Thr Leu
                              85                  90                  95

          Lys Thr Gly Glu Ala Phe Leu Ala Gly Leu Ala Pro Gln Cys Gly Leu
                          100                 105                 110

          Thr Ile His His Gln Gln Asn Leu Glu Lys Ala Asp Pro Leu Phe His
                      115                 120                 125

          Pro Val Lys Ala Gly Thr Cys Ser Met Asp Lys Thr Gln Val Gln Gln
                  130                 135                 140

          Ala Val Glu Lys Glu Ala Gln Thr Pro Ile Asp Asn Leu Asn Gln His
          145                 150                 155                 160

          Tyr Ile Pro Phe Leu Ala Leu Met Asn Thr Thr Leu Asn Phe Ser Thr
                          165                 170                 175

          Ser Ala Trp Cys Gln Lys His Ser Ala Asp Lys Ser Cys Asp Leu Gly
                      180                 185                 190

          Leu Ser Met Pro Ser Lys Leu Ser Ile Lys Asp Asn Gly Asn Lys Val
                      195                 200                 205

          Ala Leu Asp Gly Ala Ile Gly Leu Ser Ser Thr Leu Ala Glu Ile Phe
              210                 215                 220

          Leu Leu Glu Tyr Ala Gln Gly Met Pro Gln Ala Ala Trp Gly Asn Ile
          225                 230                 235                 240

          His Ser Glu Gln Glu Trp Ala Ser Leu Leu Lys Leu His Asn Val Gln
                          245                 250                 255

          Phe Asp Leu Met Ala Arg Thr Pro Tyr Ile Ala Arg His Asn Gly Thr
                      260                 265                 270

          Pro Leu Leu Gln Ala Ile Ser Asn Ala Leu Asn Pro Asn Ala Thr Glu
                  275                 280                 285

```
Ser Lys Leu Pro Asp Ile Ser Pro Asp Asn Lys Ile Leu Phe Ile Ala
    290             295             300

Gly His Asp Thr Asn Ile Ala Asn Ile Ala Gly Met Leu Asn Met Arg
305             310             315             320

Trp Thr Leu Pro Gly Gln Pro Asp Asn Thr Pro Pro Gly Gly Ala Leu
            325             330             335

Val Phe Glu Arg Leu Ala Asp Lys Ser Gly Lys Gln Tyr Val Ser Val
            340             345             350

Ser Met Val Tyr Gln Thr Leu Glu Gln Leu Arg Ser Gln Thr Pro Leu
            355             360             365

Ser Leu Asn Gln Pro Ala Gly Ser Val Gln Leu Lys Ile Pro Gly Cys
    370             375             380

Asn Asp Gln Thr Ala Glu Gly Tyr Cys Pro Leu Ser Thr Phe Thr Arg
385             390             395             400

Val Val Ser Gln Ser Val Glu Pro Gly Cys Gln Leu Gln
            405             410
```

<210> 5
<211> 33
<212> PRT
<213> Buttiauxella sp.

<400> 5

```
Met Thr Ile Ser Ala Phe Asn Arg Lys Lys Leu Thr Leu His Pro Gly
1               5               10              15

Leu Phe Val Ala Leu Ser Ala Ile Phe Ser Leu Gly Ser Thr Ala Tyr
            20              25              30

Ala
```

<210> 6
<211> 440
<212> PRT
<213> Artificial Sequence

<220>
<223> Phyzyme XP

<400> 6

```
Met Lys Ala Ile Leu Ile Pro Phe Leu Ser Leu Leu Ile Pro Leu Thr
1               5               10              15
```

```
Pro Gln Ser Ala Phe Ala Gln Ser Glu Pro Glu Leu Lys Leu Glu Ser
            20              25              30

Val Val Ile Val Ser Arg His Gly Val Arg Ala Pro Thr Lys Ala Thr
            35              40              45

Gln Leu Met Gln Asp Val Thr Pro Asp Ala Trp Pro Thr Trp Pro Val
            50              55              60

Lys Leu Gly Trp Leu Thr Pro Arg Gly Gly Glu Leu Ile Ala Tyr Leu
65              70              75              80

Gly His Tyr Gln Arg Gln Arg Leu Val Ala Asp Gly Leu Leu Ala Lys
                85              90              95

Lys Gly Cys Pro Gln Ser Gly Gln Val Ala Ile Ile Ala Asp Val Asp
            100             105             110

Glu Arg Thr Arg Lys Thr Gly Glu Ala Phe Ala Ala Gly Leu Ala Pro
            115             120             125

Asp Cys Ala Ile Thr Val His Thr Gln Ala Asp Thr Ser Ser Pro Asp
            130             135             140

Pro Leu Phe Asn Pro Leu Lys Thr Gly Val Cys Gln Leu Asp Asn Ala
145             150             155             160

Asn Val Thr Asp Ala Ile Leu Ser Arg Ala Gly Gly Ser Ile Ala Asp
                165             170             175

Phe Thr Gly His Arg Gln Thr Ala Phe Arg Glu Leu Glu Arg Val Leu
                180             185             190

Asn Phe Pro Gln Ser Asn Leu Cys Leu Lys Arg Glu Lys Gln Asp Glu
                195             200             205

Ser Cys Ser Leu Thr Gln Ala Leu Pro Ser Glu Leu Lys Val Ser Ala
            210             215             220

Asp Asn Val Ser Leu Thr Gly Ala Val Ser Leu Ala Ser Met Leu Thr
225             230             235             240

Glu Ile Phe Leu Leu Gln Gln Ala Gln Gly Met Pro Glu Pro Gly Trp
                245             250             255

Gly Arg Ile Thr Asp Ser His Gln Trp Asn Thr Leu Leu Ser Leu His
            260             265             270
```

```
Asn Ala Gln Phe Tyr Leu Leu Gln Arg Thr Pro Glu Val Ala Arg Ser
        275                 280                 285

Arg Ala Thr Pro Leu Leu Asp Leu Ile Met Ala Ala Leu Thr Pro His
        290                 295                 300

Pro Pro Gln Lys Gln Ala Tyr Gly Val Thr Leu Pro Thr Ser Val Leu
305                 310                 315                 320

Phe Ile Ala Gly His Asp Thr Asn Leu Ala Asn Leu Gly Gly Ala Leu
                325                 330                 335

Glu Leu Asn Trp Thr Leu Pro Gly Gln Pro Asp Asn Thr Pro Pro Gly
                340                 345                 350

Gly Glu Leu Val Phe Glu Arg Trp Arg Arg Leu Ser Asp Asn Ser Gln
        355                 360                 365

Trp Ile Gln Val Ser Leu Val Phe Gln Thr Leu Gln Gln Met Arg Asp
        370                 375                 380

Lys Thr Pro Leu Ser Leu Asn Thr Pro Pro Gly Glu Val Lys Leu Thr
385                 390                 395                 400

Leu Ala Gly Cys Glu Glu Arg Asn Ala Gln Gly Met Cys Ser Leu Ala
                405                 410                 415

Gly Phe Thr Gln Ile Val Asn Glu Ala Arg Ile Pro Ala Cys Ser Leu
                420                 425                 430

Arg Ser His His His His His His
        435                 440
```

**Description**

**TECHNICAL FIELD**

The invention relates to a method of feeding an animal, in particular a mono-gastric animal, non mono-gastric animal, ruminant animal or aquatic animal and is strictly defined by the claims.

In particular the invention relates to a method of feeding an animal with a feed, wherein said feed comprises a phytase, wherein said phytase results in an improvement in one or more of said animal's biophysical characteristics when compared to the equivalent use of *Peniophora lycii* phytase or an *E. coli* phytase.

The invention further relates to the use of phytase in feed and described are methods of producing such feed.

**BACKGROUND**

For optimum growth and improvement in other biophysical characteristics, animals must obtain adequate nutrition, minerals and vitamins. The use of active agents, such as enzymes, in foods and animal feed is common to assist in achieving this goal.

Enzymes are known to improve digestibility of food or animal feed, reduce anti-nutritional factors in food and animal feed, and improve animal productivity.

When compared with dry feed mixes, feed pellets have properties that are favored by the industry, such as improved feed quality, decreased pathogens, lower dust levels during manufacture, good handling, and more uniform ingredient dosing.

Some essential feed components are absent, present in reduced levels or present only in an inactive or inaccessible form in natural and manufactured feed.

Phytic acid (and its salt phytate) is the major phosphorous storage compound of most seeds and cereal grains (Zhou, J. R., and Erdman, J. W., Critical Reviews in Food Science and Nutrition., 1995, Vol. 35, Issue 6, pp 495-508). Phosphorus in the phytic acid or phytate form is poorly digested by animal such as monogastric animals. Phytic acid has a strong ability to chelate metal ions, especially zinc, calcium, copper and iron. This binding results in insoluble salts which are poorly absorbed from the gastrointestinal tract, which results in poor bioavailability of minerals (Zhou and Erdman, as above and Sebastian, S. et al., World's Poultry Science Journal., 1998, Vol.54, pp 27-47).

In addition to chelating metal ions it is well established that at acidic pH range phytate interacts with dietary proteins leading to the formation of phytate-protein aggregates and precipitates, which have decreased accessibility to proteases, thus possibly resulting in inefficient protein digestion (Knuckles, B. E. Effect of phytate and partially hydrolyzed phytate on in vitro protein digestibility.J. Food Sci. 1985, 50, 1080-1082; Konietzny, U.; Greiner, R. Phytic acid and nutritional impact. In Encyclopedia of Food Sciences and Nutrition,second edition; Caballero, B., Trugo, L., Finglas, P. M., Eds; Elsevier Science: Amsterdam, 2003: vol. 7, pp 4546-4563; Kies, A. K.; De Jonge, L. H. ; Kemme, P. A.; Jongbloed, A. W. Interaction between protein, phytate, and microbial phytase. In vitro studies. J. Agric. Food Chem. 2006, 54, 1753-1758; Vaintraub, I. A.; Bulmaga, V. P. Effect of phytate on the in vitro activity of digestive proteinases. J. Agric. Food Chem. 1991, 39, 859-861; Carnovale, E.; Lugaro, E.; Lombardi-Boccia, G. Phytic acid in Faba bean and pea: effect on protein availability. Cereal Chem. 1988, 65, 114-117; A. J. Cowieson, V. Ravindranand P. H. Selle Influence of Dietary Phytic Acid and Source of Microbial Phytase on Ileal Endogenous Amino Acid Flows in Broiler Chickens. Poult Sci. 2008. 87, 2287-2299) .

Phytase enzymes, such as e.g. the 6-phytase BP17 derived from *Buttiauxella sp.,* are added to foods and feeds to increase mineral, and in particular phosphate availability and thus increasing the nutritional value of the product. Phytase enzymes added to foods and feeds have also been shown to increase the amount of amino acids and energy digested and absorbed from the diet (Ravindran et al., J. Poult. Sci. 1999 Vol. 78 pp. 699-706). In addition, phytases added to animal feed may reduce phosphate pollution in the environment (Oh, B. C., et al., Appl. Microbiol Biotechnol, 2004, Vol. 63, pp 362-372). WO2010/122532 discloses a method of feeding an animal with a feed supplement comprising a phytase of Buttiauxella sp. (BP-17) in combination with a lipase for enhancing the growth rate of the animals.

EP0619369 and US5554399 disclose enzyme compositions comprising a phytase and an acid phosphatase and use of the enzyme composition in food, pelleted feed and fodder.

WO2004071218 discloses increasing the amount of minerals in a food. WO2004071218 discloses a preparation comprising an active phytase, a phytate and an essential cation. WO2004071218 discloses that the preparation may be added to any food or drink product for human consumption or to condiments such as curry powder.

The processing of the food or animal feed, for example under heat and high pressure, can denature the phytase and reduce its activity.

Phytases with improved heat stability are known.

Some animal such as monogastric animals are known to contain no or negligible amounts of endogenous phytase in the stomach and small intestine, and are therefore dependent on supplemental plant and/or microbial or fungal phytase for hydrolization of phytic acid in the proximal digestive tract (Pallauf, J. and Rimbach, G. Arch. Anim. Nutr., 1997, Vol.50, pp 301-319). Additional phytase may be added to the feed of animal such as monogastric animals.

The present invention seeks to overcome some of the problems associated with poor biophysical characteristics in animals, especially due to the inaccessibility or lack of nutrients, minerals and vitamins, especially phosphate, especially due to phytic acid/phytates.

The present invention further seeks to overcome the problems associated with the anti-nutritional properties of phytic acid leading to improved availability of nutrients, minerals, vitamins and energy and consequently improved bio-physical characteristics of animals.

The present invention will now be described. For ease of reference we have described elements of the present invention under one or more headings. It is to be noted that the teachings under each of the headings also applies to the teachings under the other headings.

## SUMMARY OF INVENTION

In one aspect, there is provided a method of feeding an animal, such as a mono-gastric animal, non mono-gastric animal, ruminant animal or aquatic animal, with a feed, wherein said feed comprises a phytase, wherein said phytase results in an improvement in one or more of said animal's biophysical characteristics when compared to the equivalent use of *Peniophora lycii* phytase or an *E. coli* phytase, wherein said improvements of said animal's biophysical characteristics comprises an increase in weight gain, wherein the increase in weight gain is at least 2% over a period of at least 21 days when the phytase is dosed at an amount of at least 500 FTU/kg feed, further wherein said phytase is or is obtainable from or is derivable from a *Buttiauxella* species.

In another aspect, there is provided a method as described above wherein said phytase results in an improvement in said animal's biophysical characteristics as a food source.

In a further aspect, there is provided a method as described above wherein the improvement in said animal's biophysical characteristics comprises a further improvement in one or more of body weight; mass; body fat percentage; height; body fat distribution; growth; growth rate; egg size; egg weight; egg mass; egg laying rate; mineral absorption; mineral excretion, mineral retention; bone density; bone strength; feed conversion rate; retention and/or a secretion of any one or more of copper, sodium, phosphorous, nitrogen and calcium; amino acid retention or absorption; mineralisation and bone mineralization.

There is provided, in a further aspect, uses of a feed comprising a phytase, wherein said phytase results in an improvement in one or more of said animal's biophysical characteristics when compared to the equivalent use of *Peniophora lycii* phytase or an *E. coli* phytase.

Phytase enzymes, such as BP17 (SEQ ID NO: 1) are added to animal feed to increase phosphate availability thus increasing the nutritional value of the product. The present description provides methods and uses for phytase in feed. In a preferred aspect the phytase used is BP17.

The improvements in nutrient, phosphate, and mineral availability obtained with added phytase are dependent amongst others on the biophysical and chemical characteristics of the diet, the type and age of the animal consuming the feed, and the source, type and concentration of the phytase used.

The improvement obtained in nutrient, phosphate, and mineral availability from adding phytase to feed increases with the concentration of active phytase present in the feed consumed by the animal. When comparing the improvement in one or more of said animal's biophysical characteristics compared to animals which have been fed or invoved in a feed method comprising phytase from different phytase sources such as *Peniophora lycii* phytase or an *E. coli* phytase, this must be done using equivalent concentrations of phytase, determined using the same analytical methodology, as well as using equivalent diets and animals.

In one particular aspect the present invention demonstrates that if BP17 or other phytases, measured as determined by the specified method of analysis, are added to feed, this results in an improvement in one or more of said animal's biophysical characteristics when compared to the equivalent use of *Peniophora lycii* phytase or an *E. coli* phytase.

## SOME ADVANTAGES

The present invention enables the biophysical characteristics of animals, such as mono-gastric animals, non mono-gastric animals, ruminant animals or aquatic animals, to undergo an improvement. In particular the invention enables many types of animals to undergo an improvement in their biophysical characteristics through a feed method. This method can be applied in the normal farmyard or other commercial animal rearing environment, and also in a small holding or domestic environment. No laboratory is necessary.

In particular the present invention relates to an improvement in said animal's biophysical characteristics as a food source. This enables more valuable animals to be reared, without great cost to the farmer or owner. In addition the welfare and health of animals fed according to the described methods may improve.

The present invention also demonstrates that efficacy in some animal species between different phytases can be highly variable. This may enable more effective phytases to be selected to feed to different species, thus benefitting the animals by increased nutrition and benefitting the owners by potentially reduced cost and waste.

**DESCRIPTION OF THE FIGURES**

The present invention will be described by reference to the following Figures:

Fig. 1 shows a graphical representation of the results of Ileal amino acid digestibility (%) in broiler chickens at 21 days of age (also shown in Table 1.2). This demonstrates that the BP17 phytase increases the digestibility of various amino acids in broiler chicken compared to the competitor's phytase product.

Fig. 2 shows a graphical representation of the mineral digestibility of a control compared to 3 phytase containing feeds.

Fig. 3 shows a graphical representation of the weight gain (BWG), feed intake, feed conversion ratio (FCR) and calorie conversion of chickens on various phytase feeds compared to the control.

Fig. 4 shows a graphical representation of the tract digestibility trial in chickens.

Fig. 5 shows a graphical representation of bone mineralisation of a control compared to chickens on a phytase feed.

Fig. 6 shows a graphical representation of bone mineralization of chickens on various phytase feeds compared to both positive and negative controls.

Fig. 7 shows a graphical representation of the weight gain (BWG), feed intake, feed conversion ratio (FCR) and calorie conversion of turkeys on various does of phytase in feeds compared to the control.

Fig. 8 shows a graphical representation of the tract digestibility trial and bone mineralisation measurements in turkeys.

Fig. 9 shows a graphical representation of the Cu tract digestibility trial in turkeys.

Fig. 10 shows a graphical representation of the effect of different doses of BP17 on layer chickens.

Fig. 11 shows a graphical representation of the effect of different doses of BP17 on on the daily retention (g/bird/day) of dietary nitrogen (NR), Calcium (Ca), Phosphorous (PR) and Sodium (NaR) in layer chickens.

Fig. 12 shows a graphical representation of the results for laying rate of laying hens fed with feed comprising varying levels of BP17, compared to both positive and negative controls.

Fig. 13 shows a graphical representation of the results for egg weight, feed intake and feed conversion ratio (FCR) of laying hens from 23 to 26 weeks of age fed with feed comprising varying levels of BP17, compared to both positive and negative controls.

Fig. 14 shows a graphical representation of the results of feeding various levels of BP17 phytase to weaned piglets.

Fig. 15 shows a graphical representation of the results of the comparison of BP17 phytase to *E. coli* phytase in broilers, using ileal phosphorus and amino acid digestibility, Phosphorus retention and calcium digestibility as measurements of performance..

Fig. 16 shows a graphical representation of the results of the comparison of BP17 phytase to *E. coli* phytase in broilers using apparent metabolisable energy as a measurement of performance.

Fig. 17 shows a graphical representation of the results of the comparison of BP17 phytase to *E. coli* phytase in broilers using nutrient digestibility as a measurement of performance.

Fig. 18 shows a graphical representation of the results of the comparison of BP17 phytase Phyzyme® XP.

Fig. 19 shows a graphical representation of the effect of different phytases on phosphate digestibility in Example 21.

Fig. 20 shows a graphical representation of the Water quality criteria throughout Example 23.

Fig. 21 shows a graphical representation of the determination of the pH Optima of BP17 phytase compared to 3 different commercial phytase sources.

Fig. 22 shows a graphical representation of the activity of BP17 phytase at different pH, as measured by the amount of phosphorus released from Na-phytate over varying pH.

Fig. 23 shows a graphical representation of the relative activity of BP17 phytase at releasing phosphorus from phytate at different pH, with activity of each phytase at pH 5.5 set at 100%.

## SEQUENCES

SEQ ID NO: 1= BP17, a variant phytase comprising 12 amino acid substitutions compared to the wild type (SEQ ID NO:4), lacking the signal sequence (SEQ ID NO:5).

SEQ ID NO: 2 = BP11, a variant phytase comprising 11 amino acid substitutions compared to the wild type (SEQ ID NO: 4), lacking the signal sequence (SEQ ID NO: 5).

SEQ ID NO: 3 = BP111, a variant phytase comprising 21 amino acid substitutions compared to the wild type (SEQ ID NO:4), lacking the signal sequence (SEQ ID NO: 5).

SEQ ID NO: 4 = wild type phytase encoded by *Buttiauxella sp.* strain *P* 1-29 deposited under accession number NCIMB 41248, lacking the signal sequence (SEQ ID NO: 5).

SEQ ID NO: 5 = signal sequence of wild-type *Buttiauxella sp.* strain *P* 1-29 deposited under accession number NCIMB 41248 (SEQ ID NO: 4).

SEQ ID NO:6 = Phyzyme XP.

## DETAILED DESCRIPTION

The present inventors have surprisingly found a method of feeding an animal, such as a mono-gastric animal, non mono-gastric animal, ruminant animal, or aquatic animal, with a feed, wherein said feed comprises a phytase, wherein said phytase results in an improvement in one or more of said animal's biophysical characteristics when compared to the equivalent use of *Peniophora lycii* phytase or an *E. coli* phytase.

More specifically, the improvement in said animal's biophysical characteristics is an increase in weight gain and one or more of: body weight; mass; height; growth; growth rate; egg size; egg weight; egg mass; egg laying rate; mineral absorption; mineral retention; bone density; bone strength; feed conversion rate; retention of any one or more of copper, sodium, phosphorous, nitrogen and calcium; amino acid retention or absorption; mineralization and bone mineralization.

More specifically, the improvement in said animal's biophysical characteristics is a decrease in any one or more of: body fat percentage; body fat distribution; mineral excretion, a secretion of any one or more of copper, sodium, phosphorous, nitrogen and calcium.

More specifically, the improvement in said animal's biophysical characteristics is (a) an increase in weight gain and one or more of: body weight; mass; height; growth; growth rate; egg size; egg weight; egg mass; egg laying rate; mineral absorption; mineral retention; bone density; bone strength; feed conversion rate; retention of any one or more of copper, sodium, phosphorous, nitrogen and calcium; amino acid retention or absorption; mineralization and bone mineralization and/or (b) an decrease in any one or more of: body fat percentage; body fat distribution; mineral excretion, a secretion of any one or more of copper, sodium, phosphorous, nitrogen and calcium.

Further, the present inventors surprisingly found that said phytase in particular results in an improvement in said animal's biophysical characteristics as a food source.

### General Definitions

As used herein, the term "phytase" refers to an enzyme (i.e. a polypeptide having phytase activity) that catalyzes the hydrolysis of esters of phosphoric acid, including phytate and phytic acid, and releases inorganic phosphate.

Further aspects regarding the term "phytase" are presented herein in a later section.

As used herein, the term "biophysical characteristics" encompasses all measures of the growth, maturity and health of an animal. The term "biophysical characteristics" may be synonymous with digestive-physiological and/or performance characteristics. Biophysical characteristics may include but are not limited to: body weight; weight gain; mass; body fat percentage; height; body fat distribution; growth; growth rate; egg size; egg weight; egg mass; egg laying rate; mineral absorption; mineral excretion, mineral retention; bone density; bone strength; feed conversion rate (FCR); retention and/or a secretion of any one or more of copper, sodium, phosphorous, nitrogen and calcium; amino acid retention or absorption; mineralisation and bone mineralization. The biophysical characteristics are further discussed below.

An animal's biophysical characteristic "as a food source" refers to biophysical characteristics which increase the value of the animal as a food source, particularly a human food source. These biophysical characteristics may improve the health, weight, size, fat content, rate of growth, time to reach maturity, taste and other characteristics related to ease of use in food production. In addition, these biophysical characteristics may include but are not limited to the following:- maturity and health of an animal. Biophysical characteristics may include but are not limited to: body weight; weight gain; mass; body fat percentage; height; body fat distribution; growth; growth rate; egg size; egg weight; egg mass; egg laying rate; mineral absorption; mineral excretion; mineral retention; bone density; bone strength; feed conversion rate; retention and/or a secretion of any one or more of copper, sodium, phosphorous, nitrogen and calcium; amino acid retention or absorption; mineralisation and bone mineralization. Such an animal with these improved biophysical characteristics may have higher value as food, a higher market value, better taste, more nutritional value and be used in different cooking methods.

A "food source" may encompass any aspect of an animal such as meat, protein, fat, coat or fur, feathers, milk or eggs.

An "improvement" refers to an improvement better than that shown when compared to the equivalent use of other feeds. These other feeds may not comprise a phytase or they may contain another phytase, such as *Peniophora lycii* phytase or an *E. coli* phytase. Such an improvement may comprise an increase or decrease in a characteristic. For example an increase or decrease in weight, and increase or decrease in mineral retention.

The improvement in biophysical characteristics may be at least 0.5%, at least 1%, at least 2%, at least 3%, at least 5%, at least 10%, at least 12%, at least 15%, at least 20% or at least 30%. In some embodiments the improvement may be at least 50% or at least 100% or at least 150%.

The percentage improvement in biophysical characteristics may be in one aspect in comparison to the use of a feed not comprising a phytase. In another aspect the percentage improvement in biophysical characteristics may be in comparison to another phytase, especially *Peniophora lycii* phytase and/or an *E. coli* phytase.

The term "weight gain" includes body weight gain, weight gain of any meat cuts, weight gain of legs and limbs, an increase in protein weight, an increase in fat weight and an increase in bone weight.

The increase in weight gain is at least 2%, maybe at least 3%, at least 5%, at least 10%, at least 12%, at least 15%, at least 20% or at least 30%. In some embodiments the improvement may be at least 50% or at least 100%.

The increase in weight gain may be in respect to a control in which the feed used does not comprise a phytase. In another aspect the increase in weight gain may be with respect to the use of a feed comprising another phytase, especially *Peniophora lycii* phytase and/or an *E. coli* phytase.

As used herein, the term "thermostable" refers to the ability of an enzyme to retain activity after exposure to elevated temperatures. A thermostable enzyme has an increased resistance against structural or functional breakdown at elevated temperatures. The phrase "increased thermostability" refers to an enzyme which is more thermostable, and in some embodiments more thermostable when compared to a wild-type enzyme such as SEQ ID NO: 4.

As used herein, the term "$T_m$" refers to the melting temperature of an enzyme such as phytase.

The term "mineralization" or "mineralisation" encompasses mineral deposition or release of minerals. Minerals may be deposited or released from the body of the animal. Minerals may be released from the feed. Minerals may include any minerals necessary in an animal diet, and may include calcium, copper, sodium, phosphorus, iron and nitrogen.

Thermostability can be measured using the apparent melting temperature ($Tm_{app}$).

The term "derived from" encompasses the terms "originated from," "obtained from," "obtainable from," "isolated from," and "created from."

The methods of feeding described herein may involve any steps which can normally be used in feeding animals. For example, the methods may comprise mixing the feed, admixing, mashing the feed, making a bran and adding supplements, lipids, carbohydrates, proteins, nutrients, nutrition, vitamins, minerals and taste improvers to the feed. The method of feeding may be applied to animals regularly, daily, several times daily or irregularly.

In one aspect the method of feeding an animal with a feed is not a method of treatment or therapy.

**Phytase**

The present invention relates to the use of a phytase.

As used herein, the term "phytase" means a protein or polypeptide which is capable of catalyzing the hydrolysis of esters of phosphoric acid, including phytate and phytic acid, and releasing inorganic phosphate. Some phytases in addition to phytate are capable of hydrolyzing at least some of the inositol-phosphates of intermediate degrees of phosphorylation.

The term "phytase" may be one phytase or a combination of phytases unless the context clearly dictates otherwise.

In the present context the term "phytase" relates to an exogenous phytase supplemented to the feed, i.e. a supplemental phytase. However, the present invention does provide for the additional presence of endogenous phytase. Thus, alternatively expressed, for the present invention the term "phytase" means at least an exogenous phytase supplemented to the feed, i.e. at least a supplemental phytase.

Phytase enzymes, such as e.g. the 6-phytase BP17 derived from *Buttiauxella sp.,* are added to foods and animal feeds to increase phosphate availability thus increasing the nutritional value of the product. The processing of the food or animal feed, for example under heat and high pressure, can denature the phytase and reduce its activity.

The phytase used in the present invention is a phytase from or obtainable from or derivable from a *Buttiauxella* species which is suitable for use in foods or animal feeds.

A phytase is classified as a 6-phytase (classified as E.C. 3.1.3.26) or a 3-phytase (classified as E.C. 3.1.3.8).

The phytase used in the method of invention is a 6-phytase (E.C. 3.1.3.26).

Further described are the following commercial products presented in the Table below:

| Commercial product® | Company | Phytase type | Phytase source |
|---|---|---|---|
| Finase® | ABVista | 3-phytase | *Trichoderma reesei* |
| Finase® EC | ABVista | 6-phytase | *E. coli* gene expressed in *Trichoderma reesei* |
| Natuphos® | BASF | 3-phytase | *Aspergillus Niqer* |
| Natuzyme | Bioproton | phytase (type not specified) | *Trichoderma longibrachiatum/Trichoderma reesei* |
| OPTIPHOS® | Huvepharma AD | 6-phytase | *E. coli* gene expressed in *Pichia pastoris* |
| Phytase sp1002 | DSM | 3-phytase | A Consensus gene expressed in *Hansenula polymorpha* |
| Quantum®2500D, 5000L | ABVista | 6-phytase | *E. coli* gene expressed in *Pichia pastoris* or *Trichoderma reesei* |
| Ronozyme® Hi-Phos (M/L) | DSM/Novozymes | 6-phytase | *Citrobacter braakii* gene expressed in *Aspergillus oryzae* |
| Rovabio® PHY | Adisseo | 3-phytase | *Penicillium funiculosum* |

The phytase is a *Buttiauxella* phytase, e.g. a *Buttiauxella agrestis* phytase; for example: the phytase enzymes taught in WO 2006/043178, WO 2008/097619, WO2009/129489, WO2006/038128, WO2008/092901, PCT/US2009/41011 or PCT/IB2010/051 804.

In one aspect, the enzyme used is BP17 or a polypeptide shown in SEQ ID NO:1 or a variant thereof, such as a polypeptide sequence having at least 70% identity thereto, preferably having at least 75% identity thereto, preferably having at least 80% identity thereto, preferably having at least 85% identity thereto, preferably having at least 90% identity thereto, preferably having at least 95% identity thereto, preferably having at least 96% identity thereto, preferably having at least 97% identity thereto, preferably having at least 98% identity thereto, preferably having at least 99% identity thereto. BP17 is an enzyme variant of a *Buttiauxella sp.* Phytase and is described in e.g. WO2008/097619. The sequence for BP17 (excluding signal peptide), which is used as a reference for position numbering of amino acids throughout, is shown as SEQ ID No. 1 of the present application.

In a highly preferred embodiment, the enzyme used is BP17 and described in e.g. WO2008/097619. BP17 is an enzyme variant of a *Buttiauxella sp.* phytase. The sequence for BP17 (excluding signal peptide) is shown as SEQ ID No. 1.

In one aspect, the enzyme used is BP11 or a polypeptide shown in SEQ ID NO:2 or a variant thereof, such as a sequence having at least 70% identity thereto, preferably having at least 75% identity thereto, preferably having at least 80% identity thereto, preferably having at least 85% identity thereto, preferably having at least 90% identity thereto, preferably having at least 95% identity thereto, preferably having at least 96% identity thereto, preferably having at least 97% identity thereto, preferably having at least 98% identity thereto, preferably having at least 99% identity thereto. BP11 is an enzyme variant of a *Buttiauxella sp.* Phytase and is described in e.g. WO 06/043178. The sequence for BP11 (excluding signal peptide) is shown as SEQ ID No. 2.

Thus, in another embodiment, the enzyme used is BP11 as e.g. described in WO 06/043178. BP11 is currently used in bioethanol production. The sequence for BP11 (excluding signal peptide) is shown as SEQ ID No. 2.

In one aspect, the enzyme used is BP111 or a polypeptide shown in SEQ ID NO:3 or a variant thereof, such as a sequence having at least 70% identity thereto, preferably having at least 75% identity thereto, preferably having at least 80% identity thereto, preferably having at least 85% identity thereto, preferably having at least 90% identity thereto, preferably having at least 95% identity thereto, preferably having at least 96% identity thereto, preferably having at least 97% identity thereto, preferably having at least 98% identity thereto, preferably having at least 99% identity thereto. BP111 is an enzyme variant of a *Buttiauxella sp.* Phytase and is described in e.g. WO 2009/129489. The sequence for BP111 (excluding signal peptide) is shown as SEQ ID No. 3.

Thus, in another embodiment, the enzyme used is BP111. The sequence for BP111 (excluding signal peptide) is shown as SEQ ID No. 3.

All of these phytases are variants of the wild-type sequence such as that derived from *Buttiauxella sp.* strain *P* 1-29 deposited under accession number NCIMB 41248, having the sequence is shown as SEQ ID No. 4.

In their mature form, the above detailed phytase enzymes lack a signal sequence. The appropriate signal sequence derived from *Buttiauxella sp.* strain *P* 1-29 deposited under accession number NCIMB 41248 is shown as SEQ ID No. 5.

In one embodiment, the phytase is produced in a *Trichoderma* host cell. As used herein, the term "*Trichoderma*" or "*Trichoderma* sp." refers to any fungal genus previously or currently classified as *Trichoderma.*

Alternatively the phytase include an *E. coli* phytase, e.g. the phytase marketed under the name Phyzyme® XP by Danisco Animal Nutrition.

Further described is a *Citrobacter* phytase from, derived from, obtained and/or obtainable from e.g. *Citrobacter freundii,* preferably *C.freundii* NCIMB 41247 and variants thereof e.g. as disclosed in WO2006/038062 and WO2006/038128 *Citrobacter braakii* ATCC 51113 as disclosed in WO2006/037328 (incorporated herein by reference), as well as variants thereof e.g. as disclosed in WO2007/112739, *Citrobacter amalonaticus*, preferably *Citrobacter amalonaticus* ATCC 25405 or *Citrobacter amalonaticus* ATCC 25407 as disclosed in WO2006037327, *Citrobacter gillenii,* preferably *Citrobacter gillenii* DSM 13694 as disclosed in WO2006037327, or *Citrobacter intermedius, Citrobacter koseri, Citrobacter murliniae, Citrobacter rodentium, Citrobacter sedlakii, Citrobacter werkmanii, Citrobacter youngae, Citrobacter* species polypeptides or variants thereof.

Further described is a phytase from, derived from, obtained and/or obtainable from *Hafnia,* e.g. from *Hafnia alvei,* such as the phytase enzyme(s) taught in US2008263688, which reference is incorporated herein by reference.

Further described is a phytase from, derived from, obtained and/or obtainable from *Aspergillus*, e.g. from *Apergillus orzyae.*

Further described is a phytase from, derived from, obtained and/or obtainable from *Penicillium*, e.g. from *Penicillium funiculosum.*

The phytase is present in the feed in an amount of at least 500/kg feed, preferably in range of about 100 FTU/kg to about 3000 FTU/kg feed, preferably about 200 FTU/kg to about 2000 FTU/kg feed, more preferably about 300 FTU/kg feed to about 1500 FTU/kg feed, more preferably about 400 FTU/kg feed to about 1000 FTU/kg feed.

In one embodiment the phytase is present in the feed or feedstuff at more than about 100 FTU/kg feed, suitably 200 FTU/kg feed, suitably more than about 300 FTU/kg feed, suitably more than about 400 FTU/kg feed.

In one embodiment the phytase is present in the feed or feedstuff at less than about 2000 FTU/kg feed, suitably less than about 1500 FTU/kg feed, suitably less than about 1000 FTU/kg feed.

In one embodiment, the phytase is present in the feed additive composition in range of about 40 FTU/g to about 100,000 FTU/g composition, more preferably about 100 FTU/g to about 50,000 FTU/g composition, more preferably about 150 FTU/g to about 40,000 FTU/g composition, more preferably about 40 FTU/g to about 40,000 FTU/g composition, more preferably about 80 FTU/g composition to about 20,000 FTU/g composition, and even more preferably about 100 FTU/g composition to about 10,000 FTU/g composition, and even more preferably about 200 FTU/g composition to about 10,000 FTU/g composition.

In one embodiment the phytase is present in the feed additive composition at more than about 40 FTU/g composition, suitably more than about 60 FTU/g composition, suitably more than about 100 FTU/g composition, suitably more than about 125 FTU/g composition, suitably more than about 150 FTU/g composition, suitably more than about 200 FTU/g composition.

In one embodiment the phytase is present in the feed additive composition at less than about 40,000 FTU/g composition, suitably less than about 20,000 FTU/g composition, suitably less than about 15,000 FTU/g composition, suitably less than about 10,000 FTU/g composition.

It will be understood that as used herein one unit of phytase (FTU) is defined as the quantity of enzyme that releases 1 micromol of inorganic phosphorus/min from 0.00015 mol/L of sodium phytate at pH 5.5 at 37 degrees C (Denbow, L. M., V. Ravindran, E. T. Kornegay, Z. Yi, and R. M. Hulet. 1995. Improving phosphorus availability in soybean meal for broilers by supplemental phytase. Poult. Sci.74:1831-1842).

Preferably the phytases used herein have a high phytase activity ratio between pH 2.5 and 5.5 compared to known fungal phytases and *E. coli* phytases.

In one embodiment suitably the enzyme is classified using the E.C. classification above, and the E.C. classification designates an enzyme having that activity when tested in the assay taught herein for determining 1 FTU.

The amount of Phytase Units added to the food or animal feed will depend on the composition of the food or feed itself. Foods and feeds containing lower amounts of available phosphorous will generally require higher amounts of phytase activity. The amount of phytase required may be determined by the skilled person.

In one embodiment, the phytase is in solution or in a liquid form. In one preferred embodiment, the phytase is in a liquid formulation comprising water, sorbitol, sodium chloride (NaCl), potassium sorbate (K-Sorbate) and sodium benzoate, and optionally non-active fermentation solids.

In another embodiment, the phytase is in the solid state.

In a further embodiment, the phytase is spray dried onto a solid support.

In another embodiment, the phytase is incorporated into a granule such as a multi-layered granule.

In one embodiment, the phytase is thermostable, pH stable, low pH tolerant, high pH tolerant, pepsin resistant or shows increased or decreased exo-specificity, or a combination of these properties. The phytase used in the invention may show differing properties compared to a wild-type phytase, particularly the wild-type phytase from which it was derived.

The uses and methods of the invention are compared to *Peniophora lycii* phytase and/or an *E. coli* phytase.

As used herein, the term "phytase activity is retained" and "retains phytase activity" refers to the amount of enzyme activity of a phytase. This may be after one or more of the following treatments during food or animal feed pelleting: heating, increased pressure, increased pH, decreased pH, storage, drying, exposure to surfactants, exposure to solvents, and mechanical stress.

**Pellets and Pelleting**

As used herein, the terms "pellets" and "pelleting" refer - for example - to solid, rounded, spherical and cylindrical tablets or pellets and the processes for forming such solid shapes, particularly feed pellets and solid, extruded animal feed. Pellets may comprise granules and/or multi-layered granules or liquid phytase to be applied after the feed pelleting process.

Known food and animal feed pelleting manufacturing processes generally include admixing together food or feed ingredients for about 1 to about 5 minutes at room temperature, transferring the resulting admixture to a surge bin, conveying the admixture to a steam conditioner, optionally transferring the steam conditioned admixture to an expander, transferring the admixture to the pellet mill or extruder, and finally transferring the pellets into a pellet cooler( Fairfield, D. 1994. Chapter 10, Pelleting Cost Center. In Feed Manufacturing Technology IV. (McEllhiney, editor), American Feed Industry Association, Arlington, Va., pp. 110-139).

Pellets used in the methods of the present invention are typically produced by a method in which the temperature of a feed mixture is raised to a high level in order to kill bacteria. The temperature is often raised by steam treatment prior to pelleting, a process known as conditioning. Subsequently, the conditioned feed mixture is passed through a die to produce pellets of a particular size. The feed mixture is prepared by mixing granules and/or multi-layered granules described herein with food or animal feed as described herein. Pellets may comprise the enzymes mentioned herein. Specifically at least one enzyme (i.e. phytase) mentioned herein.

The steam conditioner treats the admixture for about 20 to about 90 seconds, and up to several minutes, at about 85°C to about 95°C. The terms "conditioning" and "steam conditioning", as used herein, refers to this step in the pellet manufacture process. The amount of steam may vary in accordance with the amount of moisture and the initial temperature of the food or animal feed mix. About 4% to about 6% added steam has been reported in pelleting processes, and the amount is selected to produce less than about 18% moisture in the mash prior to pelleting, or up to about 28% moisture in mash intended for extrusion.

The terms "steam conditioning", "steam treatment" and "steam" are herein used interchangeably.

An optional expander process occurs for about 4 to about 10 seconds at a temperature range of about 100°C to about 140°C. The pellet mill portion of the manufacturing process typically operates for about 3 to about 5 seconds at a temperature of about 85°C to about 95°C.

"Unpelleted mixtures" refer to premixes or precursors, base mixes, mash, and diluents for pellets. Premixes typically contain vitamins and trace minerals. Base mixes typically contain food and animal feed ingredients such as dicalcium phosphate, limestone, salt and a vitamin and mineral premix, but not grains and protein ingredients. Diluents include, but are not limited to, grains (for example wheat middlings and rice bran) and clays, such as phyllosilicates (the magnesium silicate sepiolite, bentonite, kaolin, montmorillonite, hectorite, saponite, beidellite, attapulgite, and stevensite). Clays also function as carriers and fluidizing agent, or diluents, for food and animal feed premixes. Mash typically comprises a complete animal diet. For example, the mash comprises or consists of corn, soybean meal, soy oil, salt, DL Methionine, limestone, dicalcium phosphate and vitamins and minerals. In one example, the mash consists of 61.10% corn, 31.43% soybean meal 48, 4% soy oil, 0.40% salt, 0.20% DL Methionine, 1.16% limestone, 1.46% dicalcium phosphate and 0.25% vitamins and minerals.

In one embodiment, a food or an animal feed for use in the invention is produced by admixing at least one food or feed ingredient (such as a mash) with a phytase in solution, steam conditioning the resulting admixture followed by pelleting the admixture.

In one embodiment, a food or an animal feed for use in the invention is produced by admixing at least one food or animal feed ingredient (such as a mash) with a phytase in the solid state (such as in a multi-layered granule), steam conditioning the resulting admixture followed by pelleting the admixture.

In one embodiment the food or animal feed for use in the invention is in pellet, granule, meal, mash, liquid, wet form, capsule or spray form.

As used herein, the term "heat-treated food or animal feed pellets" refers to unpelleted admixtures which are subjected to a heat treatment (such as steam conditioning) at a temperature of at least 90°C for at least 30 seconds (such as 30 seconds at 90°C and/or 30 seconds at 95°C). The admixture is then, for example, extruded to form the animal feed pellets. For example, the admixture is conditioned with steam for 30 seconds at 90°C. In another example, the admixture is conditioned with steam for 30 seconds at 95°C.

In one aspect a feed of the present invention comprises a steam treated pelletised feed composition comprising a granule comprising a core and one or more coatings. The core may be a salt granule or the like onto which an enzyme solution may have been sprayed so as to form a layer thereon. The core comprises one or more active compounds, such as at least the phytase of the present invention. At least one of the coatings can be a moisture barrier coating. In some embodiments at least one of the coatings comprises a salt. For certain embodiments, the granules are approximately 210 to 390 $\mu$m in size. In some embodiments, the granules may be up to 450 $\mu$m or more in size or up to 500 $\mu$m or more in size. Examples of such an embodiment may be found in WO 2006/034710, WO 00/01793, WO 99/32595, WO 2007/044968, WO 00/47060, WO 03/059086, WO 03/059087, WO 2006/053564 and US 2003/0054511.

A preferred salt for the coating of the pellets is one or more of that described in WO2006/034710. Examples of preferred salts for coating the pellets include one or more of: $Na_2SO_4$ NaCl, $Na_2CO_3$, $NaNO_3$, $Na_2HPO_4$, $Na_3PO_4$, $NH_4CL$, $(NH_4)_2HPO_4$, $NH_4H_2PO_4$, $(NH_4)_2SO_4$, KCl, $K_2HPO_4$, $KH_2PO_4$, $KNO_3$, $K_2SO_4$, $KHSO_4$, $MgSO_4$, $ZnSO_4$ and sodium citrate or mixtures thereof. For some aspects, examples of more preferred salts for coating the pellets include one or more sulphates, such as one or more $Na_2SO_4$, $(NH_4)_2SO_4$, $K_2SO_4$, $KHSO_4$, $MgSO_4$, $ZnSO_4$ or mixtures thereof. For some aspects, examples of more preferred salts for coating the pellets include one or more $Na_2SO_4$, $(NH_4)_2SO_4$, and $MgSO_4$ or mixtures thereof. For some aspects, a preferred salt for coating the pellets is or includes at least $Na_2SO_4$.

In certain aspects the feed of the present invention comprises a granule that comprises a core, wherein the core comprises at least a phytase according to the present invention, and wherein the core is coated with one or more coatings, wherein at least one of the coatings comprises a moisture barrier. The granule may be a steam treated granule. The granule may be a steam treated pelletised granule.

In certain aspects the feed of the present invention comprises a granule, wherein the granule comprises a core that comprises at least a phytase according to the present invention, and wherein the core is coated with one or more coatings, wherein at least one of the coatings comprises a salt that is capable of acting as a moisture barrier. The granule may be a steam treated granule. The granule may be a steam treated pelletised granule.

In certain aspects the feed of the present invention comprises a granule, wherein the granule comprises a core that comprises at least a phytase according to the present invention, and wherein the core is coated with one or more coatings, wherein at least one of the coatings comprises one or more of $Na_2SO_4$ NaCl, $Na_2CO_3$, $NaNO_3$, $Na_2HPO_4$, $Na_3PO_4$, $NH_4CL$, $(NH_4)_2HPO_4$, $NH_4H_2PO_4$, $(NH_4)_2SO_4$, KCl, $K_2HPO_4$, $KH_2PO_4$, $KNO_3$, $K_2SO_4$, $KHSO_4$, $MgSO_4$, $ZnSO_4$ and sodium citrate or mixtures thereof. The granule may be a steam treated granule. The granule may be a steam treated pelletised granule.

In certain aspects the feed of the present invention comprises a granule, wherein the granule comprises a core that comprises at least a phytase according to the present invention, and wherein the core is coated with one or more coatings, wherein at least one of the coatings comprises one or more sulphates, such as one or more $Na_2SO_4$, $(NH_4)_2SO_4$, $K_2SO_4$, $KHSO_4$, $MgSO_4$, $ZnSO_4$ or mixtures thereof. The granule may be a steam treated granule. The granule may be a steam treated pelletised granule.

In certain aspects the feed of the present invention comprises a granule, wherein the granule comprises a core that comprises at least a phytase according to the present invention, and wherein the core is coated with one or more coatings, wherein at least one of the coatings comprises one or more of $Na_2SO_4$, $(NH_4)_2SO_4$, and $MgSO_4$ or mixtures thereof. The granule may be a steam treated granule. The granule may be a steam treated pelletised granule.

In certain aspects the feed of the present invention comprises a granule, wherein the granule comprises a core that comprises at least a phytase according to the present invention, and wherein the core is coated with one or more coatings, wherein at least one of the coatings is or includes at least $Na_2SO_4$. The granule may be a steam treated granule. The granule may be a steam treated pelletised granule.

In certain aspects the feed of the present invention comprises a granule that comprises a core, wherein the core comprises at least BP17 phytase according to the present invention, and wherein the core is coated with one or more coatings, wherein at least one of the coatings comprises a moisture barrier. The granule may be a steam treated granule. The granule may be a steam treated pelletised granule.

In certain aspects the feed of the present invention comprises a granule, wherein the granule comprises a core that comprises at least BP17 phytase according to the present invention, and wherein the core is coated with one or more coatings, wherein at least one of the coatings comprises a salt that is capable of acting as a moisture barrier. The granule may be a steam treated granule. The granule may be a steam treated pelletised granule.

In certain aspects the feed of the present invention comprises a granule, wherein the granule comprises a core that comprises at least BP17 phytase according to the present invention, and wherein the core is coated with one or more coatings, wherein at least one of the coatings comprises one or more of $Na_2SO_4$ NaCl, $Na_2CO_3$, $NaNO_3$, $Na_2HPO_4$, $Na_3PO_4$, $NH_4CL$, $(NH_4)_2HPO_4$, $NH_4H_2PO_4$, $(NH_4)_2SO_4$, KCI, $K_2HPO_4$, $KH_2PO_4$, $KNO_3$, $K_2SO_4$, $KHSO_4$, $MgSO_4$, $ZnSO_4$ and sodium citrate or mixtures thereof. The granule may be a steam treated granule. The granule may be a steam treated pelletised granule.

In certain aspects the feed of the present invention comprises a granule, wherein the granule comprises a core that comprises at least BP17 phytase according to the present invention, and wherein the core is coated with one or more coatings, wherein at least one of the coatings comprises one or more sulphates, such as one or more $Na_2SO_4$, $(NH_4)_2SO_4$, $K_2SO_4$, $KHSO_4$, $MgSO_4$, $ZnSO_4$ or mixtures thereof. The granule may be a steam treated granule. The granule may be a steam treated pelletised granule.

In certain aspects the feed of the present invention comprises a granule, wherein the granule comprises a core that comprises at least BP17 phytase according to the present invention, and wherein the core is coated with one or more coatings, wherein at least one of the coatings comprises one or more of $Na_2SO_4$, $(NH_4)_2SO_4$, and $MgSO_4$ or mixtures thereof. The granule may be a steam treated granule. The granule may be a steam treated pelletised granule.

In certain aspects the feed of the present invention comprises a granule, wherein the granule comprises a core that comprises at least BP17 phytase according to the present invention, and wherein the core is coated with one or more coatings, wherein at least one of the coatings is or includes at least $Na_2SO_4$. The granule may be a steam treated granule. The granule may be a steam treated pelletised granule.

In certain aspects the feed of the present invention comprises a granule, wherein the granule comprises a core that comprises at least BP17 or a polypeptide shown in SEQ ID NO:1 or a variant thereof, such as a sequence having at least 70% identity thereto, preferably having at least 75% identity thereto, preferably having at least 80% identity thereto, preferably having at least 85% identity thereto, preferably having at least 90% identity thereto, preferably having at least 95% identity thereto, preferably having at least 96% identity thereto, preferably having at least 97% identity thereto, preferably having at least 98% identity thereto, preferably having at least 99% identity thereto.

In certain aspects the feed of the present invention comprises a granule that comprises a core, wherein the core comprises at least BP11 phytase according to the present invention, and wherein the core is coated with one or more coatings, wherein at least one of the coatings comprises a moisture barrier. The granule may be a steam treated granule. The granule may be a steam treated pelletised granule.

In certain aspects the feed of the present invention comprises a granule, wherein the granule comprises a core that comprises at least BP11 phytase according to the present invention, and wherein the core is coated with one or more coatings, wherein at least one of the coatings comprises a salt that is capable of acting as a moisture barrier. The granule may be a steam treated granule. The granule may be a steam treated pelletised granule.

In certain aspects the feed of the present invention comprises a granule, wherein the granule comprises a core that comprises at least BP11 phytase according to the present invention, and wherein the core is coated with one or more coatings, wherein at least one of the coatings comprises one or more of $Na_2SO_4$ NaCl, $Na_2CO_3$, $NaNO_3$, $Na_2HPO_4$, $Na_3PO_4$, $NH_4CL$, $(NH_4)_2HPO_4$, $NH_4H_2PO_4$, $(NH_4)_2SO_4$, KCI, $K_2HPO_4$, $KH_2PO_4$, $KNO_3$, $K_2SO_4$, $KHSO_4$, $MgSO_4$, $ZnSO_4$ and sodium citrate or mixtures thereof. The granule may be a steam treated granule. The granule may be a steam treated pelletised granule.

In certain aspects the feed of the present invention comprises a granule, wherein the granule comprises a core that comprises at least BP11 phytase according to the present invention, and wherein the core is coated with one or more coatings, wherein at least one of the coatings comprises one or more sulphates, such as one or more $Na_2SO_4$, $(NH_4)_2SO_4$, $K_2SO_4$, $KHSO_4$, $MgSO_4$, $ZnSO_4$ or mixtures thereof. The granule may be a steam treated granule. The granule may be a steam treated pelletised granule.

In certain aspects the feed of the present invention comprises a granule, wherein the granule comprises a core that comprises at least BP11 phytase according to the present invention, and wherein the core is coated with one or more coatings, wherein at least one of the coatings comprises one or more of $Na_2SO_4$, $(NH_4)_2SO_4$, and $MgSO_4$ or mixtures thereof. The granule may be a steam treated granule. The granule may be a steam treated pelletised granule.

In certain aspects the feed of the present invention comprises a granule, wherein the granule comprises a core that comprises at least BP11 phytase according to the present invention, and wherein the core is coated with one or more coatings, wherein at least one of the coatings is or includes at least $Na_2SO_4$. The granule may be a steam treated granule. The granule may be a steam treated pelletised granule.

In certain aspects the feed of the present invention comprises a granule that comprises a core, wherein the core comprises at least BP111 phytase according to the present invention, and wherein the core is coated with one or more coatings, wherein at least one of the coatings comprises a moisture barrier. The granule may be a steam treated granule. The granule may be a steam treated pelletised granule.

In certain aspects the feed of the present invention comprises a granule, wherein the granule comprises a core that comprises at least BP111 phytase according to the present invention, and wherein the core is coated with one or more coatings, wherein at least one of the coatings comprises a salt that is capable of acting as a moisture barrier. The granule may be a steam treated granule. The granule may be a steam treated pelletised granule.

In certain aspects the feed of the present invention comprises a granule, wherein the granule comprises a core that comprises at least BP111 phytase according to the present invention, and wherein the core is coated with one or more coatings, wherein at least one of the coatings comprises one or more of $Na_2SO_4$ NaCl, $Na_2CO_3$, $NaNO_3$, $Na_2HPO_4$, $Na_3PO_4$, $NH_4Cl$, $(NH_4)_2HPO_4$, $NH_4H_2PO_4$, $(NH_4)_2SO_4$, KCl, $K_2HPO_4$, $KH_2PO_4$, $KNO_3$, $K_2SO_4$, $KHSO_4$, $MgSO_4$, $ZnSO_4$ and sodium citrate or mixtures thereof. The granule may be a steam treated granule. The granule may be a steam treated pelletised granule.

In certain aspects the feed of the present invention comprises a granule, wherein the granule comprises a core that comprises at least BP111 phytase according to the present invention, and wherein the core is coated with one or more coatings, wherein at least one of the coatings comprises one or more sulphates, such as one or more $Na_2SO_4$, $(NH_4)_2SO_4$, $K_2SO_4$, $KHSO_4$, $MgSO_4$, $ZnSO_4$ or mixtures thereof. The granule may be a steam treated granule. The granule may be a steam treated pelletised granule.

In certain aspects the feed of the present invention comprises a granule, wherein the granule comprises a core that comprises at least BP111 phytase according to the present invention, and wherein the core is coated with one or more coatings, wherein at least one of the coatings comprises one or more of $Na_2SO_4$, $(NH_4)_2SO_4$, and $MgSO_4$ or mixtures thereof. The granule may be a steam treated granule. The granule may be a steam treated pelletised granule.

In certain aspects the feed of the present invention comprises a granule, wherein the granule comprises a core that comprises at least BP111 phytase according to the present invention, and wherein the core is coated with one or more coatings, wherein at least one of the coatings is or includes at least $Na_2SO_4$. The granule may be a steam treated granule. The granule may be a steam treated pelletised granule.

For certain embodiments, the granules are approximately 210 to 390 $\mu$m in size. Examples of such an embodiment may be found in WO 2006/034710, WO 00/01793, WO 99/32595, WO 2007/044968, WO 00/47060, WO 03/059086, WO 03/059087, WO 2006/053564 and US 2003/0054511.

A preferred salt for the coating of the pellets is one or more of that described in WO2006/034710. Examples of preferred salts for coating the pellets include one or more of: $Na_2SO_4$ NaCl, $Na_2CO_3$, $NaNO_3$, $Na_2HPO_4$, $Na_3PO_4$, $NH_4Cl$, $(NH_4)_2HPO_4$, $NH_4H_2PO_4$, $(NH_4)_2SO_4$, KCl, $K_2HPO_4$, $KH_2PO_4$, $KNO_3$, $K_2SO_4$, $KHSO_4$, $MgSO_4$, $ZnSO_4$ and sodium citrate or mixtures thereof. For some aspects, examples of more preferred salts for coating the pellets include one or more sulphates, such as one or more $Na_2SO_4$, $(NH_4)_2SO_4$, $K_2SO_4$, $KHSO_4$, $MgSO_4$, $ZnSO_4$ or mixtures thereof. For some aspects, examples of more preferred salts for coating the pellets include one or more $Na_2SO_4$, $(NH_4)_2SO_4$, and $MgSO_4$ or mixtures thereof. For some aspects, a preferred salt for coating the pellets is or includes at least $Na_2SO_4$.

A method for manufacturing a feed composition is also described herein. In one aspect this method comprises the steps of: i. mixing feed components with granules comprising a core and a coating wherein the core comprises an active compound, such as an enzyme including phytase, and the coating comprises a salt, ii. steam treating said composition (i), and iii. pelleting said composition (ii).

**Food and feed**

A "feed" and a "food," respectively, means any natural or artificial diet, meal or the like or components of such meals intended or suitable for being eaten, taken in, digested, by an animal and a human being, respectively.

As used herein, the term "food" is used in a broad sense - and covers food and food products for humans as well as food for animals (i.e. a feed). The term "foodstuff" encompasses any ingredient which may be used for food.

The term "feed" is used with reference to products that are fed to animals in the rearing of livestock. The term "feed" may encompass a feed *per se* or a feed composition or a component thereof. The terms "feed" and "animal feed" are used interchangeably. In a preferred embodiment, the food or feed is for consumption by animals such as monogastric animals, non mono-gastric animals, ruminant animals or aquatic animals - for example - swine (e.g. pig), poultry (e.g. turkey, chicken, duck), cattle and fish. In a most preferred embodiment the food or feed is for consumption by chickens, turkeys, pigs, cattle or fish. The term "feedstuff" encompasses any ingredient which may be used for feed.

In a preferred embodiment, the food or feed is for consumption by non-monogastric animals. In another preferred embodiment, the food or feed is for consumption by non-monogastric animals such as ruminant and non-ruminant animals.

In preferred embodiment the non-monogastric animals include, but are not limited to, the following: Ruminants from the *Bovinae* sub family including Bison, Yak, Cattle, Cow, Buffalo, Bovine, Livestock, Ox, Steer, Nilagi, and dairy and beef producing animals; Ruminants of *Bos taurus* and *Bos Indicus* such as cows, bulls, steers, beef cattle, dairy cattle and calves; Ruminants of the Cervidae sub family including, Deer, Reindeer, Antelope, Moose, Elk and Muntjac; or Ruminants of the Caprinae sub family including Sheep, Goat, Lambs, Chamois and Pronghorn; or non-ruminants such as Giraffe, or non-ruminants such as Equus or Equine species such as Horse, Donkey, and Mule; or non-ruminants such as Camelidae including Llama, Alpaca and Camel.

In another preferred embodiment, the food or feed is for consumption by aquatic animals such as - for example - fish, agastric fish, gastric fish, freshwater fish, marine fish and shrimp and other crustaceans.

In another preferred embodiment, the food or feed is for consumption by mono-gastric animals such as - for example - pigs, poultry such as ducks, chickens and turkeys, dogs, cats, and humans.

The food or feed may be in the form of a solution or as a solid - depending on the use and/or the mode of application and/or the mode of administration. In some embodiments, the enzymes mentioned herein may be used as - or in the preparation or production of - a food or feed substance.

As used herein the term "food or feed ingredient" includes a formulation, which is or can be added to foods or foodstuffs and includes formulations which can be used at low levels in a wide variety of products. The food ingredient may be in the form of a solution or as a solid - depending on the use and/or the mode of application and/or the mode of administration. The enzymes described herein may be used as a food or feed ingredient or in the preparation or production. The enzymes may be - or may be added to - food supplements.

Feed compositions for animals such as monogastric animals, non monogastric animals, ruminant animals and aquatic animals typically include composition comprising plant products which contain phytate. Such compositions include cornmeal, soybean meal, rapeseed meal, cottonseed meal, maize, wheat, barley and sorghum-based feeds. Phytase may be - or may be added to - foods or feed substances and compositions.

In one embodiment, the food or animal feed is a liquid such as a liquid feed.

In another embodiment, the food or animal feed is a solid.

In another embodiment, the animal feed is feed for poultry (poultry feed).

In one embodiment, the animal feed is feed for chickens (chicken feed).

In another embodiment, the animal feed is feed for turkeys (turkey feed).

In another embodiment, the animal feed is feed for ducks (duck feed).

In one embodiment, the animal feed is feed for pigs (pig feed).

The food or animal feed may comprise vegetable proteins. Vegetable proteins may be derived from legumes, oil seeds, nuts and cereals. Examples of sources of vegetable proteins include, but are not limited to, plants from the families *Fabaceae* (*Leguminosae*), *Poaceae*, *Cruciferaceae*, and *Chenopodiaceae.*

Suitable sources of vegetable proteins are soy beans, soy bean meal, cereals (such as maize (corn), wheat, oats, barley, rye, and sorghum), cereal meals (such as corn meal, wheat meal, oat meal, barley meal, rye meal, sorghum meal, and canola meal), brans (such as what bran, and oat bran), oil seeds (such as rapeseed and sunflower seeds), oil seed meals (such as rapeseed meal), cottonseed meal, cabbage, beet and sugar beet. These vegetable proteins are examples of food ingredients and animal feed ingredients.

The food or animal feed may comprise animal proteins. Suitable animal proteins include, but are not limited to, fish-meal and whey. These animal proteins are examples of food ingredients and animal feed ingredients.

The food or animal feed may comprise additives. Suitable additives include, but are not limited to, enzyme inhibitors, vitamins, trace minerals, macro minerals, coloring agents, aroma compounds and antimicrobial peptides. These additives are examples of food ingredients and animal feed ingredients.

The food or animal feed or ingredients thereof may be a liquid.

The food or animal feed or ingredients thereof may be a solid. Examples include corn, wheat or soy meal.

As used herein, the term "digestibility" refers to the ability of an animal to absorb or retain nutrition from a food or feed rather than excreting it. The word "nutrition" in this context may include minerals, fats, lipids, vitamins, proteins, amino acids, carbohydrates and starches. A measure of digestability is the amount of any aspect of nutrition which is retained by the animal rather than excreted. This may be expressed as a percentage.

**Animals**

As used herein, the term "animal" includes all monogastric animals, all non-monogastric animals, all ruminant animals and all aquatic animals.

Examples of animals include mono-gastric animals such as pigs, poultry such as ducks, chickens and turkeys, dogs, cats, and humans.

Wherein the animal is a chicken, it may be any type or breed of chicken. In preferred embodiments the chicken is a broiler or layer or poult. The terms "chicken" and "hen" are used interchangeably herein.

Wherein the animal is a turkey, it may be any type or breed of turkey. For example the turkey may be a poult.

Wherein the animal is a pig, it may be any type or breed of pig. In preferred embodiments, the pig is a sow, piglet, swine, hog or a grower finisher pig.

As used herein, the term "animal" includes all non-monogastric animals. In preferred embodiments non-monogastric animals include, but are not limited to, the following:- Ruminants from the *Bovinae* sub family including Bison, Yak, Cattle, Cow, Buffalo, Bovine, Livestock, Ox, Steer, Nilagi, and dairy and beef producing animals; Ruminants of *Bos taurus* and *Bos Indicus* such as cows, bulls, steers, beef cattle, dairy cattle and calves; Ruminants of the Cervidae sub family including, Deer, Reindeer, Antelope, Moose, Elk and Muntjac; or Ruminants of the Caprinae sub family including Sheep, Goat, Lambs, Chamois and Pronghorn; or non-ruminants such as Giraffe, or non-ruminants such as Equus or Equine species such as Horse, Donkey, and Mule; or non-ruminants such as Camelidae including Llama, Alpaca and Camel.

An animal may be both a non mono-gastric animal and also a ruminant animal.

The terms "non mono-gastric", "non-monogastric", "non mono gastric" and non-mono-gastric" are used interchangeably herein.

The terms "mono-gastric" and "monogastric" are used interchangeably herein.

As used herein, the term "animal" includes all aquatic animals.

Examples of aquatic animals include shrimp and other crustaceans, for example Peneids Shrimp: Black tiger shrimp *Peneaus monodon,* white shrimp *Penaeus vananamei.* Other examples of aquatic animals include all fish. All fish includes freshwater fish, gastric fish including all tilapia species, (for example *Oreochromis niloticus* and *O. mossanbicus*) and all catfish species, (for example *Pangasus* spp and Channel catfish *Ictalurus punctatus).* All fish also includes agastric fish including all carp species (for example common Carp *Cyprinus carpio* and grass Carp *Ctenopharyngodon idella*), Salmonids (for example Atlantic salmon (*Salmo salar*), Pacific salmon (*Oncorhynchus spp*), Rainbow trout (*Oncorhynchus mykiss*).) and all marine fish (for example eel, seabass and seabream families).

**Nucleotides and polypeptides**

As used herein, the term "polynucleotide" refers to a polymeric form of nucleotides of any length and any three-dimensional structure and single- or multi-stranded (*e.g.*, single-stranded, double-stranded, triple-helical, etc.), which contain deoxyribonucleotides, ribonucleotides, and/or analogs or modified forms of deoxyribonucleotides or ribonucleotides, including modified nucleotides or bases or their analogs. Because the genetic code is degenerate, more than one codon may be used to encode a particular amino acid, and the present description encompasses polynucleotides which encode a particular amino acid sequence. Any type of modified nucleotide or nucleotide analog may be used, so long as the polynucleotide retains the desired functionality under conditions of use, including modifications that increase nuclease resistance (*e.g.*, deoxy, 2'-O--Me, phosphorothioates, etc.). Labels may also be incorporated for purposes of detection or capture, for example, radioactive or nonradioactive labels or anchors, *e.g.*, biotin. The term polynucleotide also includes peptide nucleic acids (PNA). Polynucleotides may be naturally occurring or non-naturally occurring. The terms "polynucleotide" and "nucleic acid" and "oligonucleotide" and "nucleotide sequence" are used herein interchangeably. Polynucleotides of the description may contain RNA, DNA, or both, and/or modified forms and/or analogs thereof. A sequence of nucleotides may be interrupted by non-nucleotide components. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR$_2$ ("amidate"), P(O)R, P(O)OR', CO or CH$_2$ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. Polynucleotides may be linear or circular or comprise a combination of linear and circular portions.

As used herein, "polypeptide" refers to any composition comprising or comprised of amino acids and recognized as a protein by those of skill in the art. The conventional one-letter or three-letter code for amino acid residues is used herein. The terms "polypeptide" and "protein" and "amino acid sequence" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

**Enzymes**

The term "enzyme" as used herein refers to a protein which catalyses the chemical reactions of other substances without itself being destroyed or altered upon completion of the reactions.

The enzyme can be a wild-type, which is an enzyme present in nature, or a variant. A "variant" is an enzyme having an amino acid sequence which has one or several insertions, deletions and/or substitutions compared with the parent sequence from which the variant is derived such as a wild-type enzyme or even a variant enzyme, and which retains a functional property and/or enhances a property, e.g. an enhanced activity, of the enzyme. As used herein, the term "amino acid sequence" is synonymous with the term "polypeptide" and/or the term "protein". A variant enzyme may also be referred to as a modified or altered enzyme.

The term "active enzyme" as used herein refers to an enzyme which retains its catalytic function. For example, phytase is capable of catalyzing the hydrolysis of esters of phosphoric acid.

The term "inactive enzyme" refers to enzyme which is present in a sample but is incapable of performing its catalytic function. Inactivation may occur due to denaturation, aggregation, deamidation or oxidation of the enzyme, due to heat treatment or due to chemical treatment or treatment or processing by another enzyme such as proteolysis by a protease. Inactivation may also be due to a chemical inhibitor. In the case where the enzyme is phytase, such as e.g. phytase BP17, an inhibitor which can be used is Myoinositol Hexasulphate (MIHS). Inactivation may be complete, which means there no enzyme activity, or partial wherein some residual activity remains.

As used herein, a "vector" refers to a polynucleotide sequence designed to introduce nucleic acids into one or more cell types. Vectors include cloning vectors, expression vectors, shuttle vectors, plasmids, phage particles, cassettes and the like.

As used herein, the term "expression" refers to the process by which a polypeptide is produced based on the nucleic acid sequence of a gene. The process includes both transcription and translation. Expression may involve the use of a host organism to produce the polypeptide. A host organism, also referred to simply as a host, can include prokaryotes and eukaryotes, and may in some embodiments include bacterial and fungal species.

As used herein, "expression vector" refers to a DNA construct containing a DNA coding sequence (*e.g.*, gene sequence) that is operably linked to one or more suitable control sequence(s) capable of affecting expression of the coding sequence in a host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. The plasmid is the most commonly used form of expression vector. However, the description is intended to include such other forms of expression vectors that serve equivalent functions and which are, or become, known in the art.

A "promoter" refers to a regulatory sequence that is involved in binding RNA polymerase to initiate transcription of a gene. The promoter may be an inducible promoter or a constitutive promoter. A non-limiting example of an inducible promoter which may be used is *Trichoderma reesei* cbh1, which is an inducible promoter.

The term "operably linked" refers to juxtaposition wherein the elements are in an arrangement allowing them to be functionally related. For example, a promoter is operably linked to a coding sequence if it controls the transcription of the coding sequence.

"Under transcriptional control" is a term well understood in the art that indicates that transcription of a polynucleotide sequence depends on its being operably linked to an element which contributes to the initiation of, or promotes transcription.

"Under translational control" is a term well understood in the art that indicates a regulatory process which occurs after mRNA has been formed.

A "gene" refers to a DNA segment that is involved in producing a polypeptide and includes regions preceding and following the coding regions as well as intervening sequences (introns) between individual coding segments (exons).

As used herein, the term "host cell" refers to a cell or cell line into which a recombinant expression vector for production of a polypeptide may be transfected for expression of the polypeptide. Host cells include progeny of a single host cell, and the progeny may not necessarily be completely identical (in morphology or in total genomic DNA complement) to the original parent cell due to natural, accidental, or deliberate mutation. A host cell includes cells transfected or transformed *in vivo* with an expression vector. "Host cell" refers to both cells and protoplasts created from the cells of a filamentous fungal strain and particularly a *Trichoderma* sp. strain.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

A "signal sequence" (also termed "presequence," "signal peptide," "leader sequence," or "leader peptide") refers to a sequence of amino acids bound to the N-terminal portion of a protein which facilitates the secretion of the mature form of the protein from the cell (e.g. SEQ ID NO: 5). The signal sequence targets the polypeptide to the secretory pathway and is cleaved from the nascent polypeptide once it is translocated in the endoplasmic reticulum membrane. The mature form of the extracellular protein (e.g. SEQ ID NO: 1) lacks the signal sequence which is cleaved off during the secretion process.

The term "selective marker" or "selectable marker" refers to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell.

The term "culturing" refers to growing a population of microbial cells under suitable conditions for growth, in a liquid or solid medium.

The term "heterologous" in reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes. The term "homologous" in reference to a polynucleotide or protein refers to a polynucleotide or protein that occurs naturally in the host cell.

The term "introduced" in the context of inserting a nucleic acid sequence into a cell includes "transfection," "transformation," or "transduction" and refers to the incorporation of a nucleic acid sequence into a eukaryotic or prokaryotic cell wherein the nucleic acid sequence may be incorporated into the genome of the cell (e.g., chromosome, plasmid, plastid, or mitochondrial DNA), converted into an autonomous replicon, or transiently expressed.

As used herein, the terms "transformed," "stably transformed," and "transgenic" refer to a cell that has a non-native (e.g., heterologous) nucleic acid sequence integrated into its genome or as an episomal plasmid that is maintained through multiple generations.

The terms "recovered," "isolated," "purified," and "separated" as used herein refer to a material (e.g., a protein, nucleic acid, or cell) that is removed from at least one component with which it is naturally associated. For example, these terms may refer to a material which is substantially or essentially free from components which normally accompany it as found in its native state, such as, for example, an intact biological system.

As used herein, the terms "modification" and "alteration" are used interchangeably and mean to change or vary. In the context of modifying or altering a polypeptide, these terms may mean to change the amino acid sequence, either directly or by changing the encoding nucleic acid, or to change the structure of the polypeptide such as by glycosylating the enzyme.

"NCIMB" refers to NCIMB Ltd located in Aberdeen, Scotland (www.NCIMB.com).

The practice of the present invention will employ, unless otherwise indicated, conventional techniques of molecular biology (including recombinant techniques), microbiology, cell biology, and biochemistry, which are within the skill of the art. Such techniques are explained fully in the literature, for example, Molecular Cloning: A Laboratory Manual, second edition (Sambrook et al., 1989 Molecular Cloning: A Laboratory Manual); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); and Gene Transfer and Expression: A Laboratory Manual (Kriegler, 1990).

Unless defined otherwise herein, all technical and scientific terms used herein have the same meaning as commonly understood by one of ordinary skill in the art to which this invention belongs. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present invention.

Unless otherwise indicated, nucleic acids are written left to right in 5' to 3' orientation; amino acid sequences are written left to right in amino to carboxy orientation, respectively.

Numeric ranges provided herein are inclusive of the numbers defining the range.

"A," "an" and "the" include plural references unless the context clearly dictates otherwise.

## Sequence Identity or Sequence Homology

The present description also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid or a nucleotide sequence which may be at least 75, 80, 85 or 90% identical, in some embodiments at least 95 or 98% identical to the subject sequence. Typically, the homologues will comprise the same active sites etc. as the subject amino acid sequence for instance. Although homology can also be considered in terms of similarity (i.e. amino acid residues having similar chemical properties/functions), in the context of the present description it is preferred to express homology in terms of sequence identity.

In some embodiments, a homologous sequence is taken to include an amino acid sequence or nucleotide sequence which has one or several additions, deletions and/or substitutions compared with the subject sequence.

In some embodiments the present description relates to a protein whose amino acid sequence is represented herein or a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In some embodiments the present description relates to a nucleic acid sequence (or gene) encoding a protein whose amino acid sequence is represented herein or encoding a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In the present context, a homologous sequence is taken to include a nucleotide sequence which may be at least 75, 80, 85 or 90% identical, in some embodiments at least 95 or 98% identical to a nucleotide sequence encoding a polypeptide of the present description (the subject sequence). Typically, the homologues will comprise the same sequences that code for the active sites etc. as the subject sequence. Although homology can also be considered in terms of similarity (i.e. amino acid residues having similar chemical properties/functions), in the context of the present description it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalizing unduly the overall homology score. This is achieved by inserting "gaps" in the sequence alignment to try to maximize local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimized alignments with fewer gaps. Most alignment programs allow the gap penalties to be modified. However, it is preferred to use the default values when using such software for sequence comparisons.

Calculation of maximum % homology therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, for example % sequence identity. The software typically does this as part of the sequence comparison and generates a numerical result.

Should Gap Penalties be used when determining sequence identity, then the following parameters can be used for pairwise alignment for example:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, CLUSTAL may be used with the gap penalty and gap extension set as defined above.

Suitably, the degree of identity with regard to a nucleotide sequence is determined over at least 20 contiguous nucleotides, for example over at least 30 contiguous nucleotides, for example over at least 40 contiguous nucleotides, for example over at least 50 contiguous nucleotides, for example over at least 60 contiguous nucleotides, for example over at least 100 contiguous nucleotides.

Suitably, the degree of identity with regard to a nucleotide sequence may be determined over the whole sequence.

**Variants/Homologues/Derivatives**

The present description also encompasses the use of variants, homologues and derivatives of any amino acid sequence of a protein or of any nucleotide sequence encoding such a protein.

Here, the term "homologue" means an entity having a certain homology with the subject amino acid sequences and the subject nucleotide sequences. Here, the term "homology" can be equated with "identity".

In the present context, a homologous sequence is taken to include an amino acid sequence which may be at least 75, 80, 85 or 90% identical, for example at least 95, 96, 97, 98 or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present description it is preferred to express homology in terms of sequence identity.

In the present context, an homologous sequence is taken to include a nucleotide sequence which may be at least 75, 80, 85 or 90% identical, for example at least 95, 96, 97, 98 or 99% identical to a nucleotide sequence encoding an enzyme of the present description (the subject sequence). Typically, the homologues will comprise the same sequences that code for the active sites etc. as the subject sequence. Although homology can also be considered in terms of similarity (i.e. amino acid residues having similar chemical properties/functions), in the context of the present description it is preferred to express homology in terms of sequence identity.

As discussed above, homology comparisons can be conducted by eye, or more usually, with the aid of readily available sequence comparison programs, for both polypeptide and nucleotide sequences.

As described above, % homology may be calculated over contiguous sequences.

More complex alignment methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. It is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software than can perform sequence comparisons include, but are not limited to, the BLAST package (see Ausubel et al., 1999 Short Protocols in Molecular Biology, 4th Ed - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

As described above, once the software has produced an optimal alignment, it is possible to calculate % homology, for example % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and in some embodiments in the same line in the third column may be substituted for each other:

| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present description also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-l-phenylalanine*, L-allyl-glycine*, β-alanine*, L-α-amino butyric acid*, L-γ-amino butyric acid*, L-α-amino isobutyric acid*, L-ε-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) such as 4-methyl-Phe*, pentamethyl-Phe*, L-Phe (4-amino)", L-Tyr (methyl)*, L-Phe (4-isopropyl)*, L-Tic (1,2,3,4-tetrahydroisoquinoline-3-carboxyl acid)*, L-diaminopropionic acid# and L-Phe (4-benzyl)*. The notation * has been utilized for the purpose of the discussion above (relating to homologous or non-homologous substitution), to indicate the hydrophobic nature of the derivative whereas # has been utilized to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present description may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present description, it is to be understood that the nucleotide sequences described herein may be modified by any method available in the art. Such modifications may be carried out in order to enhance the *in vivo* activity or life span of nucleotide sequences of the present description.

The present description also encompasses the use of nucleotide sequences that are complementary to the sequences presented herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present description but fall within the scope of the description can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridizing to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the description.

Variants and strain/species homologues may also be obtained using degenerate PCR which will use primers designed to target sequences within the variants and homologues encoding conserved amino acid sequences within the sequences of the present description. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterized sequences. This may be useful where for example silent codon sequence changes are required to optimize codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) of the description may be used to produce a primer, e.g. a PCR primer, a primer for an alternative amplification reaction, a probe e.g. labeled with a revealing label by conventional means using radioactive or non-radioactive labels, or the polynucleotides may be cloned into vectors. Such primers, probes and other fragments will be at least 15, for example at least 20, for example at least 25, 30 or 40 nucleotides in length, and are also encompassed by the term polynucleotides of the description as used herein.

Polynucleotides such as DNA polynucleotides and probes according to the description may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

The present description may also relate to the use of a feed comprising an enzyme of any of SEQ ID NOs:1-4 or a polypeptide derived from this (parent) enzyme by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

The enzymes of the present description are used in food or feed, in the preparation of food or feed and/or in food or feed additives or their preparation. In some embodiments the enzymes of the current description may form a composition with other food or feed ingredients, or may be added to a composition of food or feed ingredients. In some embodiments the enzymes of the present description are more thermostable than comparative or similar enzymes used in food or feed production.

**Utility**

The present invention provides a method of feeding an animal, such as a mono-gastric animal, non mono-gastric animal, ruminant animal or aquatic animal, with a feed which results in an improvement of one or more of said animals' biophysical characteristics.

Such properties allow the methods of this description to be used in improving output for farmers and animal breeders, improving productivity and improving animal welfare.

In particular, the methods of the current description may be used for increasing improvement in an animal's biophysical characteristics as a food source.

This enables animals to be produced which have a higher value as food, a higher market value, animals which taste better, animals which may be used in different cooking methods, animals which are more nutritionally valuable. Particularly as a human food source.

The present invention will now be described by way of examples.

The following examples are intended to illustrate, but not limit, the invention.

**EXAMPLES**

In the following Examples the following Measurement Terms are used.

**Measurement terms**

The term "FCR" stands for "feed conversion ratio" as used herein. This is a measure of feed efficiency and is calculated as follows: FCR (Feed Conversion Ratio) = FI / BWG, where FI = feed intake of the animal over a specified time period, and BWG = gain in body weight over the same period. BWG is calculated by subtracting the start weight of the animal from the weight of the animal at the end of the trial period. The feed conversion ratio is essentially how many kilograms of feed needed to be consumed per kg of weight gain. This is calculated by taking the total amount of feed consumed (in kg, either for a given period or over the whole trial) and dividing it by the bodyweight gain (in kg, either for the given period or over the whole trial).

Phytase enzyme activity is expressed at FTU/kg feed. One unit of phytase (FTU) is defined as the quantity of enzyme that releases 1 micromol of inorganic phosphorus/min from 0.00015 mol/L of sodium phytate at pH 5.5 at 37 degrees C (Denbow, L. M., V. Ravindran, E. T. Kornegay, Z. Yi, and R. M. Hulet. 1995. Improving phosphorus availability in soybean meal for broilers by supplemental phytase. Poult. Sci.74:1831-1842).

The term "AME" stands for "apparent metabolisable energy" and is an estimate of the available energy in feedstuffs. The AME is calculated as follows: AME = IE - FE - UE, where IE = Gross Energy Ingested by the animal, FE is the Gross energy remaining in the faeces, and UE is the urinary energy (Sibbald, I.R. 1980. BioScience, Vol. 30, No. 11, Nov., 1980).

The term "AMEn" refers to the apparent metabolisable energy corrected for nitrogen

"Calorie conversion" is the number of calories the animal consumed for every kg of bodyweight gain (expressed as kcal/kg). The number if calculated by taking the feed intake (in kg, either for a given period or over the whole trial) and multiplying it by the respective diet ME content (jn kcals/kg), giving the total number of calories consumed. This is then divided by the bodyweight gain (in kg) to give calorie conversion. The "calorie conversion" is equal to AME consumed by the animal over a specified period / body weight gain. Calorie conversion is used as a measure of the efficiency of feed energy utilization. The smaller the number, the more efficient the animal. By adding enzymes results in an improvement (lowers) calorie conversion.

The terms "FCE" or "G:F" stand for "Feed Conversion Efficiency", which is how many kilograms of weight gain are achieved per kilogram of feed consumed. This is calculated by taking the bodyweight gain (in kg, either for a given period or over the whole trial) and dividing it by the amount of feed consumed (in kg, either for the given period or over the whole trial).

The terms "digestibility coefficients" and "coefficient of digestibility" are used interchangeably and are calculated by the amount of a nutrient that is ingested / the amount of that nutrient remaining in the digesta (either ileal digesta or fecal digesta). If the digestibility coefficient is x 100 it can also be called "percent digestibility".

The term "ADG" stands for "Average Daily Gain", calculated by dividing the total weight gain for the trial period by the number of days.

The term "ADFI" stands for "Average Daily Feed Intake", which is calculated by dividing the total feed intake for the trial period by the number of days of the trial.

The term "DE" stands for "digestible energy". This is calculated by multiplying the energy of the diet by the GE digestibility.

The term "GE" stands for "Gross Energy".

The term "CP" stands for crude protein.

The term "DM" stands for "Dry Matter".

The term "AA" stands for "amino acid".

The term "NC" stands for "negative control".

The term "PC" stands for "positive control".

The term "DE" stands for "digestible energy", which is the proportion of the potential energy in a feed which in fact digested.

The term "CP" stands for "crude protein".

The term "EAA stands for "essential amino acids". For example in fish these are arginine, histidine, isoleucine, leucine, lysine, methionine, phenylalanine, threonine, tryptophan and valine.

The term "DCP" stands for "DiCalcium Phosphate" which can be used as a diet supplement.

Wherein linear and quadratic effects are referred to in the Examples, this refers to whether the response to increasing the enzyme dose is a straight line (linear) or has curvature (quadratic effects).

In the following Examples the following enzymes are studied:

BP17 - supplied by Danisco Animal Nutrition

*Peniophora lycii* phytase (sometimes referred to as Phytase R) - supplied by DSM. This phytase is sold under the trade name Ronozyme® P/Bio-Feed® Phytase/ZY®. The phytase is a 6-phytase produced by *Aspergillus oryzae* carrying a gene coding for phytase from *Peniophora lycii.*

Phyzyme® XP (*E. coli* phytase) - supplied by Danisco Animal Nutrition.

**Phytase assay in feed (FTU/kg feed)**

Phytase enzyme is extracted from feed, using a dilute sodium acetate buffer, and the extract is filtered. The extracts are incubated at 37°C for exactly 5 minutes with a sodium phytate (dodecasodium salt, from rice (P3168), Sigma Chemical Company) solution in sodium acetate buffer, containing calcium chloride and a trace of Tween 20, at 37°C and pH 5.5, for 60 minutes. The enzyme incubation is stopped, and the released phosphate determined, by the addition of a molybdo-vanadate reagent, and the absorbance is measured at 415nm. A calibration curve, generated by incubation of 0 to 4 mM (0 to 4 $\mu$mole/ml) potassium dihydrogen phosphate solutions under the same conditions, is used to calculate the phytase activity.

**Example 1: Performance in animal feed in broiler chicken**

The performance of BP17 in animal feed was evaluated in a broiler chicken growth trial with the following parameters: digestibility of phosphate, calcium, nitrogen, amino acids, energy and weight gain, feed intake, calorie conversion and feed conversion ratio (FCR), as described above.

1.1 Material and Methods

192 male day old broiler chicks were allocated to 4 treatments with 6 replicate cages per treatment (8 birds/cage). The birds were fed a commercial control diet from days 0-4, the treatment diets were fed from days 5-21. The control diet was based on corn and soybean meal (48% CP). The control diet was formulated to have a Ca:AvP (Calcium:available phosphate ratio) ratio of 2.14, by altering the Ca inclusion this was fed un-supplemented or supplemented with 500 or 1,000 FTU/kg feed BP17 or with 1,850 FTU/kg feed of the commercial *P.lycii* phytase (Phytase R). All diets were fed as mash *ad libitum.*

On day 21 faeces were collected for determination of apparent metabolisable energy (AME, as described above), total tract; nitrogen, phosphorus and calcium digestibility. Also on day 21 all birds were euthanased and ileal contents collected to determine the digestibility of amino acids using an inert dietary marker (titanium dioxide).

Table 1.1 Diets (kg/tonne) as fed

| Ingredients | Control |
|---|---|
| Maize | 613 |
| Soybean Meal 48%CP | 342 |
| Soybean Oil | 10.1 |
| L-Lysine HCl | 3.0 |
| DL-methionine | 3.1 |
| L-Threonine | 1.2 |
| Salt | 3.3 |
| Limestone | 8.3 |
| Dicalcium Phosphate | 9.8 |
| Trace mineral/ vitamin premix | 3.0 |
| BP17 (FTU/kg feed) | 0/500/1000 |
| *Peniophora lycii* phytase (FTU/kg feed) | 0/1850 |
| Indigestible marker | 3 |
| **Calculated analysis** | |
| Crude protein (%) | 22 |
| Metabolisable energy (MJ/kg) | 12.5 |
| Metabolisable energy (kcal/kg) | 2990 |
| Calcium (%) | 0.60 |
| Total P (%) | 0.55 |
| Lysine (%) | 1.40 |
| Digestible lysine (%) | 1.24 |
| Methionine (%) | 0.64 |
| Digestible methionine (%) | 0.62 |
| Methionine + Cysteine (%) | 1.00 |
| Digestible methionine + cysteine (%) | 0.87 |
| Available phosphorus (%) | 0.28 |
| Sodium (%) | 0.16 |
| Ca:AvP | 2.14 |

1.2 Results

Table 1.2: Ileal amino acid digestibility (%) at 21 days of age

| | Control | BP17 500FTU | BP17 1.000FTU | Phytase R 1,850FTU |
|---|---|---|---|---|
| Threonine | 62.6[b] | 74.3[a] | 77.3[a] | 74.0[a] |
| Valine | 64.1[b] | 77.9[a] | 77.7[a] | 77.9[a] |
| Methionine | 87.3[b] | 92.3[a] | 93.7[a] | 92.1[a] |
| Isoleucine | 69.4[b] | 80.5[a] | 83.0[a] | 80.8[a] |
| Leucine | 69.9[b] | 81.1[a] | 83.5[a] | 81.9[a] |
| Phenylalanine | 71.6[b] | 81.9[a] | 84.3[a] | 82.4[a] |
| Histidine | 70.3[b] | 81.4[a] | 82.9[a] | 81.4[a] |
| Arginine | 79.2[b] | 86.4[a] | 90.1[a] | 86.6[a] |
| Serine | 65.3[b] | 78.2[a] | 81.9[a] | 77.5[a] |
| Glutamic acid | 78.6[c] | 85.9[b] | 88.9[a] | 86.3[b] |
| Proline | 70.4[b] | 80.0[a] | 82.2[a] | 80.3[a] |
| Glycine | 62.0[b] | 74.7[a] | 77.6[a] | 74.8[a] |
| Tyrosine | 73.2[b] | 82.0[a] | 84.7[a] | 81.2[a] |

(continued)

| | Control | BP17 500FTU | BP17 1.000FTU | Phytase R 1,850FTU |
|---|---|---|---|---|
| Alanine | $67.2^b$ | $79.6^a$ | $82\text{-}0^a$ | $80.2^a$ |
| Lysine | $77.2^c$ | $85.2^b$ | $89.5^a$ | $85.8^{ab}$ |
| Cyctine | $51.9^c$ | $66.2^{ab}$ | $69.3^a$ | $62.1^b$ |
| Aspartic acid | $79.9^c$ | $81.4^b$ | $85.9^a$ | $81.0^b$ |
| All amino acids | $72.3^b$ | $82.0^a$ | $84.9^a$ | $82.1^a$ |

*abc Values without a common superscript are significantly different (P<0.05). Only values that have no common superscript are significantly different All values in the same column will be assigned superscripts (a,b,c), but only values that have no common superscript differ statistically. Stated another way, all values that have an "a" are not different.*

### 1.2.1 Amino acid digestibility

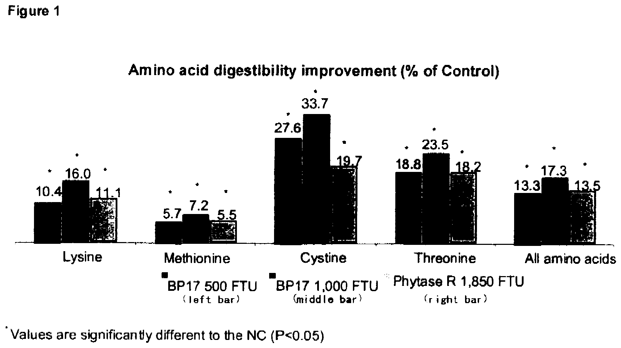

The results shown in Table 1.2 and Figure 1 demonstrate that the BP17 phytase increases the digestibility of various amino acids in broiler chicken compared to the competitor's phytase product. Average amino acid digestibility was improved by 13.3% and 17.3% with 500 FTU and 1000 FTU/kg of BP17.

### 1.2.2 Digestibility of minerals and phytate hydrolysis

The results of the mineral digestibility and the phytate hydrolysis are shown in Table 1.3. The BP17 phytase improved the phosphate digestibility in chicken by 10% compared to the control. The digestibility of calcium is increased by at least 11% compared to the control and the commercial *P. lycii* product when BP17 is included in the chicken diet. AME was improved by 90kcals (2.8%) versus the control diet.

The phytate degradation was increased by 86% compared to the control and the commercial P. *lycii* product when BP17 is used in the chicken diet.

### 1.2.3 Performance

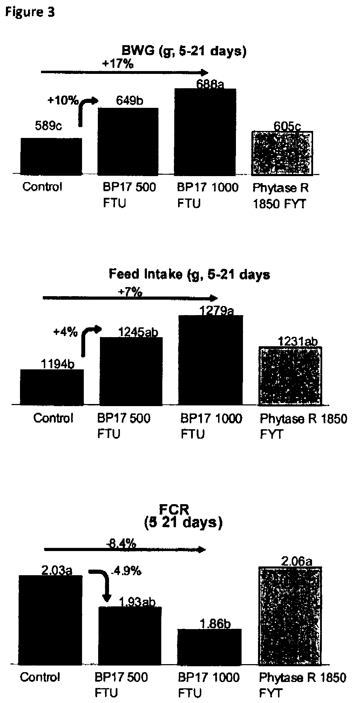

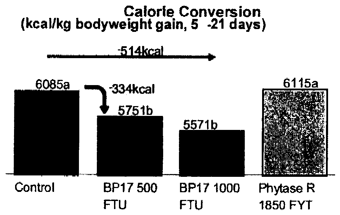

The results of the performance trial are shown in Table 1.3. The BP17 phytase improved the weight gain (BWG) of the chicken by 17% compared to the control (Figure 3). The feed intake of the chicken receiving the BP17 phytase increased by 7% compared to the chicken in the control group. The feed conversion ratio (FCR) was significantly improved (reduction of the FCR by 8.4%) by the BP17 phytase compared to the control. The calorie conversion was improved by 514 kcal of the chicken receiving the BP17 phytase compared to the control.

### 1.2.4 Total tract digestibility

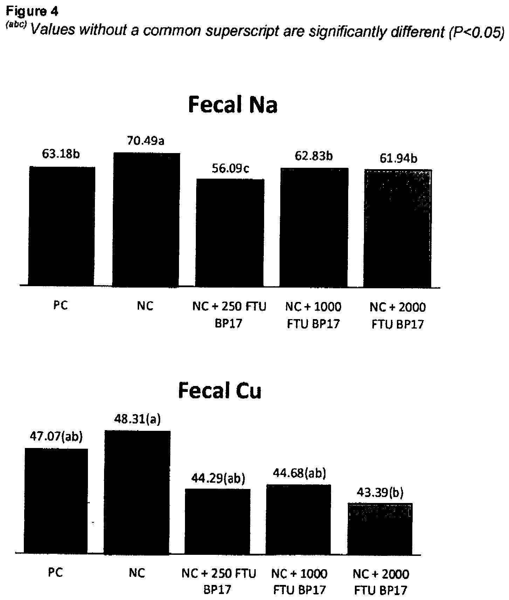

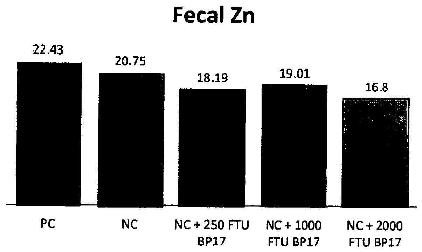

The results of the tract digestibility trial are shown in Figure 4 and in Table 1.3. The BP17 phytase reduced the secretion of sodium (by 11%), copper and zinc (by 7.5% and 8.4% respectively), and thus increase the digestibility of these essential minerals or trace elements in broiler chicken.

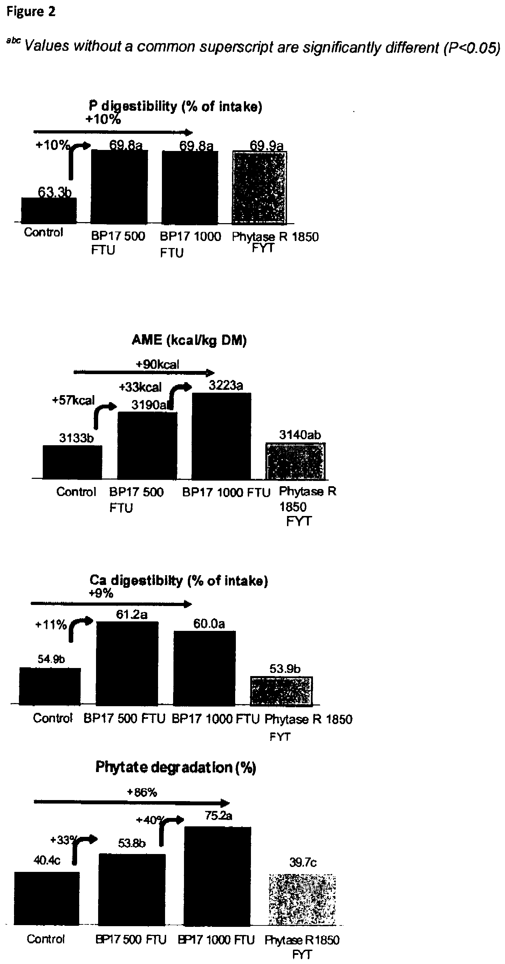

Table 1.3 Results (also shown in Figures 2, 3 and 4)

| | Control | BP17 500 FTU | BP17 1,000 FTU | Phytase R 1,850 FTU |
|---|---|---|---|---|
| **BWG (g)** | $589^c$ | $649^b$ | $688^a$ | $605^c$ |
| **Feed Intake (g)** | $1194^b$ | $1245^{ab}$ | $1279^a$ | $1231^{ab}$ |
| **FCR** | $2.03^a$ | $1.93^{ab}$ | $1.86^b$ | $2.06^a$ |
| **Feed Intake** | $1194^b$ | $1245^{ab}$ | $1279^a$ | $1231^{ab}$ |
| **Calorie Conversion** | $6085^a$ | $5751^b$ | $5571^b$ | $6115^a$ |
| **Ileal DM Digestibility, %** | $63.73^b$ | $69.40^a$ | $72.76^a$ | $71.00^a$ |
| **Ileal GE Digestibility, %** | $64.61^b$ | $71.63^a$ | $74.09^a$ | $72.95^a$ |
| **Fecal DM, %Digestibility** | 75.88 | 76.76 | 78.43 | 77.59 |

(continued)

|  | Control | BP17 500 FTU | BP17 1,000 FTU | Phytase R 1,850 FTU |
|---|---|---|---|---|
| **AME kcal/kg DM** | 3133[b] | 3190[ab] | 3223[a] | 3140[ab] |
| **Fecal N Digestibility, %** | 69.75[a] | 67.91[ab] | 71.93[a] | 65.40[b] |
| **Fecal P Digestibility, %** | 63.3[b] | 69.8[a] | 69.8[a] | 69.9[a] |
| **Fecal Ca Digestibilty, %** | 54.9[b] | 61.2[a] | 60.0[a] | 53.9[b] |
| **Fecal GE Digestibility, %** | 68.2 | 68.8 | 67.4 | 68.2 |
| **Fecal Na digestibility, %** | 70.49[a] | 56.09[c] | 62.83[b] | 61.94[b] |
| **Fecal Cu digestibility, %** | 48.31[(a)] | 44.29[(ab)] | 44.68[(ab)] | 43.39[(b)] |
| **Fecal Zn digestibility, %** | 20.75 | 18.19 | 19.01 | 16.8 |
| **Phytate Degradation, %** | 40.4[c] | 53.8[b] | 75.2[a] | 39.7[c] |
| *abc Values without a common superscript are significantly different (P<0.05)* | | | | |

## Example 2: Bone mineralisation in broiler chicken

The purpose of this example was to demonstrate the effect of BP17 on the bone mineralisation in broiler chicken.

2.1 Material and Methods

250 male 7-day old broiler chicks were allocated to 5 treatments with 10 replicate cages per treatment (5 chicks/cage). The positive control (PC) and negative control (NC) diets were based on corn and soybean meal (48% CP, %CP = Crude Protein expressed as a %. Crude Protein = analyzed Nitrogen x 6.25). The trial ran for 14 days. The NC diet was reduced in available phosphorus by 0.16%, diets were modified by removing 9.3g/kg feed of monocalcium phosphate. Negative control diets were fed un-supplemented or supplemented with 250FTU, 1,000FTU or 2,000FTU/kg feed BP17. All diets were fed as mash *ad libitum.*

Table 2.1: Diets: (kg/tonne) as fed

| Ingredients | Positive Control | Negative Control |
|---|---|---|
| Corn | 594 | 619 |
| Soybean Meal 48%CP | 331 | 325 |
| Soybean Oil | 29.4 | 18.6 |
| L-Lysine HCl | 0.52 | 0.24 |
| DL-methionine | 2.31 | 2.31 |
| L-threonine | 2 | 2 |
| Salt | 2.8 | 2.9 |
| Limestone | 12.3 | 14 |
| Monocalcium phosphate | 16.2 | 6.9 |
| Trace mineral/ vitamin premix | 3.5 | 3.5 |
| BP17 (FTU/kg feed) | - | -/250/1,000/2,000 |
| **Calculated analysis** | | |
| Crude protein (%) | 21 | 21 |
| Metabolisable energy (MJ/kg) | 12.8 | 12.6 |
| Metabolisable energy (kcal/kg) | 3060 | 3010 |
| Calcium | 0.99 | 0.83 |
| Total P (%) | 0.67 | 0.50 |
| Available phosphorus (%) | 0.40 | 0.24 |
| Digestible lysine (%) | 0.98 | 0.95 |
| Methionine (%) | 0.55 | 0.55 |

(continued)

| Calculated analysis | | |
|---|---|---|
| Digestible methionine (%) | 0.51 | 0.51 |
| Methionine + cysteine (%) | 0.90 | 0.90 |
| Digestible methionine + cysteine (%) | 0.79 | 0.79 |
| Sodium (%) | 0.20 | 0.20 |

On day 21 faeces were collected for determination of AME, total tract; nitrogen, phosphorus and calcium digestibility. Also on day 21 all birds were euthanased and ileal contents collected to determine the digestibility of amino acids using an inert dietary marker (titanium dioxide). Bone mineralisation was determined by measuring tibia ash content and expressing this as a percentage of dry tibia weight.

2.2 Results

The results of the bone mineralisation are shown in Table 2.2. The BP17 phytase improved the Tibia ash significantly compared to NC (+10%) and all BP17 phytase treatments have similar tibia ash content as found for PC. Tibia ash and bone ash is used to estimate bone mineralisation (bone strength). A higher value indicates stronger bones.

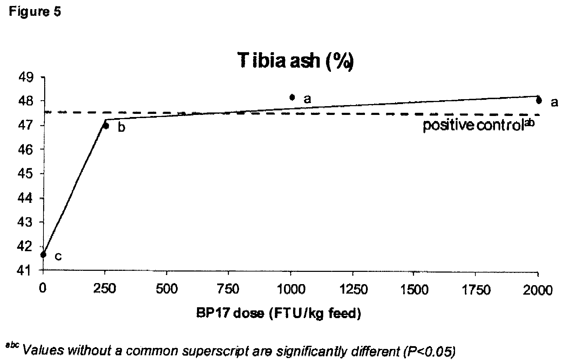

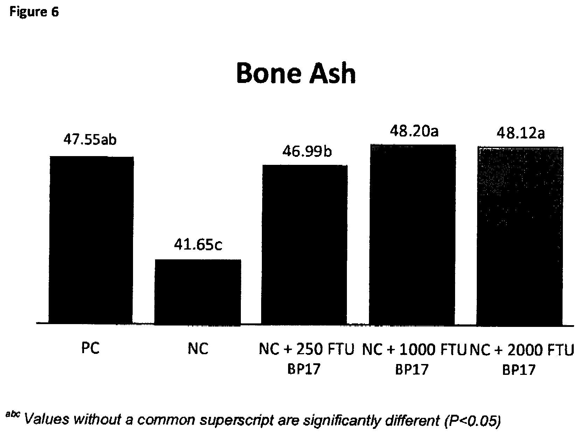

Table 2.2 Effects of BP17 on Bone Ash (%) (also shown in Figures 5 and 6)

| Treatment | Bone Ash (%) |
|---|---|
| Positive Control | 47.55[ab] |
| Negative Control (NC) | 41.65[c] |
| NC + 250 FTU/kg BP17 | 46.99[b] |
| NC + 1000 FTU/kq BP17 | 48.20[a] |
| NC + 2000 FTU/kq BP17 | 48.12[a] |

**Example 3: Performance in animal feed in turkey**

The performance of BP17 in animal feed was evaluated in a Turkey growth trial with the following parameters: digestibility of phosphate, calcium, nitrogen, protein and energy, and weight gain, feed intake, calorie conversion, FCR and Tibia ash.

3.1 Materials and methods

720 male 7-day old turkey poults were allocated to 8 treatments with 9 replicates pens per treatment (10 poults/cage). Treatment diets were fed from days 7-28. The control diet was based on corn and soybean meal (48% CP). The negative control diet was reduced in available phosphorus by 0.12% this was achieved through removing dicalcium phosphate. The negative control diet was fed un-supplemented or supplemented with 250, 1,000 or 2,000 FTU/kg feed BP17 phytase or 250, 500 or 1,000 FTU/kg feed Phyzyme XP phytase. All diets were fed pelleted *ad libitum.*

Feed intake was measured daily throughout the treatment period, poult weight was measured on days 7 and 28 these measurements were used to calculate the performance parameters. Faeces were collected from days 24-28 and analysed for total tract digestibility calculations. On day 28, 4 poults per pen were euthanased and the right tibia collected for determination of bone ash.

Table 3.1: Experimental design

| Treatment | Level |
|---|---|
| 1 | Positive Control |
| 2 | Negative Control |
| 3 | BP17 250 FTU/kg |
| 4 | BP17 1,000 FTU/kg |

(continued)

| Treatment | Level |
|---|---|
| 5 | BP17 2,000 FTU/kg |
| 6 | *E. coli* Phytase 250 FTU/kg |
| 7 | *E. coli* Phytase 1,000 FTU/kg |
| 8 | *E. coli* Phytase 2,000 FTU/kg |

Table 3.2: Diet composition

| Ingredients | Positive Control | Negative Control |
|---|---|---|
| Corn | 324 | 324 |
| Corn gluten meal | 60 | 60 |
| Soybean (full fat) | 72 | 72 |
| Soybean Meal | 350 | 350 |
| Rapeseed Meal | 50 | 50 |
| Sunflower Seed Meal | 10 | 10 |
| Fish Meal | 10 | 10 |
| Corn Starch | 12.16 | 12.16 |
| Soybean Oil | 45 | 45 |
| L-Lysine HCl | 3.6 | 3.6 |
| DL-methionine | 2.6 | 2.6 |
| L-Threonine | 0.2 | 0.2 |
| L-Tryptophan | 0.4 | 0.3 |
| Sodium Bicarbonate | 3.1 | 3.1 |
| Salt | 1.5 | 1.5 |
| Limestone | 13.6 | 14.2 |
| Monocalcium Phosphate | 5.3 | 5.3 |
| Dicalcium phosphate | 6.436 | 0 |
| Trace mineral/ vitamin premix BP17/Phyzyme XP (FTU/kg feed) | 5<br>- | 5<br>-/250/1,000/2,000 |
| **Calculated analysis** | | |
| Crude protein (%) | 26 | 26 |
| Metabolisable energy (MJ/kg) | 12.2 | 12.2 |
| Metabolisable energy (kcal/kg) | 2915 | 2915 |
| Calcium (%) | 1.00 | 0.87 |
| Total P (%) | 0.73 | 0.61 |
| Available phosphorus (%) | 0.42 | 0.30 |
| Lysine (%) | 1.76 | 1.76 |
| Digestible lysine (%) | 1.52 | 1.52 |
| Methionine (%) | 0.67 | 0.67 |
| Digestible methionine (%) | 0.61 | 0.61 |
| Methionine + Cysteine (%) | 1.10 | 1.10 |
| Digestible methionine + Cysteine (%) | 0.94 | 0.94 |
| Sodium (%) | 0.19 | 0.19 |

3.2 Results

The results of the study in turkey are shown in Table 3.3. At all dose levels the BP17 phytase resulted in a higher BWG, a higher feed intake and a lower FCR and lower calorie conversion compared to the Phyzyme (*E. coli*)

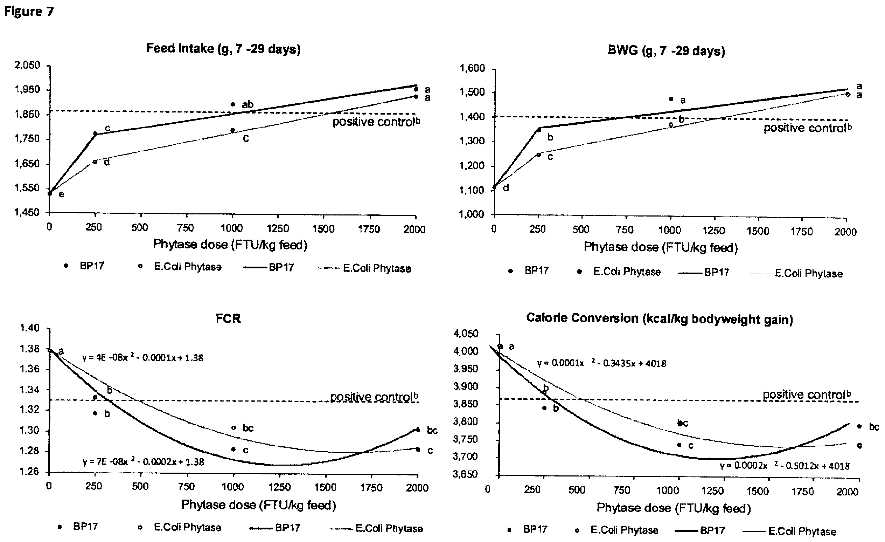

phytase (Figure 7). Addition of BP17 phytase at 250-2000 FTU/kg to the negative control diet resulted in better BWG (+ 21 to 36%), FCR (4.3 to 5.7% improvement), calorie conversion (175 kcals to 220 kcals/kg), P digestibility (+ 22 to 41%), Ca digestibility (+ 23.5 to 46.5%) and Tibia Ash (+ 13.4 to 33.4%).

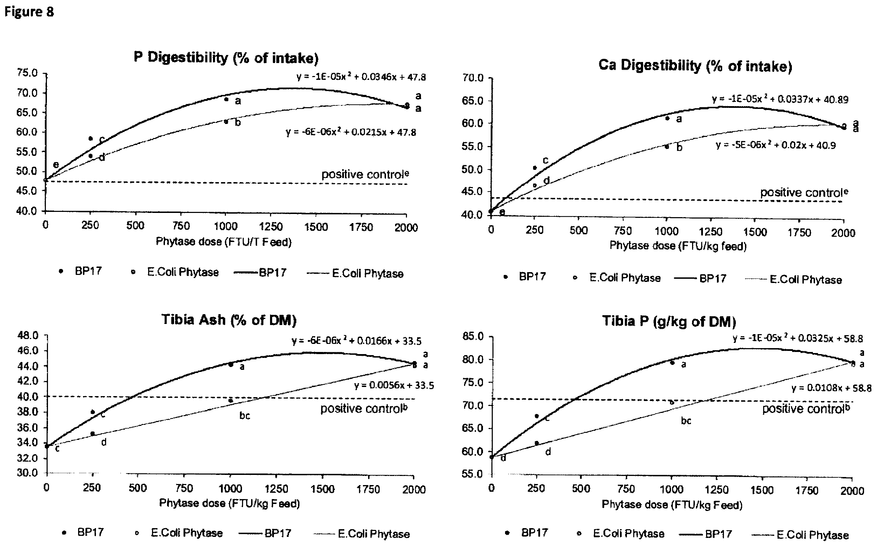

At all dose levels the BP17 phytase resulted in a higher phosphous and calcium digestibility and an increase in tibia ash content and tibia P compared to the *E. coli* Phytase (Figure 8).

It is concluded that based on the *in vivo* efficacy in turkeys between the phytases can be highly different.

Table 3.3: Results: Performance, digestibility and tibia analysis results over 21 day trial (also shown in Figures 7, 8 and 9).

| | | Phytase FTU/kg feed | | | | |
|---|---|---|---|---|---|---|
| | | PC | NC | 250 | 1,000 | 2,000 |
| Feed Intake (g) | | 1866[b] | 1530[e] | 1775[c] | 1899[ab] | 1966[a] |
| | *E. coli* Phytase | | | 1660[d] | 1792[c] | 1936[a] |
| BWG (g) | BP17 | 1403[b] | 1112[d] | 1348[b] | 1481[a] | 1510[a] |
| | *E. coli* Phytase | | | 1246[c] | 1376[b] | 1508[a] |
| FCR | BP17 | 1.33[b] | 1.38[a] | 1,32[c] | 1.28[c] | 1.30[bc] |
| | *E. coli* Phytase | | | 1.33[b] | 1.30[bc] | 1.28[c] |
| Calorie Conversion (kcal/kg) | BP17 | 3869[b] | 4018[a] | 3843[b] | 3741[c] | 3798[bc] |
| | *E. coli* Phytase | | | 3886[b] | 3802[bc] | 3744[c] |
| P Digestibility (%) | BP17 | 47.6[e] | 47.8[e] | 58.4[c] | 68.7[a] | 67.2[a] |
| | *E. coli* Phytase | | | 54.0[d] | 63.0[b] | 67.9[a] |
| Ca Digestibility (%) | BP17 | 43.8[c] | 40.9[e] | 50.5[c] | 61.6[a] | 59.9[a] |
| | *E. coli* Phytase | | | 46.6[d] | 55.3[b] | 60.5[a] |
| Tibia Ash (%) | BP17 | 40.1[b] | 33.5[c] | 38.0[c] | 44.3[a] | 44.7[a] |
| | *E. coli* Phytase | | | 35.2[d] | 39.6[bc] | 44.3[a] |
| Tibia P (g/kg) | BP17 | 71.4[b] | 58.8[d] | 67.8[c] | 79.7[a] | 80.2[a] |
| | *E. coli* Phytase | | | 61.9[d] | 70.9[bc] | 79.6[a] |
| Digestible P Contribution | BP17 | - | 0.73* | 0.0647 | 0.1275 | 0.1183 |
| | *E. coli* Phytase | | | 0.0378 | 0.0927 | 0.1226 |
| GE | BP17 *E. coli* Phytase | 70.4[c] | 71.9[a] | 71.6[ab] | 71.4[ab] | 71.5[ab] |
| | | | | 71.2[abc] | 71.5[ab] | 70.9[bc] |
| AMEn | BP17 *E. coli* Phytase | 3090[c] | 3153[a] | 3137[ab] | 3128[ab] | 3133[ab] |
| | | | | 3120[abc] | 3134[ab] | 3107[bc] |
| Tibia Ca | BP17 *E. coli* Phytase | 145[b] | 119[d] | 136[c] | 159[a] | 159[a] |
| | | | | 131[c] | 142[b] | 159[a] |
| *contribution of additional digestible P in comparison to NC for phytase treatments | | | | | | |

## Example 4: Performance in animal feed in turkey

The performance of BP17 phytase in animal feed was evaluated in a turkey growth trial with the following parameters: digestibility of copper.

4.1 Materials and methods

336 male turkeys were allocated 7 treatments with 12 replicate pens per treatment (4 per cage). Treatment diets were fed from days 7-23. The positive control diet was based on wheat and soybean meal formulated with 0.98% P and 1.2% Ca inclusions. The negative control diet was a wheat + soybean meal diet with an inclusion of 0.82% P and 1.05% Ca. The negative control diet was fed un-supplemented or supplemented with 250, 500, 750, 1,000 or 2,000 FTU/kg feed BP17. All diets were fed pelleted *adlibitum.*

Feed intakes were measured and recorded for day started, daily feed intake and refusals (data not shown). Ileal digesta was collected from each bird for analysis.

Table 4.1: Experimental design

| Treatment | Level |
|-----------|-------|
| 1 | Positive Control |
| 2 | Negative Control |
| 3 | BP17 250 FTU/kg |
| 4 | BP17 500 FTU/kg |
| 5 | BP17 750 FTU/kg |
| 6 | BP17 1000 FTU/kg |
| 7 | BP17 2000 FTU/kg |

Table 4.2: Diet composition (%, as fed)

| Ingredients: Kg/tonne | Positive control - starter | Negative control - starter |
|-----------------------|----------------------------|----------------------------|
| Wheat | 48.0 | 49.9 |
| Soybean meal -48 | 43.61 | 43.07 |
| Soya oil | 3.0 | 2.5 |
| salt | 0.30 | 0.30 |
| MCP | 2.43 | 1.70 |
| Limestone | 1.45 | 1.36 |
| Vitamin premix | 0.50 | 0.50 |
| Lysine-HCl | 0.168 | 0.158 |
| DL-Methionine | 0.217 | 0.204 |
| L-threonine | 0.026 | 0.030 |
| TiTO2 | 0.3 | 0.3 |
| **Total** | **100** | **100** |
| **Nutrient Composition** | | |
| Crude Protein | 26.66 | 26.60 |
| Poult ME kcal/kg | 2844 | 2842 |
| Ca, % | 1.2 | 1.05 |
| P, % | 0.98 | 0.82 |
| Av. P, % | 0.70 | 0.55 |
| Lys% | 1.59 | 1.57 |
| Met % | 0.59 | 0.58 |
| Met + Cys % | 1.03 | 1.02 |
| Na, % | 0.19 | 0.19 |

4.2 Results

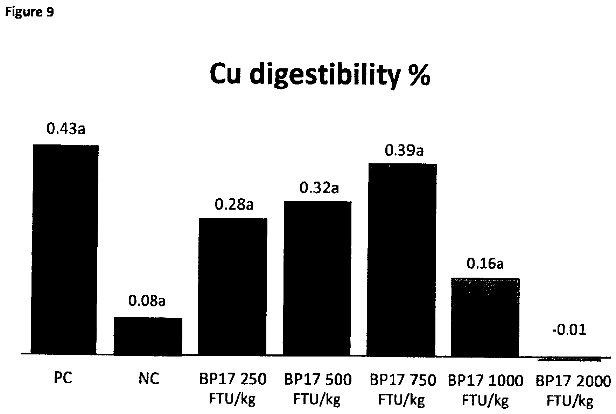

Mineral digestibility is shown in Table 4.3 and Figure 9. For Cu an increase in digestibility was found (x 4.8 compared to the negative control), by adding BP17 phytase.

Table 4.3: Copper digestibility

| Level | Cu Digestibility (%) |
|---|---|
| Positive Control | 0.43 |
| Negative Control | 0.08 |
| BP17 250 FTU/kg | 0.28 |
| BP17 500 FTU/kg | 0.32 |
| BP17 750 FTU/kg | 0.39 |
| BP17 1000 FTU/kg | 0.16 |
| BP17 2000 FTU/kg | 0.01 |

**Example 5: Performance in animal feed in layers**

The performance of BP17 in animal feed was evaluated in a layer growth trial with the following parameters: digestibility of phosphate, calcium, nitrogen, sodium and energy.

5.1 Materials and Methods

In a layer digestibility trial 96 layers were allocated to one of four treatments, with twelve replicates per treatments. Layers were housed in pairs of two per cage. The positive control diet was based on wheat, barley, soybean meal and rapeseed meal formulated with 0.80% P and 4.0% Ca inclusions. The negative control diet was wheat, barley, soybean meal and rapeseed meal based diet with an inclusion of 0.36% P and 4.23% Ca. The negative control diet was fed un-supplemented or supplemented with 250 or 2,000 FTU/kg feed BP17. All diets were fed as meal *ad libitum.* Diets were fed for 6 weeks. Faeces samples were collected for 39-42.

Table 5.1 Study design

| Treatment | Description | Inclusion (g/MT) | No. of replicates | No of birds/Replicate |
|---|---|---|---|---|
| **1** | **Positive Control (PC)** | 0 | 12 | 24 |
| **2** | **Negative Control (NC)** | 0 | 12 | 24 |
| **3** | **NC + 250 FTU/kg BP17 phytase** | 500 | 12 | 24 |
| **4** | **NC + 2,000 FTU/kg BP17 phytase** | 500 | 12 | 24 |
| *Note: Inclusion rate of enzyme sample for each treatment was 500g/tonne and the enzyme had a different activity.* | | | | |

Table 5.2 Dietary composition (as fed basis) of the Positive (PC) and Negative control (NC) diets

| Ingredients | Positive control | Negative control |
|---|---|---|
| Wheat (11.3%CP) | 48.4 | 50.9 |
| Barley | 20 | 20 |
| Soybean Meal 48%CP | 5.3 | 4.7 |
| Rapeseed Meal | 5 | 5 |
| Maize gluten meal (60%) | 7.5 | 7.5 |
| Soybean Oil | 2.3 | 1.6 |

(continued)

| Ingredients | Positive control | Negative control |
|---|---|---|
| L-Lysine HCl | 0.364 | 0.377 |
| DL-methionine | 0.101 | 0.101 |
| L-threonine | 0.004 | 0.004 |
| Salt | 0.31 | 0.30 |
| Dicalcium Phosphate | 1.75 | 0.0 |
| Limestone | 8.18 | 8.76 |
| Vit and Min. | 0.5 | 0.5 |
| Titanium Dioxide | 0.3 | 0.3 |

Table 5.3: Determined dietary analysis

| Treatment | DM | AHEE | CP | NDF | P | Ca | N |
|---|---|---|---|---|---|---|---|
| | g/kg | g/kg DM | g/kgDM | g/kgDM | g/kgDM | g/kgDM | g/kgDM |
| PC | 873.7 | 54.8 | 182 | 123 | 8.02 | 40.2 | 1.70 |
| NC | 871.9 | 47.9 | 188 | 112 | 4.14 | 48.5 | 1.77 |
| BP17, 250 | 877.7 | 49.7 | 187 | 105 | 3.90 | 54.1 | 2.05 |
| BP17, 2,000 | 873.3 | 50.0 | 186 | 113 | 4.44 | 39.5 | 1.47 |
| *Where DM=Dry Matter, AHEE=Acid hydrolysed Ether Extract, CP= Crude Protein and NDF= Neutral detergent fibre.* | | | | | | | |

5.2 Results

Layers fed up to 2,000 FTU/kg feed of BP17 improved body weight in comparison to NC (Table 5.4). Improvement of AME (up to 6.7%)), DM, N (up to 11%), Ca (up to 37%), P (up to 86%) and Na digestibility (up to 22%) of adding BP17 to the feed was found (Table 5.5). Furthermore, improvement of retention (g/bird/day) of N (up to 16%), Ca (up to 51%), P (up to 79%) and Na (up to 33%) was found (Table 5.6).

Table 5.4: Effect of different doses of BP17 on body weights of layers during experimental period

| Treatment | Body weights (kg/bird) |
|---|---|
| **Enzymes** | |
| PC | 1813.5b |
| NC | 1745.1a |
| BP17, 250 | 1728.5a |
| BP17, 2,000 | 1828.1b |
| **LSD** | 56.00 |
| **P-Value** | 0.001 |
| **Age (Weeks)** | |
| 21 | 1719.5a |
| 26 | 1838.1b |
| **LSD** | 37.53 |
| **P-Value** | <0.001 |
| **Enzyme*Age** | |

(continued)

| Age (Weeks) | |
|---|---|
| P-Value | 0.468 |
| *There is a statistically significant difference when P<0.05; LSD - Least Significant Differences of Means.* | |

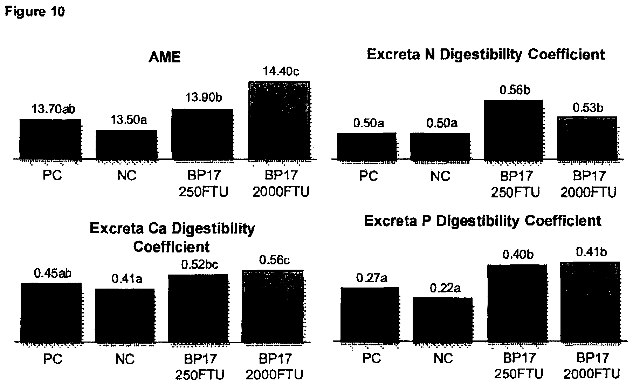

Table 5.5: Effect of different doses of BP17 on excreta dietary apparent metabolisable energy (AME; MJ/Kg DM), dry matter digestibility (DMD), nitrogen digestibility coefficient (NED), calcium digestibility coefficient (CaED), Phosphorous digestibility coefficient (PED) and sodium digestibility coefficient (NaED) (see also Figure 10)

| Treatment | AME | DMD | NED | CaED | PED | NaED |
|---|---|---|---|---|---|---|
| **PC** | 13.7[ab] | 0.717[a] | 0.502[a] | 0.453[ab] | 0.272[a] | 0.562 |
| **NC** | 13.5[a] | 0.717[a] | 0.502[a] | 0.408[a] | 0.218[a] | 0.486 |
| **BP17, 250** | 13.9[b] | 0.746[b] | 0.559[b] | 0.522[bc] | 0.395[b] | 0.594 |
| **BP17, 2,000** | 14.4[c] | 0.740[b] | 0.530[b] | 0.558[c] | 0.406[b] | 0.562 |
| **LSD** | 0.254 | 0.019 | 0.024 | 0.076 | 0.077 | 0.103 |
| **P-value** | <0.001 | 0.021 | <0.001 | 0.003 | <0.001 | 0.201 |
| *There is a statistically significant difference when P<0.05; LSD - Least Significant Differences of Means* | | | | | | |

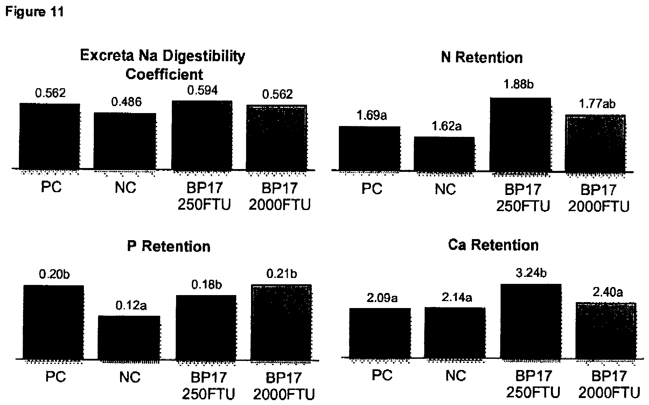

Table 5.6: The effect of different doses of BP17 on the daily retention (g/bird/day) of dietary nitrogen (NR), Calcium (Ca), Phosphorous (PR) and Sodium (NaR) (also shown in Figure 11)

| Treatment | NR | CaR | PR | NaR |
|---|---|---|---|---|
| **PC** | 1.687[a] | 2.094[a] | 0.201[b] | 0.108[a] |
| **NC** | 1.618[a] | 2.143[a] | 0.115[a] | 0.102[a] |
| **BP17, 250** | 1.882[b] | 3.241[b] | 0.176[b] | 0.136[b] |
| **BP17, 2,000** | 1.770[ab] | 2.403[a] | 0.206[b] | 0.095[a] |
| **LSD** | 0.147 | 0.419 | 0.045 | 0.021 |
| **P-value** | 0.008 | <0.001 | 0.002 | 0.004 |
| *There is a statistically significant difference when P<0.05; LSD - Least Significant Differences of Means* | | | | |

### Example 6: Performance in animal feed in layers

The performance of BP17 phytase in animal feed was evaluated in a layer growth trial with the following parameters FCR, Egg mass, Egg weight, and laying rate.

6.1 Material and Methods

Five Hundred and seventy six layers were used in a performance trial. The positive control diet was based on wheat, barley and soybean meal diet formulated with 0.46% P and 3.80% Ca inclusions. The negative control diet was a wheat, barley and soybean meal diet with an inclusion of 0.34% P and 3.65% Ca. The negative control diet was fed un-supplemented or supplemented with 250, 500, 1,000 or 2,000 FTU/kg feed BP17 phytase. Birds were feed ad libitum a meal diet. Feed conversion, egg mass, egg weight and laying rate were recorded during the trial.

Table 6.1: The description of experimental treatments and diet codes

| Trt | Description | Phytase inclusion (FTU/kg) | Diet code |
|---|---|---|---|
| 1 | Negative control (NC) | - | A |

(continued)

| Trt | Description | Phytase inclusion (FTU/kg) | Diet code |
|---|---|---|---|
| 2 | NC plus 1.3 g DCP-P (positive control, PC) | - | B |
| 3 | NC plus 250 FTU BP17 Phytase[1] | 250 | C |
| 4 | NC plus 500 FTU BP17 Phytase | 500 | D |
| 5 | NC plus 1,000 FTU BP17 Phytase | 1,000 | E |
| 6 | NC plus 2,000 FTU BP17 Phytase | 2,000 | F |
| [1] The test product was sprayed on the pelleted diets | | | |

Table 6.2: Feed Composition of the Experimental Diets

| | Neg. Control | Pos. Control |
|---|---|---|
| | Low ret. P | +1.3 g DCP-P |
| | g/kg | g/kg |
| Wheat | 450.00 | 450.00 |
| Barley | 150.00 | 150.00 |
| Soy bean meal (48) | 141.00 | 141.00 |
| Maize gluten meal (60) | 53.00 | 53.00 |
| Wheat middlings | 10.00 | 10.00 |
| Maize starch | 50.00 | 50.00 |
| Animal fat | 17.00 | 17.00 |
| Soya oil | 10.00 | 10.00 |
| Salt | 1.70 | 1.70 |
| NaHCO3 | 0.60 | 0.60 |
| Premix min+vit[2] | 10.00 | 10.00 |
| $Cr_2O_3$[1] | 0.75 | 0.75 |
| Limestone | 88.20 | 88.20 |
| | | |
| Maize starch | 11.314 | 11.314 |
| $CaCO_3$ | 0.594 | 3.000 |
| DCP-anhydrate | 0.000 | 6.436 |
| Diamol | 5.842 | 0.000 |
| | 1000.000 | 1000.000 |

Table 6.3: Analysed and calculated[1] nutrient contents in the experimental diets

| Diet | DM | CP | Ash | Fat | Cr[2] | NDF | GE | P | Ca | Phytate-P |
|---|---|---|---|---|---|---|---|---|---|---|
| | g/kg | g/kg | g/kg | g/kg | g/kg | g/kg | MJ/kg | g/kg | g/kg | g/kg |
| | | | | | | | | | | |
| A | 898 | 185 (185) | 110 | 50 (48) | 0.546 | 91 | 15.59 | 3.4 (3.3) | 33.8 (36.5) | 2.5 (2.1) |
| B | 895 | n.a. | 110 | n.a. | 0.537 | n.a. | n.a. | 4.6 (4.6) | 34.9 (38.0) | 2.6 (2.1) |

(continued)

| Diet | DM | CP | Ash | Fat | Cr[2] | NDF | GE | P | Ca | Phytate-P |
|---|---|---|---|---|---|---|---|---|---|---|
| | g/kg | g/kg | g/kg | g/kg | g/kg | g/kg | MJ/kg | g/kg | g/kg | g/kg |
| C | 900 | n.a. | n.a. | n.a. | 0.532 | n.a. | n.a. | 3.4 | 33.5 | n.a. |
| D | 901 | n.a. | n.a. | n.a. | 0.536 | n.a. | n.a. | 3.3 | 33.9 | n.a. |
| E | 899 | n.a. | n.a. | n.a. | 0.521 | n.a. | n.a. | 3.3 | 35.1* | n.a. |
| F | 902 | n.a. | n.a. | n.a. | 0.493 | n.a. | n.a. | 3.2 | 33.8 | n.a. |

[1] *Calculated vales are given between brackets*
[2] *Cr Digestibility was calculated with Cr = 0.595 g/kg DM for all diets (average of A to E)*
*n.a. not analysed as all diets were obtained from the same batch of basal diet*
*\* Not included in calculation of the average nutrient content of diet A, C-F*

6.1 Results

Adding BP17 phytase improved the laying rate in comparison to NC (Table 6.3). Furthermore, egg weight, egg mass and feed intake improved by up to 2%, 4% and 4% respectively by adding BP17 phytase to the feed, without any effect on FCR (Table 6.4).

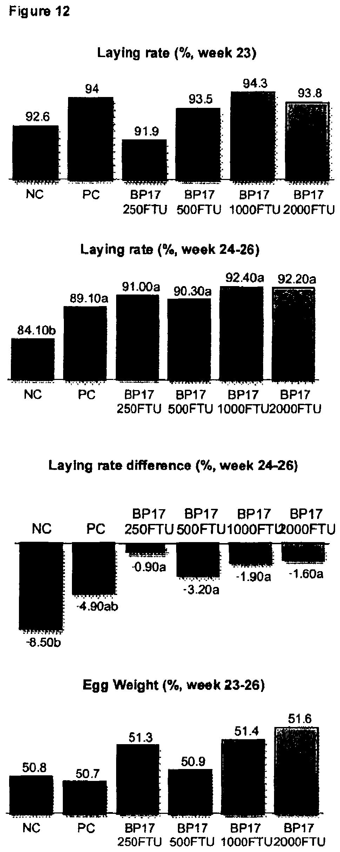

Table 6.4: Results for laying rate of the laying hens (also shown in Figure 12).

| | | | wk 23 | | wk 24-26 | | | |
|---|---|---|---|---|---|---|---|---|
| | | | Laying[1] | | Laying | | Laying | |
| | BP17 | | rate | | rate | | Rate | |
| Trt. | FTU/kg | | % | | % | | Δ (%) | |
| | | | | | | | | |
| 1 | 0 | | 92.6 | | 84.1 | b | -8.5 | b |
| 2 | 0 | | 94.0 | | 89.1 | a | -4.9 | ab |
| 3 | 250 | | 91.9 | | 91.0 | a | -0.9 | a |
| 4 | 500 | | 93.5 | | 90.3 | a | -3.2 | a |
| 5 | 1,000 | | 94.3 | | 92.4 | a | -1.9 | a |
| 6 | 2,000 | | 93.8 | | 92.2 | a | -1.6 | a |
| | | | | | | | | |
| P (trt) | | | NS | | <0.001 | | 0.01 | |
| Lsd (trt) | | | 3.6 | | 3.5 | | 4.5 | |
| | | | | | | | | |
| P (dose, lin) | | | NS | | <0.001 | | 0.049 | |
| P (dose, quad) | | | NS | | 0.002 | | 0.072 | |
| LSD (dose) | | | 3.8 | | 3.4 | | 4.7 | |

*NS: non significant (P≥0.10)*
*a,b Mean values without a common superscript letter within a column are significantly different (P<0.05)*
*Linear and quadratic dose effect was tested excluding PC*

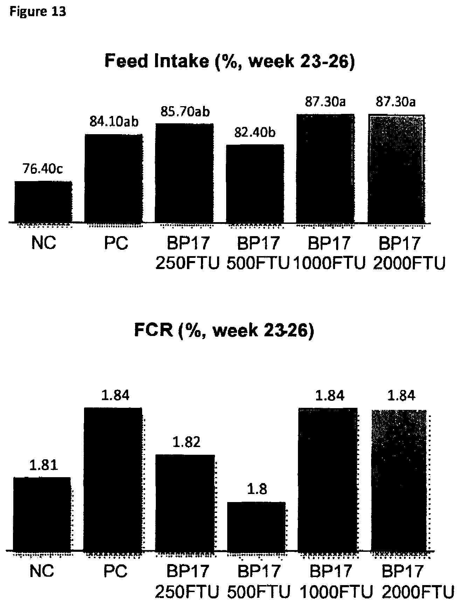

Table 6.5: Results for egg weight, feed intake (FI) and feed conversion ratio (FCR) of the laying hens during the experimental period from 23 to 26 weeks of age (see also Figure 13).

| | BP17 | Egg weight | Egg mass | | FI | | FCR |
|---|---|---|---|---|---|---|---|
| Trt. | FTU/kg | % | g/d | | % | | % |
| | | | | | | | |
| 1 | 0 | 50.8 | 42.2 | b | 76.4 | c | 1.81 |
| 2 | 0 | 50.7 | 45.7 | a | 84.1 | ab | 1.84 |
| 3 | 250 | 51.3 | 47.2 | a | 85.7 | ab | 1.82 |
| 4 | 500 | 50.9 | 45.9 | a | 82.4 | b | 1.80 |
| 5 | 1,000 | 51.4 | 47.5 | a | 87.3 | a | 1.84 |
| 6 | 2,000 | 51.6 | 47.4 | a | 87.3 | a | 1.84 |
| | | | | | | | |
| P (trt) | | NS | <0.001 | | <0.001 | | NS |
| Lsd (trt) | | 0.8 | 1.9 | | 4.15 | | 0.074 |
| | | | | | | | |
| P (dose, lin) | | 0.048 | <0.001 | | <0.001 | | NS |
| P (dose, quad) | | 0.864 | 0.001 | | 0.005 | | NS |
| LSD (dose) | | 0.8 | 2.0 | | 4.29 | | 0.078 |
| NS non significant (P≥0.10) [a,b] Mean values without a common superscript letter within a column are significantly different (P<0.05) Linear and quadratic dose effect was tested excluding PC | | | | | | | |

**Example 7: Performance in animal feed in layers**

The performance of BP17 in animal feed was evaluated in a layer growth trial with the following parameters: FCR, Egg mass, Egg weight, laying rate, egg quality and bird weight

7.1 Material and Methods

720 layers -weeks old layers were allocated to 9 treatments with 16 replicate pens per treatment (5 layers/cage). Treatment diets were fed from week 26-60. The control diet was based on corn and soybean meal (48% CP), the negative control diet was reduced in available phosphorus by 0.20 during stage 1 and by 0.18% during stage 2, this was achieved through removing dicalcium phosphate. The negative control diet was fed un-supplemented or supplemented with 300, 600 or 900 FTU/kg feed BP17 phytase, 300, 600 or 900 FTU/kg feed Phytase XP or 900 FTU/kg feed *P. lycii* Phytase. All diets were fed as meal *ad libitum.*

Excreta were collected for 4 days in week 12 and 24. Performance, including feed intake, weight gain and egg production and egg quality was measured daily throughout the treatment period, were used to calculate the performance parameters.

Table 7.1: Dietary treatments, enzyme identification and incorporation rates

| Trt.* | Description | Inclusion (g/MT) | Excreta to be collected for 4 days at | Excreta to be collected for 4 days at |
|---|---|---|---|---|
| **1** | Positive Control (PC) | 0 | Week 12 | Week 24 |
| **2** | Negative Control (NC) | 0 | Week 12 | Week 24 |
| **3** | NC + 300FTU/kg BP17 | 60 | Week 12 | Week 24 |

(continued)

| Trt.* | Description | Inclusion (g/MT) | Excreta to be collected for 4 days at | Excreta to be collected for 4 days at |
|---|---|---|---|---|
| 4 | NC + 600FTU/kg BP17 | 120 | Week 12 | Week 24 |
| 5 | NC + 900FTU/kg BP17 | 180 | Week 12 | Week 24 |
| 6 | NC + 300FTU/kg Phytase XP | 30 | Week 12 | Week 24 |
| 7 | NC + 600FTU/kg Phytase XP | 60 | Week 12 | Week 24 |
| 8 | NC + 900FTU/kg Phytase XP | 90 | Week 12 | Week 24 |
| 9 | NC + *P. lycii* Phytase (CT) | 90 | Week 12 | Week 24 |

Table 7.2: Diet Formulations

| Layers : 90% prod., 115 g/ d intake | | | | |
|---|---|---|---|---|
| | stage 1 diets | | stage 2 diets | |
| Ingredient | Positive control | Negative Control | Positive control | Negative Control |
| Maize | 57.67 | 59.31 | 57.14 | 58.64 |
| SBM 48 | 22.09 | 21.87 | 22.17 | 21.93 |
| Soya oil | 2.5 | 1.95 | 2.56 | 2.09 |
| Rice Bran | 5.0 | 5.0 | 5.0 | 5.0 |
| Salt | 0.33 | 0.33 | 0.30 | 0.3 |
| Lysine HCL | 0.032 | 0.007 | 0.032 | 0.027 |
| DL Met | 0.167 | 0.165 | 0.169 | 0.166 |
| L-Tryptophan | 0.005 | 0.005 | 0.005 | 0.005 |
| Limestone | 8.54 | 8.86 | 9.14 | 9.40 |
| Dical Phos | 1.26 | 0.10 | 1.03 | 0 |
| Sodium bicarb | 0 | 0 | 0.048 | 0.046 |
| Vit / Min | 0.4 | 0.4 | 0.4 | 0.4 |
| Inert marker AiA | 2.0 | 2.0 | 2.0 | 2.0 |
| **Nutrients** | | | | |
| CP | 16.35 | 16.35 | 16.35 | 16.35 |
| MEP kcal/kg | 2820 | 2818 | 2811 | 2811 |
| Ca% | 3.63 | 3.47 | 3.8 | 3.65 |
| Total P% | 0.62 | 0.41 | 0.58 | 0.39 |
| Av P% | 0.33 | 0.13 | 0.29 | 0.11 |
| Met % | 0.428 | 0.427 | 0.430 | 0.428 |
| Lys % | 0.858 | 0.835 | 0.860 | 0.852 |
| M+C % | 0.710 | 0.710 | 0.711 | 0.710 |

(continued)

| Nutrients | | | | |
|---|---|---|---|---|
| Thr % | 0.62 | 0.62 | 0.62 | 0.62 |

7.2 Results

In Table 7.3 production performance and egg quality are reported. Hens on diets with BP17 phytase, overall, performed better than hens on NC and fed competitors phytases. Egg production was improved by up to 5.5%, egg weight by up to 3.8%, egg mass by up to 9.7% and FCR by up to 6.2%.

Table 7.3: Efficacy of BP17, Phytase XP (*E. coli*) and the *P. lyci* phytase on the performance of laying hens fed corn/soya -based diet

| Measurements | PC | NC | NC + 300 FTU BP17 | NC + 600 FTU BP17 | NC + 900 FTU BP17 | NC + 300 FTU/kg E. coli Phytase | NC + 600 FTU/kg E. coli Phytase | NC + 900 FTU/kg E. coli Phytase | NC + 450 FTU/kg *P. lycii*Phytase | LSD | P |
|---|---|---|---|---|---|---|---|---|---|---|---|
| **Production performance** | | | | | | | | | | | |
| Egg production (HH) (%) | 89.96[ab] | 87.48[b] | 92.36[a] | 91.05[a] | 91.67[a] | 90.23[ab] | 91.51[a] | 90.94[a] | 91.61[a] | 2.79 | 0.04 |
| Egg production (HD) (%) | 91.064 | 89.032 | 92.36 | 91.07 | 91.67 | 90.72 | 91.51 | 92.08 | 91.61 | 2.64 | 0.39 |
| Egg weight (g/egg) | 66.69[ab] | 64.72[c] | 67.21[a] | 65.67[bc] | 66.46[ab] | 65.81[bc] | 66.78[ab] | 65.73[bc] | 65.39[bc] | 1.39 | 0.01 |
| Egg mass (g/hen/day) | 60.00[ab] | 5665[c] | 62.18[a] | 59.77[b] | 60.93[ab] | 59.37[b] | 61.10[ab] | 59.79[b] | 59.91[b] | 2.21 | 0.006 |
| Heed intake (g/hen/day) | 119.37 | 115.81 | 117.23 | 115.84 | 118.69 | 115.12 | 117.12 | 116.49 | 115.84 | 2.99 | 0.08 |
| FCR (g feed/g egg) | 1.971[ab] | 2012[a] | 1.886[c] | 1.939[bc] | 1.951[b] | 1.932[bc] | 1.917[bc] | 1.925[bc] | 1.936[bc] | 0.05 | 0.003 |
| Feed Efficiency (%) | 50.88[bc] | 49.78[c] | 53.06[a] | 51.59[b] | 51.33[b] | 5188[ab] | 52.17[ab] | 51.97[ab] | 51.73[ab] | 1.45 | 0.003 |
| Mortality (%) | 5.00[ab] | 6.25[a] | 0[c] | 1.25[bc] | 0[c] | 1.25[bc] | 0[c] | 3.75[abc] | 0[c] | 3.94 | 0.004 |
| Weight gain (g/hen) | 8.69[a] | -173.69[c] | -47.5[b] | -10.84[ab] | -28.19[ab] | -54.22[b] | -32.28[ab] | -6.34[so] | -54.81[b] | 54.40 | 0.001 |
| **Egg quality Parameters** | | | | | | | | | | | |
| Haugh Unit | 82.75 | 87.25 | 83.37 | 85.68 | 81.87 | 81.50 | 85.12 | 84.37 | 87.81 | 6.05 | 0.38 |
| Yolk Colour | 10.75 | 10.68 | 11.37 | 11.50 | 11.12 | 11.31 | 10.75 | 11.25 | 11.18 | 0.65 | 0.11 |
| Shell Thickness | 0.42 | 0.41 | 0.39 | 0.41 | 0.40 | 0.40 | 0.40 | 0.41 | 0.42 | 0.02 | 0.13 |
| *Means with the same superscripts are not significantly different (P<0.05)* | | | | | | | | | | | |

**Example 8: Performance in animal feed in piglets**

The performance of BP17 in animal feed was evaluated in a piglet growth trial with the following parameters: digestibility of phosphate, calcium, nitrogen, DM and energy.

8.1 Materials and Methods

48 mixed gender weaned piglets (10-15kg bodyweight) were allocated to 6 treatments with 8 replicates (one piglet per replicate). The piglets were fed a commercial control diet with BP17 added at (250 FTU/kg to 1000 FTU/kg) and Phytase XP fed as 500 FTU/kg feed. Diets were fed as mash for 24 days. The positive control diet was corn barley based and formulated with 0.667% P and 0.75% Ca inclusion. The negative control diet was based on corn and barley and was formulated with 0.476% P and 0.6% Ca. Diets did not contain antimicrobial growth promoters or any alternatives.

Faeces and urine were collected separately from day 10-14 in each experimental period. Urine was collect in HCl to minimise evaporation of nitrogen. Faeces were collected by rectal stimulation twice a day. All samples were frozen (-18°C) and pooled at the end of the experiment for each animal. Digestibility analysis was then determined.

Table 8.1: Experimental design

| Treatment | Level |
|---|---|
| 1 | Positive Control |
| 2 | Negative Control |
| 3 | BP17 phytase 250FTU/kg |
| 4 | BP17 phytase 500FTU/kg |
| 5 | BP17 phytase 1,000FTU/kg |

Table 8.2: Diet composition (kg/tonne) as fed

| Ingredients | Positive Control | Negative Control |
|---|---|---|
| Maize | 20.48 | 21.77 |
| Barley | 45 | 45 |
| Cane Molasses | 1 | 1 |
| Sunflower Meal Ext. | 4 | 4 |
| Dried Whey | 5 | 5 |
| Soya protein concentrate | 4.5 | 4.5 |
| Soybean Oil | 3.31 | 2.89 |
| DL-methionine | 0.12 | 0.12 |
| Inert filler | 3 | 3 |
| Salt | 0.39 | 0.39 |
| Limestone | 0.93 | 0.93 |
| Monocalcium Phosphate | 1.05 | 0.18 |
| Trace Mineral/vitamin premix | 2.14 | 2.14 |
| Soybean Meal 47%CP | 8.5 | 8.5 |
| L-lysine HCl 79% | 0.43 | 0.43 |
| L-threonine 98% | 0.09 | 0.09 |
| L-tryptophan 98% | 0.03 | 0.03 |
| BP17 (FTU/kg feed) | - | 0/250/500/1,000 |
| Phytase XP (FTU/kg feed) | - | 0/500 |
| **Calculated analysis** | | |
| Crude protein (%) | 16.02 | 16.13 |
| DE kcal/kg *(MJ/kg)* | 3178 *(13.3)* | 3178 *(13.3)* |
| NE kcal/kg *(MJ/kg)* | 2265 *(9.48)* | 2263 *(9.47)* |
| Calcium | 0.85 | 0.72 |

(continued)

| Calculated analysis | | |
|---|---|---|
| Total P (%) | 0.6 | 0.4 |
| Digestible lysine (%) | 0.9 | 0.9 |
| Methionine (%) | 0.3 | 0.3 |
| Digestible methionine (%) | 0.3 | 0.3 |
| Methionine + Cysteine (%) | 0.6 | 0.6 |
| Digestible methionine + cysteine | 0.54 | 0.54 |
| Available phosphorus | 0.3 | 0.19 |
| Sodium | 0.22 | 0.22 |

8.2 Results

Inclusion of BP17 phytase improved P and Ca digestibility, as well as retention of P and N (Table 8.3). Supplementing a negative control diet with BP17 phytase improved digestibility of Nitrogen (by 3.5 to 8.9%), Calcium (by 17.3 to 25%) and Phosphorus (by 33.9 to 59.8%). BP17 also improved retention of Nitrogen (by 4.2 to 12.4%), Calcium (by 28.7 to 48.7%) and Phosphorus (by 34.4 to 60.4%).

Table 8.3 Digestibility improvements with BP17 phytase

| | NC | BP17 250 FTU/kg | BP17 500 FTU/kg | BP17 1000 FTU/kg |
|---|---|---|---|---|
| DM dig., % | $77.9^x$ | $79.5^{xy}$ | $79.7^{yz}$ | $81.4^z$ |
| Ash digestibility, % | $38.1^x$ | $42.7^y$ | $43.8^{yz}$ | $45.2^z$ |
| N dig., % | $72.3^x$ | $74.9^x$ | $75.6^{xy}$ | $78.8^y$ |
| Ca dig., & | 62.6 | 73.4 | 77.3 | 78.3 |
| P dig., % | $41.0^x$ | $54.9^y$ | $62.3^z$ | $65.5^z$ |
| N ret., % | $61.9^x$ | $64.5^x$ | $66.2^{xy}$ | $69.6^y$ |
| Ca ret., % | $49.4^x$ | $63.6^y$ | $70.3^z$ | $73.5^z$ |
| P ret., % | $40.7^x$ | $54.7^y$ | $62.1^z$ | $65.3^z$ |

## Example 9: Performance in animal feed in piglets

The performance of BP17 in animal feed was evaluated in a piglet growth trial with the following parameters: growth, Feed intake and FCR.

9.1 Materials and methods

48 male weaned piglets (7-11kg bodyweight) were allocated to 6 treatments with 8 replicates (one piglet per replicate). The positive control diet was wheat/soybean based with a formulated inclusion rate of 0.65% P and 0.65% Ca. The piglets were fed a wheat and soymeal based negative control diet formulated with 0.46% available P and 0.55% calcium with different levels of required enzymes added (250 FTU/kg to 2000 FTU/kg) fed as pellets for 14 days.

Urinary and faecal production was recorded twice daily (am and pm) from 10 to 14 days. Faeces were collected separately from day once a day. All samples were weighed and stored in a refrigerator (4°C) and pooled at the end of the experiment for each animal. Urine was collected twice daily in an airtight container and 25ml of sulphuric acid added to prevent volatilisation of nitrogen fraction. Digestibility analysis was then determined.

Table 9.1: Experimental design

| Treatment | Level |
|---|---|
| 1 | Positive Control |
| 2 | Negative Control |

(continued)

| Treatment | Level |
|---|---|
| 3 | BP17 phytase 250FTU/kg |
| 4 | BP17 phytase 500FTU/kg |
| 5 | BP17 phytase 1,000FTU/kg |
| 6 | BP17 phytase 2,000FTU/kg |

Table 9.2: Diet composition

| Ingredients | Positive Control | Negative Control |
|---|---|---|
| Wheat (11.3%CP) | 58.31 | 59.58 |
| Soybean Meal 48%CP | 20.79 | 20.54 |
| Fishmeal 60/9 | 1 | 1 |
| Skimmed Milk 2A | 5 | 5 |
| Dried Whey | 10 | 10 |
| Soybean Oil | 2.37 | 2 |
| L-lysine HCl | 0.38 | 0.38 |
| DL-methionine | 0.08 | 0.08 |
| L-threonine | 0.16 | 0.16 |
| Salt | 0.1 | 0.1 |
| Limestone | 0.27 | 0.67 |
| Dicalcium Phosphate | 1.06 | 0 |
| Trace Mineral/vitamin premix | 0.05 | 0.5 |
| BP17 (FTU/kg feed) | - | 0/250/500/1000/2000 |
| **Calculated analysis** | | |
| Crude protein (%) | 20.4 | 20.5 |
| DE kcal/kg *(MJ/kg)* | 3465 *(14.5)* | 3465 *(14.5)* |
| NE kcal/kg *(MJ/kg)* | 2372 *(9.92)* | 2367 *(9.90)* |
| Calcium | 0.65 | 0.55 |
| Total P (%) | 0.65 | 0.46 |
| Digestible lysine (%) | 1.23 | 1.22 |
| Methionine (%) | 0.39 | 0.39 |
| Digestible methionine (%) | 0.36 | 0.36 |
| Methionine + Cysteine (%) | 0.74 | 0.75 |
| Digestible methionine + cysteine | 0.63 | 0.63 |
| Available phosphorus | 0.44 | 0.26 |
| Sodium | 0.19 | 0.19 |

9.2 Results

Inclusion of BP17 phytase improved FCE (Feed conversion efficiency = BWG (Body weight gain) / Feed Intake), FCR, ADG (Average Daily Gain = BWG/days the animal was on test) and ADFI (Average Daily Feed Intake = Total Feed Intake / days the animal was on test) in weaned piglets (Table 9.3), furthermore, also DE (Digestible Energy = Feed energy - Fecal Energy) improved also with increasing inclusion of BP17 phytase. Feeding BP17 phytase between 250 and 2000 FTU/kg resulted in improved Average daily gain (by 14.5 to 24%), FCR (by 10.8 to 14.4%) and Digestible energy (by 2.1 to 4 %) compared to piglets fed a negative control diet.

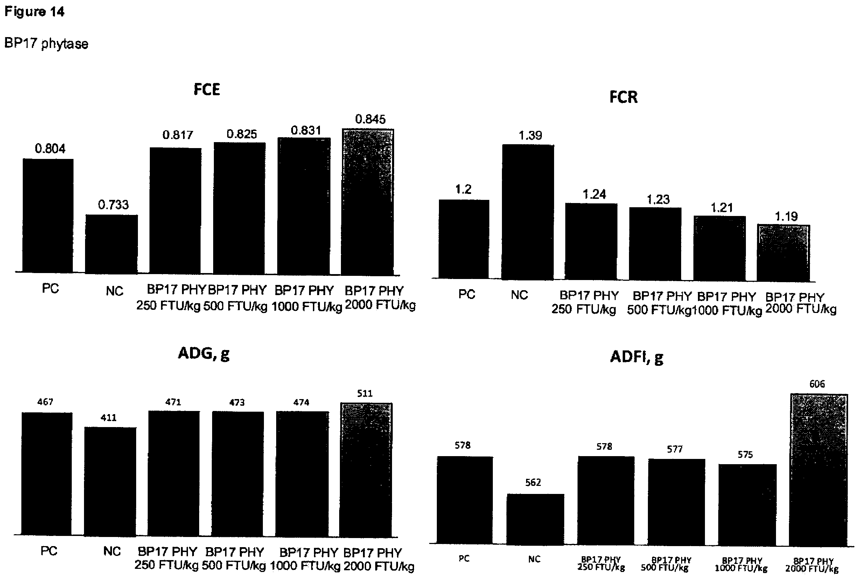

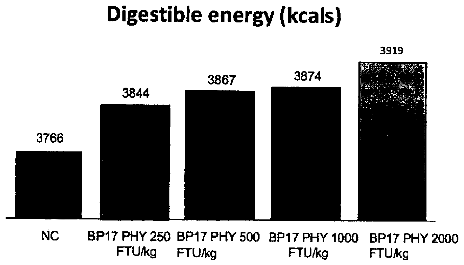

Table 9.3 Performance and Digestible energy improvements with BP17 phytase (see also Figure 14).

|  | PC | NC | BP17 phytase 250 FTU/kg | BP17 phytase 500 FTU/kg | BP17 phytase 1000 FTU/kg | BP17 phytase 2000 FTU/kg |
|---|---|---|---|---|---|---|
| ADG, g | 467 | 411 | 471 | 473 | 474 | 511 |
| ADFI, g | 578 | 562 | 578 | 577 | 575 | 606 |
| FCR | 1.25 | 1.39 | 1.24 | 1.23 | 1.21 | 1.19 |
| FCE | 0.804 | 0.733 | 0.817 | 0.825 | 0.831 | 0.845 |
| DE (kcals/kg) | - | 3766 | 3844 | 3867 | 3874 | 3919 |

**Example 10: Performance in animal feed in piglets**

The performance of BP17 in animal feed was evaluated in a piglet growth trial with the following parameters: Bone ash, Bone Ca, Bone P and Ca retention.

10.1 Materials and Methods

30 male piglets (15-20kg) were allocated 5 treatments with 6 replicates (1 pig per replicate). The positive control diet was a corn/soybean based with a formulated inclusion rate of 6.0g/kg P and 7.0g/kg Ca. The piglets were fed a corn/soybean based negative control diet formulated with 4.0g/kg P and 6.0g/kg Ca with different levels of required enzymes added (125 FTU/kg to 1,500 FTU/kg) fed as mash for 15 days. Urine was collected in environmental controlled containers from day 8-12. The faeces were collected during the same period separately. All samples were stored at -20°C over the collection period before analysis. Bone strength was determined from the metacarpals.

Table 10.1: Experimental design

| Treatment | Level |
|---|---|
| 1 | Positive Control |
| 2 | Negative Control |
| 3 | BP17 phytase 125 FTU/kg |
| 4 | BP17 phytase 250 FTU/kg |
| 5 | BP17 phytase 1,500 FTU/kg |

Table 10.2: Diet composition

|  | Positive control | Negative control |
|---|---|---|
| Corn | 624.0 | 635.0 |
| Soybean meal (47.5%) | 330.0 | 330.0 |
| Corn oil | 20.0 | 20.0 |
| Salt | 4.0 | 4.0 |
| Limestone (36% Ca) | 8.0 | 12.5 |
| Dicalcium phosphate[a] | 11.0 | 0.0 |
| Trace mineral premix[d] | 3.0 | 3.0 |
| Phytase premix[e] | - | 0.0/125/250/1,500 |
|  |  |  |
| *Calculated nutrients & energy* |  |  |
| Protein, g/kg | 208.5 | 208.5 |

(continued)

| Calculated nutrients & energy | | |
|---|---|---|
| DE, kcal/kg | 3417 | 3440 |
| Ca, g/kg | 7.0 | 6.0 |
| P, g/kg | 6.0 | 4.0 |
| *enzyme mixture are replace minimum amount of com* | | |

10.2 Results

In Table 10.3 bone P, bone Ca, bone ash and Ca retention are shown. From an inclusion of BP17 phytase at 250 FTU an improvement in bone Ca (0.4%) and bone ash (7%) was found compared to the negative control. For Ca retention an improvement was found for inclusion of BP17 phytase at 125 FTU (21%) compared to the negative control. For Bone P and bone ash inclusion of 1,500 FTU/kg returned performance to the level of the positive control.

Table 10.3 Bone parameters and Ca retention

| | PC | NC | BP17 phytase 125 FTU/kg | BP17 phytase 250 FTU/kg | BP17 phytase 1500 FTU/kg |
|---|---|---|---|---|---|
| Ca retention, % | 72.6$^v$ | 32.2$^x$ | 39.1$^{xy}$ | 48.4$^{yz}$ | 57.9$^z$ |
| Bone P, % | 18.4$^y$ | 17.9$^x$ | 17.9$^x$ | 17.9$^x$ | 18.1$^{xy}$ |
| Bone Ca, % | 41.1 | 40.5 | 40.4 | 40.7 | 40.9 |
| Bone ash, % | 36.5$^z$ | 29.7$^x$ | 28.8$^x$ | 31.8$^y$ | 34.7$^z$ |

**Example 11: Performance in animal feed in grower-finisher pigs**

The performance of BP17 in animal feed was evaluated in a grower-finisher pig growth trial with the following parameters: digestibility of phosphate, calcium, nitrogen, energy, and DM, and growth, feed intake and FCR.

11.1 Materials and Methods

72 grower finisher pigs (40-60kg bodyweight) were allocated 6 treatments with 12 replicates. The pigs were fed a standard commercial mash diet before the trial started. The positive control diet was barley, wheat and soybean meal diet formulated with 0.54% P and 0.68% Ca. The negative control diet was based on barley, wheat and soybean meal diet with 0.37% P and 0.59% Ca inclusion and was fed either un-supplemented or supplemented with BP17 250, 500, 1,000 and 2,000 FTU/kg. Feed and water were freely available.

Urine and faeces were collected from day 5 to 7. All samples were collected in environmentally controlled containers. The samples were stored at -20°C and pooled for each animal for digestibility analysis. Total tract digestibility of P, Ca, N, energy and DM, beside DE was calculated. Performance for the pigs was calculated.

Table 11.1: Experimental design

| Treatment | Level |
|---|---|
| 1 | Positive Control |
| 2 | Negative Control |
| 3 | BP17 phytase 250FTU/kg |
| 4 | BP17 phytase 500FTU/kg |
| 5 | BP17 phytase 1,000FTU/kg |
| 6 | BP17 phytase 2,000FTU/kg |

Table 11.2: Diet composition

| Ingredients | Positive Control | Negative Control |
|---|---|---|
| Wheat (11.3%CP) | 35 | 36.11 |
| Barley | 40.8 | 41.46 |
| Soybean Meal 48%CP | 12.01 | 12.76 |
| Rapeseed Meal | 5 | 3.5 |
| Choice White Grease | 3.13 | 2.65 |
| L-lysine HCl | 0.28 | 0.27 |
| DL-methionine | 0.04 | 0.05 |
| L-threonine | 1 | 0.04 |
| Inert marker | 0.43 | 1 |
| Sodium Bicarbonate | 0.14 | 0.42 |
| Salt | 0.14 | 0.14 |
| Limestone | 0.84 | 1.2 |
| Dicalcium Phosphate | 0.89 | 0 |
| Trace Mineral/vitamin premix | 0.4 | 0.4 |
| BP17BP17 (FTU/kg feed | - | 0/250/500/1,000/2,000 |
| **Calculated analysis** | | |
| Crude protein (%) | 16.2 | 16.2 |
| DE kcal/kg *(MJ/kg)* | 3250 *(13.6)* | 3250 *(13.6)* |
| NE kcal/kg *(MJ/kg)* | 2308 *(9.66)* | 2303 *(9.64)* |
| Calcium | 0.68 | 0.59 |
| Total P (%) | 0.54 | 0.37 |
| Digestible lysine (%) | 0.80 | 0.79 |
| Methionine (%) | 0.28 | 0.29 |
| Digestible methionine (%) | 0.25 | 0.26 |
| Methionine + Cysteine (%) | 0.60 | 0.60 |
| Digestible methionine + cysteine | 0.48 | 0.49 |
| Available phosphorus | 0.30 | 0.14 |
| Sodium | 0.21 | 0.20 |

11.2 Results

Inclusion of BP17 phytase improved the total tract digestibility of P (by up to 135%), Ca (by up to 28.1%), N (by up to 3.3%), DM (by up to 2.1%), and DE (by up to 40 kcals; Table 11.3). In Table 11.4, performance for the pigs is shown. Improvements were observed in ADG (up to 58%) and FCR (up to 2%) after inclusion of BP17 phytase, without influencing the feed intake for the pigs.

Table 11.3 Digestibility improvements with BP17 in grower-finisher pigs

| | NC | BP17 phytase 250 FTU/kg | BP17 phytase 500 FTU/kg | BP17 phytase 1000 FTU/kg | BP17 phytase 2000 FTU/kg |
|---|---|---|---|---|---|
| DM dig., % | 82.5[x] | 83.9[y] | 84.0[y] | 83.9[y] | 84.2[y] |
| N dig., % | 81.9[x] | 84.4[y] | 84.1[y] | 84.6[y] | 84.5[y] |
| Ca dig., & | 54.3[x] | 67.7[y] | 69.3[y] | 68.4[y] | 69.6[y] |
| P dig., % | 25.9[x] | 51.5[y] | 53.8[yx] | 57.2[zv] | 61.0[v] |
| DE (kcals/kg) | 3411 | 3449 | 3439 | 3451 | 3446 |

Table 11.4 Performance of grower-finisher pigs fed diets containing BP17

| | NC | BP17 phytase 250 FTU/kg | BP17 phytase 500 FTU/kg | BP17 phytase 1000 FTU/kg | BP17 phytase 2000 FTU/kg |
|---|---|---|---|---|---|
| ADG, q | $298^x$ | $443^y$ | $465^y$ | $450^y$ | $472^y$ |
| ADFI, kg | 1.43 | 1.43 | 1.39 | 1.41 | 1.4 |
| FCR | $5.22^y$ | $3.45^x$ | $3.11^x$ | $3.19^x$ | $3.12^x$ |
| G:F | $0.211^x$ | $0.312^y$ | $0.334^y$ | $0.322^y$ | $0.333^y$ |

**Example 12** - **Performance in animal feed in grower-finisher pigs**

The performance of BP17 in animal feed was evaluated in a grower-finisher pig growth trial on the ash digestibility.

12.1 Materials and Methods

45 grower finisher pigs (40-60kg bodyweight) were allocated to 5 treatments with 9 replicates. The pigs were fed a standard commercial pelleted diet before the trial started. The positive control diet was a corn/soybean meal diet formulated with 0.64% P and 0.5% Ca. The negative control diet was based on a corn/soybean meal formulated with 0.45% P and 0.4% Ca, this was fed either un-supplemented or supplemented with BP17 phytase at 250, 1,000 or 2,000 FTU/kg feed. Feed and water were freely available. Faeces were collected in the third week for three days using grab-sampling. The samples were stored in a refrigerator (4°C) and pooled for each animal for digestibility analysis. Total tract ash digestibility was calculated.

Table 12.1: Experimental design

| Treatment | Level |
|---|---|
| 1 | Positive Control |
| 2 | Negative control |
| 3 | BP17 phytase 250FTU/kg |
| 4 | BP17 phytase 1,000FTU/kg |
| 5 | BP17 phytase 2,000FTU/kg |

Table 12.2: Diet composition

| Ingredients | Positive Control | Negative Control |
|---|---|---|
| Maize | 57.33 | 58.67 |
| Cane Molasses | 4 | 4 |
| Soybean Meal 48%CP | 12.5 | 12.5 |
| Rapeseed Meal | 12.5 | 12.5 |
| Sunflower Meal Ext | 6 | 6 |
| Cod liver oil | 2.23 | 1.76 |
| L-lysine HCl | 0.2 | 0.2 |
| L-tryptophan | 0.1 | 0.1 |
| Salt | 0.25 | 0.25 |
| Limestone | 0.58 | 0.57 |
| Monocalcium Phosphate | 0.9 | 0.05 |
| Trace Mineral/vitamin premix | 0.4 | 0.4 |
| BP17 (FTU/kg feed) | - | 0/250/1,000/2,000 |

(continued)

| Calculated analysis | | |
|---|---|---|
| Crude protein (%) | 17.3 | 17.4 |
| DE kcal/kg (MJ/kg) | 3178 (13.3) | 3178 (13.3) |
| NE kcal/kg (MJ/kg) | 2251 (9.42) | 2248 (9.41) |
| Calcium | 0.5 | 0.4 |
| Total P (%) | 0.64 | 0.45 |
| Digestible lysine (%) | 0.79 | 0.79 |
| Methionine (%) | 0.3 | 0.3 |
| Digestible methionine (%) | 0.28 | 0.28 |
| Methionine + Cysteine (%) | 0.65 | 0.65 |
| Digestible methionine + cysteine | 0.51 | 0.52 |
| Available phosphorus | 0.32 | 0.13 |
| Sodium | 0.13 | 0.13 |

12.2 Results

In table 12.3, total tract digestibility of ash is shown. Inclusion of BP17 phytase increased the ash digestibility (by up to 29%), which suggests an improvement in bone strength (bone mineralisation).

Table 12.3 Improvement in ash digestibility with BP17 phytase

| | Negative control | BP17 phytase 250 FTU/kg | BP17 phytase 1000 FTU/kg | BP17 phytase 2000 FTU/kg |
|---|---|---|---|---|
| Ash digestibility (%) | 31.2$^x$ | 37$^y$ | 38.1$^y$ | 40.2$^z$ |

**Example 13: Performance in animal feed in grower-finisher pigs**

The performance of BP17 in animal feed was evaluated in a grower-finisher pig growth trial with the following parameters: Nutrient retention of Ca.

13.1 Materials and methods

54 grower finisher pigs (over 35kg bodyweight) were allocated 6 treatments with 9 replicates. The pigs were fed a standard commercial mash diet before the trial started. The positive control diet was a wheat/soybean meal diet formulated with 0.57% P and 0.53% Ca. The negative control diet was based on a wheat/soybean meal formulated with 0.37% P and 0.40% Ca, this was fed either un-supplemented or supplemented with BP17 at 250, 500, 1,000 or 2,000 FTU/kg feed for 14 days. Feed and water were freely available.
Faeces and urine were collected for four days separately. The samples were stored in a freezer (-20°C) and pooled for each animal for retention analysis.

Table 13.1: Experimental design

| Treatment | Level |
|---|---|
| 1 | Positive Control |
| 2 | Negative Control |
| 3 | BP17 phytase 250 FTU/kg |
| 4 | BP17 phytase 500 FTU/kg |
| 5 | BP17 phytase 1,000 FTU/kg |
| 6 | BP17 phytase 2,000 FTU/kg |

Table 13.2: Diet composition

| Diet formulation may need to be revised depending on pig age/weight | | |
|---|---|---|
| **Diet (age range 14-42 days)** | **Positive Control** | **Negative Control** |
| Ingredients in % | | |
| Wheat | 71.33 | 73.00 |
| Soybean Meal | 22.60 | 22.16 |
| Canola Oil | 2.21 | 1.75 |
| Dical (20% Ca; 18.5% P) | 1.07 | 0.00 |
| Limestone (36% Ca) | 0.30 | 0.58 |
| Salt | 0.30 | 0.30 |
| Lysine.HCl | 0.33 | 0.34 |
| DL-Methionine | 0.04 | 0.04 |
| L-Threonine | 0.13 | 0.13 |
| Celite | 1.00 | 1.00 |
| V/TM Premix | 0.70 | 0.70 |
| **PHYTASE PREMIX**\*\* | | |
| | | |
| TOTAL | 100 | 100 |
| | | |
| Calculated Nutrients & Energy | | |
| Protein, % | 19.45 | 19.45 |
| DE, Mj/kg | 14.32 | 14.32 |
| Ca, % | 0.53 | 0.40 |
| P, % | 0.57 | 0.37 |
| Dig. P % | 0.25 | 0.12 |
| Digestible amino acids, % | | |
| Lys | 1.04 | 1.04 |
| Met | 0.29 | 0.29 |
| Met+Cys | 0.56 | 0.57 |
| Thr | 0.65 | 0.65 |
| Trp | 0.20 | 0.20 |
| Total amino acids, % | | |
| Lys | 1.15 | 1.15 |
| Met | 0.31 | 0.31 |
| Met+Cys | 0.65 | 0.65 |
| Thr | 0.78 | 0.78 |

(continued)

| Total amino acids, % | | |
|---|---|---|
| Trp | 0.23 | 0.23 |

*[a]Provided the following per kg of premix: vitamin A 1,650,000 IU, vitamin D 165, 000 IU, vitamin E 8,000, menadione 800 mg, thiamin 200 mg, riboflavin 1,000 mg, niacin 7,000 mg, d-pantothenic acid 3,000 mg, vitamin B12 5 mg, biotin 40 mg and folic acid 400 mg*
*[b]Provided the following per kg of premix: copper 10 g, iron 16 g, manganese 5 g, zinc 20 g, iodine 100 mg, selenium 20 m*
*\*\*Phytase premix replaced minimum amount of wheat*

13.2 Results

In Table 13.3, Ca retention (%) is shown. Inclusion of BP17 phytase increased the Ca Retention by up to 40% over the NC.

Table 13.3 Calcium retention of grower-finisher pigs fed diets containing BP17 phytase

| | Negative control | BP17 phytase 250 FTU/kg | BP17 phytase 500 FTU/kg | BP17 phytase 1000 FTU/kg | BP17 phytase 2000 FTU/kg |
|---|---|---|---|---|---|
| Calcium retention (%) | 51.5[x] | 64.1[y] | 67.2[y] | 71.9[z] | 67.7[yz] |

**Example 14: Performance in animal feed in lactating sows**

The performance of BP17BP17 in animal feed was evaluated in a sow performance trial with the following parameters: digestibility of DM, Ca, P, protein and energy.

14.1 Materials and methods

56 lactating sows were allocated 7 treatments with 8 replicates. The sows were fed a commercial gestation and lactation diet adequate in P and Ca. The positive control diet is corn soybean based formulated with 0.67% P and 0.68% Ca. 4 days after farrowing the sows were fed the negative control diet based on corn/soybean and formulated with 0.48% of P and 0.54% of Ca in pellet form. This diet was fed either un-supplemented or supplemented with BP17 phytase 250, 500, 750, 1,000 and 2,000 FTU/kg. Water was available *ad libitum.*
Faeces were collected on day 17 for three days. All samples were collected in environmentally controlled containers. The samples were stored in a freezer and pooled for each animal for digestibility analysis. Ash, Ca, P and CP were determined.

Table 14.1: Experimental design

| Treatment | Level |
|---|---|
| 1 | Positive Control |
| 2 | Negative Control |
| 3 | BP17 phytase 250 FTU/kg |
| 4 | BP17 phytase 500 FTU/kg |
| 5 | BP17 phytase 750 FTU/kg |
| 6 | BP17 phytase 1,000 FTU/kg |
| 7 | BP17 phytase 2,000 FTU/kg |

Table 14.2. Diet composition

| Ingredients | Positive Control | Negative Control |
|---|---|---|
| Maize | 51.51 | 52.96 |
| Cane Molasses | 4 | 4 |
| Soybean Meal 48%CP | 14.55 | 14.43 |
| Soya Hulls | 3 | 3 |
| Rapeseed Meal | 8 | 8 |
| Sunflower Meal Ext | 8.5 | 8.5 |
| Tallow | 3.77 | 3.3 |
| Lysine 20% | 0.46 | 0.46 |
| Salt | 0.28 | 0.28 |
| Limestone | 0.52 | 0.51 |
| Monocalcium Phosphate | 1.14 | 0.29 |
| Trace Mineral/vitamin premix | 1.25 | 1.25 |
| BP17 (FTU/kg feed) | - | 0/250/500/750/1,000/2,00 0 |
| **Calculated analysis** | | |
| Crude protein (%) | 17.5 | 17.5 |
| DE kcal/kg *(MJ/kg)* | 3130 *(13.1)* | 3154 *(13.2)* |
| NE kcal/kg *(MJ/kg)* | 2258 *(9.45)* | 2256 *(9.44)* |
| Calcium | 0.68 | 0.54 |
| Total P (%) | 0.67 | 0.48 |
| Digestible lysine (%) | 0.74 | 0.74 |
| Methionine (%) | 0.30 | 0.30 |
| Digestible methionine (%) | 0.28 | 0.28 |
| Methionine + Cysteine (%) | 0.63 | 0.63 |
| Digestible methionine + cysteine | 0.49 | 0.49 |
| Available phosphorus | 0.37 | 0.18 |
| Sodium | 0.14 | 0.14 |

14.2 Results

In Table 14.3, digestibility of all nutrients is shown. Inclusion of BP17 phytase improved digestibility of Ca (by up to 24.8%), ash (by up to 15%), P (by up to 75%) and CP (by up to 1.3%).

Table 14.3 Digestibility of all nutrients

| | NC | BP17 phytase 250 FTU/kg | BP17 phytase 500 FTU/kg | BP17 phytase 750 FTU/kg | BP17 phytase 1000 FTU/kg | BP17 phytase 2000 FTU/kg |
|---|---|---|---|---|---|---|
| DM | 83 | 82.7 | 82.9 | 83 | 82.9 | 83.1 |
| Ash | 32.5$^x$ | 35.2$^y$ | 36.0$^y$ | 36.4$^{yx}$ | 36.1$^{yz}$ | 37.4$^y$ |
| CP | 84 | 84.7 | 84.3 | 84.7 | 84.8 | 85.1 |
| GE | 86.5 | 85.9 | 86.9 | 85.9 | 85.9 | 86 |
| P | 30.1$^x$ | 44.7$^y$ | 48.9$^z$ | 49.2$^z$ | 50.4$^{zv}$ | 52.7$^v$ |
| Ca | 28.6 | 32.1 | 35.7 | 35.1 | 34.5 | 35.4 |

**Example 15: Performance in animal feed in piglets**

The performance of BP17 in animal feed was evaluated in a piglet growth trial with the following parameters: digestibility of phosphate, calcium and DM and retention of phosphate and calcium.

**15.1 Materials and Methods**

70 mixed gender weaned piglets (10-13kg bodyweight) were allocated to 7 treatments with 10 replicates (one piglet per replicate). The piglets were fed a commercial control diet with BP17 added at (250 FTU/kg to 2000 FTU/kg). Diets were fed as pelleted for 12 days. The positive control diet was corn barley based and formulated with 0.63% P and 0.70% Ca inclusion. The negative control diet was based on corn and barley and was formulated with 0.45% P and 0.57% Ca. Diets did not contain antimicrobial growth promoters or any alternatives.

Faeces and urine were collected separately from day 8-12 in each experimental period. Urine was collect in HCl to minimise evaporation of nitrogen. Faeces were collected twice a day. All samples were frozen (-18°C) and pooled at the end of the experiment for each animal. Digestibility analysis was then determined.

Table 15.1: Experimental design

| Treatment | Level |
|---|---|
| 1 | Positive Control |
| 2 | Negative Control |
| 3 | BP17 phytase 250FTU/kg |
| 4 | BP17 phytase 500FTU/kg |
| 5 | BP17 phytase 750FTU/kg |
| 6 | BP17 phytase 1,000FTU/kg |
| 7 | BP17 phytase 2,000FTU/kg |

Table 15.2: Diet composition (%) as fed

| Ingredients | Positive Control | Negative Control |
|---|---|---|
| Maize | 20.48 | 21.77 |
| Barley | 45 | 45 |
| Cane Molasses | 1 | 1 |
| Sunflower Meal Ext. | 4 | 4 |
| Dried Whey | 5 | 5 |
| Soya protein concentrate | 4.5 | 4.5 |
| Soybean Oil | 3.31 | 2.89 |
| DL-methionine | 0.12 | 0.12 |
| Inert filler | 3 | 3 |
| Salt | 0.39 | 0.39 |
| Limestone | 0.93 | 0.93 |
| Monocalcium Phosphate | 1.05 | 0.18 |
| Trace Mineral/vitamin premix | 2.14 | 2.14 |
| Soybean Meal 47%CP | 8.5 | 8.5 |
| L-lysine HCl 79% | 0.43 | 0.43 |
| L-threonine 98% | 0.09 | 0.09 |
| L-tryptophan 98% | 0.03 | 0.03 |
| BP17 (FTU/kg feed) | - | 0/250/500/750/1,000/2,000 |
| **Calculated analysis** | | |
| Crude protein (%) | 16.02 | 16.13 |
| DE kcal/kg *(MJ/kg)* | 3178 *(13.3)* | 3178 *(13.3)* |
| NE kcal/kg *(MJ/kg)* | 2265 *(9.48)* | 2263 *(9.47)* |
| Calcium | 0.85 | 0.72 |
| Total P (%) | 0.6 | 0.4 |
| Digestible lysine (%) | 0.9 | 0.9 |

(continued)

| Calculated analysis | | |
|---|---|---|
| Methionine (%) | 0.3 | 0.3 |
| Digestible methionine (%) | 0.3 | 0.3 |
| Methionine + Cysteine (%) | 0.6 | 0.6 |
| Digestible methionine + cysteine | 0.54 | 0.54 |
| Available phosphorus | 0.3 | 0.19 |
| Sodium | 0.22 | 0.22 |

15.2 Results

Inclusion of BP17 phytase improved P and Ca digestibility, as well as retention of P and Ca (Table 15.3). Supplementing a negative control diet with BP17 phytase improved digestibility of Calcium (by 47.8 to 54.0%) and Phosphorus (by 82.8 to 107.1%). BP17 also improved retention of Calcium (by 42.1 to 54.9%) and Phosphorus (by 98.3 to 122.2%).

Table 15.3 Digestibility and retention improvements with BP17 phytase

| | NC | BP17 250 FTU/kg | BP17 500 FTU/kg | BP17 750 FTU/kg | BP17 1,000 FTU/kg | BP17 2,000 FTU/kg |
|---|---|---|---|---|---|---|
| DM dig., % | 73.2 | 73.2 | 73.6 | 74.2 | 74.1 | 73.4 |
| Ash dig., % | $31.1^x$ | $37.6^y$ | $37.8^{yz}$ | $38.6^{yzv}$ | $39.4^{zv}$ | $39.7^v$ |
| Ca dig., % | $44.8^x$ | $66.2^y$ | $64.7^y$ | $65.9^y$ | $68.7^y$ | $69.0^y$ |
| P dig., % | $32.5^x$ | $59.4^y$ | $62.0^{yz}$ | $63.8^{zv}$ | $66.0^{vw}$ | $67.3^W$ |
| Ca ret., % | $43.0^x$ | $61.1^y$ | $61.6^y$ | $62.7^{yz}$ | $66.6^Z$ | $66.6^z$ |
| P ret., % | $29.8^x$ | $59.1^y$ | $61.6^{yz}$ | $63.3^{zv}$ | $65.3^v$ | $66.2^v$ |
| $^{xyzvw}$ Values without a common superscript are significantly different (P<0.05). | | | | | | |

**Example 16: Performance in animal feed in piglets**

The performance of BP17 in animal feed was evaluated in a piglet growth trial with the following parameters: digestibility of phosphate, calcium, DM and energy.

16.1 Materials and Methods

48 mixed gender weaned piglets (10-14kg bodyweight) were allocated to 6 treatments with 8 replicates (one piglet per replicate). The piglets were fed a commercial control diet with BP17 added at (250 FTU/kg to 2000 FTU/kg). Diets were fed as mash for 14 days. The positive control diet was wheat and soybean meal based and formulated with 0.66% P and 0.75% Ca inclusion. The negative control diet was based on wheat and soybean meal and was formulated with 0.50% P and 0.55% Ca. Diets did not contain antimicrobial growth promoters or any alternatives.

Faeces were collected from day 10-14 in each experimental period twice a day. All samples were frozen (-18°C) and pooled at the end of the experiment for each animal. Digestibility analysis was then determined.

Table 16.1: Experimental design

| Treatment | Level |
|---|---|
| 1 | Positive Control |

(continued)

| Treatment | Level |
|---|---|
| 2 | Negative Control |
| 3 | BP17 phytase 250FTU/kg |
| 4 | BP17 phytase 500FTU/kg |
| 5 | BP17 phytase 1,000FTU/kg |
| 6 | BP17 phytase 2,000FTU/kg |

Table 16.2: Diet composition (%) as fed

| Ingredients | Positive Control | Negative Control |
|---|---|---|
| Wheat | 37.59 | 38.49 |
| Barley | 17.11 | 17.11 |
| Canola meal | 6.00 | 6.00 |
| Peas | 5.00 | 5.00 |
| Dried Whey | 7.96 | 7.96 |
| Soybean Meal 47%CP | 22.00 | 22.00 |
| Vegetable Oil | 1.00 | 1.00 |
| DL-methionine | 0.04 | 0.04 |
| Inert filler | 0.30 | 0.30 |
| Limestone | 0.83 | 0.83 |
| Dicalcium Phosphate | 1.00 | 0.10 |
| Trace Mineral/vitamin premix | 1.00 | 1.00 |
| L-lysine HCl 79% | 0.06 | 0.06 |
| L-threonine 98% | 0.11 | 0.11 |
| BP17 (FTU/kg feed) | - | 0/250/500/1,000/2,000 |

| Calculated analysis | Positive control | Negative Control |
|---|---|---|
| [VOLUME] | 100.00 | 100.00 |
| DRY_MAT (%) | 88.94 | 88.83 |
| PROTEIN (%) | 21.15 | 21.25 |
| CFAT (%) | 2.62 | 2.64 |
| CFIBRE (%) | 3.21 | 3.23 |
| ASH (%) | 6.59 | 5.72 |
| DE PIG (MJ/kg) | 13.66 | 13.78 |
| TLYSINE (%) | 1.17 | 1.18 |
| METH (%) | 0.35 | 0.36 |
| M+C (%) | 0.75 | 0.75 |
| CALCIUM (%) | 0.84 | 0.63 |
| TPHOS (%) | 0.64 | 0.48 |
| Dig. P (%) | 0.30 | 0.19 |
| PHYTATE P (%) | 0.27 | 0.27 |
| AV PHOS (%) | 0.38 | 0.22 |
| NA (%) | 0.10 | 0.10 |

16.2 Results

Inclusion of BP17 phytase improved P, DM and GE digestibility (Table 16.3). Supplementing a negative control diet with BP17 phytase improved digestibility of Phosphorus (by 106.7 to 149.6%), DM (by 0.1 to 4.3%) and GE (by 0.7 to 5.1).

Table 16.3 Digestibility improvements with BP17 phytase

|  | NC | BP17 250 FTU/kg | BP17 500 FTU/kg | BP17 1,000 FTU/kg | BP17 2,000 FTU/kg |
|---|---|---|---|---|---|
| DM dig., % | 77.4$^x$ | 79.6$^y$ | 7.5$^{xy}$ | 80.3$^z$ | 80.7$^z$ |
| GE dig., % | 75.0$^x$ | 77.9$^{yz}$ | 75.5$^{xy}$ | 78.2$^z$ | 78.8$^z$ |
| Ca dig., % | 56.3 | 63.0 | 62.5 | 67.6 | 66.6 |
| P dig., % | 28.4$^x$ | 58.7$^y$ | 64.1$^{yz}$ | 67.9$^z$ | 70.9$^z$ |
| $^{xyz}$ Values without a common superscript are significantly different (P<0.05). | | | | | |

**Example 17: Performance in animal feed in broiler chicks**

Summary of results:

BP17 showed superior ileal phosphorus and amino acid digestibility, Phosphorus retention and calcium digestibility compared to an *E. coli* Phytase.

- BP17 was on average, 156% more effective than the *E. coli* phytase based on the exponential curve for phosphorus retention
- BP17 at 699 FTU/kg feed was equivalent to 1.2 g/kg feed of P from MCP whereas, in contrast, 2311 FTU/kg feed of the *E. coli* phytase was required based on retained Phosphorus
- BP17 gave superior protein and amino acid digestibilities compared to the *E. coli* phytase

Results:

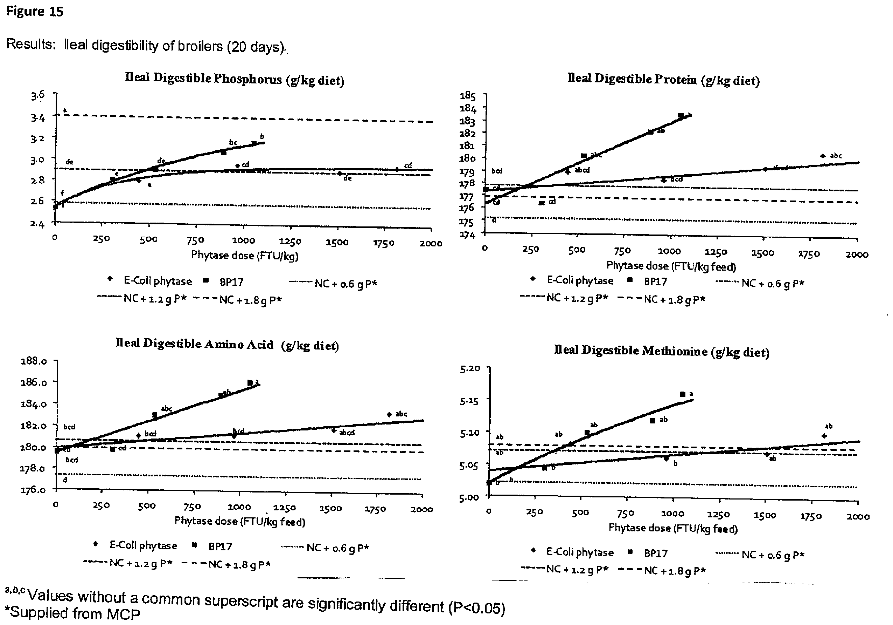

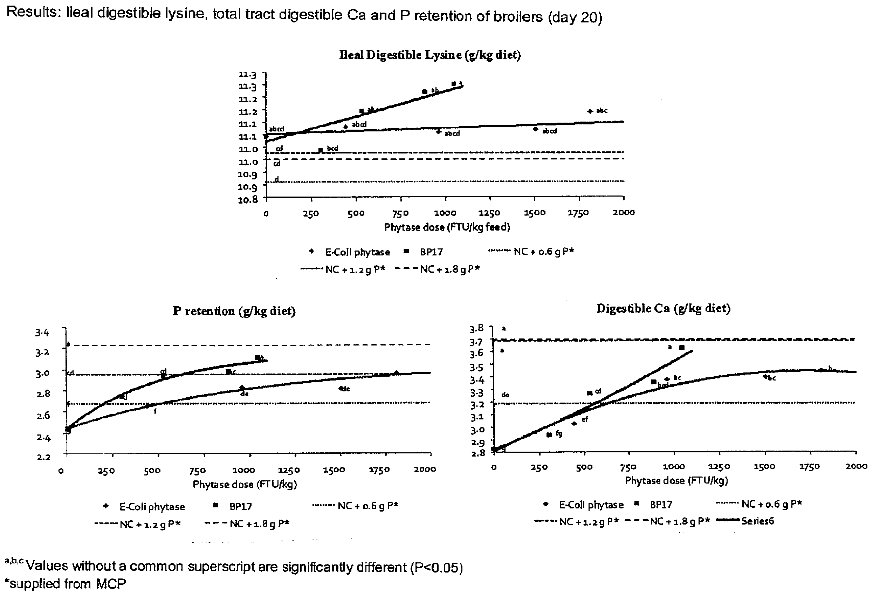

Equivalence of BP17 in FTU compared to 1 FTU of the *E. coli* phytase for ileal digestible P (g/kg diet) and retained P (g/kg diet) and relative bioefficacy of BP17 versus the *E. coli* phytase. The results of this example are shown in Figure 15.

Table 17.1: Ileal phosphorus and amino acid digestibility

|  | 250 FTU | 500 FTU | 750 FTU | 1000 FTU | Average |
|---|---|---|---|---|---|
| Ileal digestible P | 0.96 +4% | 0.78 +28% | 0.64 +56% | 0.53 +89% | 0.73 +37% |
| Retained P | 0.40 +150% | 0.39 +156% | 0.38 +163% | 0.37 +170% | 0.39 +156% |
| Levels (FTU/kg feed) of BP17 and the *E. coli* Phytase equivalent to 0.6 g or 1.2 g/kg feed of P from MCP. | | | | | |

Table 17.2: Ileal phosphorus and amino acid digestibility comparison of phytases

|  | 0.6 g P from MCP | | | 1.2 g P from MCP* | | |
|---|---|---|---|---|---|---|
|  | Ileal Digestible P (g/kg diet) | Retained P (g/kg diet) | Mean | Ileal Digestible P (g/kg diet) | Retained P (g/kg diet) | Mean |
| *E. coli* Phytase | 467 | 629 | 548 (100) | - | 2311 (100) | - |
| BP17 | 374 | 242 | 308 (56) | 963 | 699 (30) | - |
| *If the performance level of the MCP was not reached an equivalency could not be calculated and therefore a - is shown. | | | | | | |

Table 17.3: Ileal Digestibility of nutrients (%, day 20)

| | Phytase (FTU/kg feed) | | | | | NC + 0.6 g P | NC + 1.2 g P | NC +1.8 g P |
|---|---|---|---|---|---|---|---|---|
| | 0 | 250 | 500 | 750 | 1000 | | | |
| Phosphorus | | | | | | | | |
| E. coli Phytase | 59.1[e] | 65.2[d] | 68.8[bc] | 57.6[ed] | 68.6[bc] | 52.7[g] | 52.7[g] | 55.8[f] |
| BP17 | | 65.5[d] | 67.8[bc] | 71.5[ab] | 73.9[a] | | | |
| Crude protein | | | | | | | | |
| E. coli Phytase | 78.5[cd] | 79.2[bcd] | 79.0[bcd] | 79.4[abc] | 79.7[ab c] | 77.5[d] | 78.7[cd] | 78.3[cd] |
| BP17 | | 78.1[cd] | 79.8[abc] | 80.7[ab] | 81.3[a] | | | |
| Total Amino acids | | | | | | | | |
| E. coli Phytase | 80.6[cd] | 81.3[bcd] | 81.3[bcd] | 81.6[bc] | 82.0[ab c] | 79.6[d] | 81.1[cd] | 80.8[cd] |
| BP17 | | 80.7[cd] | 82.2[abc] | 83.0[ab] | 83.6[a] | | | |
| Lysine (%) | | | | | | | | |
| E. coli Phytase | 84.3[bc de] | 84.6[abcd] | 84.4[bcd] | 84.5[abcd] | 84.8[ab c d] | 82.9[e] | 83.8[cde] | 83.6[de] |
| BP17 | | 83.9[cde] | 85.1[abc] | 85.7[ab] | 5.9[a] | | | |
| Methionine (%) | | | | | | | | |
| E. coli Phytase | 89.6 | 90.8 | 90.4 | 90.6 | 90.9 | 89.7 | 90.6 | 90.7 |
| BP17 | | 90.1 | 91.1 | 91.4 | 92.2 | | | |
| Threonine (%) | | | | | | | | |
| E. coli Phytase | 72.1 | 73.0 | 72.7 | 73.3 | 73.5 | 71.0 | 72.8 | 72.5 |
| BP17 | | 72.4 | 73.8 | 74.4 | 75.0 | | | |
| a,b,c Values without a common superscript are significantly different (P<0.05) | | | | | | | | |

Table 17.4: Total tract calcium digestibility (%)

| | Phytase (FTU/kg feed) | | | | | NC + 0.6 g P | NC + 1.2 g P | NC +1.8g P |
|---|---|---|---|---|---|---|---|---|
| | 0 | 250 | 500 | 750 | 1000 | | | |
| Total Calcium | | | | | | | | |
| E. coli Phytase | 44.9[e] | 48.1[d] | 53.6[bc] | 54.0[bc] | 54.7[b] | 44,9[e] | 52.0[bc] | 52.2[bc] |
| BP17 | | 46.7[de] | 51.9[c] | 53.4[bc] | 57.6[a] | | | |
| a,b,c Values without a common superscript are significantly different (P<0.05) | | | | | | | | |

Table 17.5: Diets: (kg/tonne) as fed

| | Negative control | NC + 0.6 g P* | NC + 1.2 g P* | NC + 1.8 g P* |
|---|---|---|---|---|
| Maize | 560.3 | 560.3 | 560.3 | 560.3 |
| Maize Gluten Meal 60 | 14.8 | 14.8 | 14.8 | 14.8 |

(continued)

|  | Negative control | NC + 0.6 g P* | NC + 1.2 g P* | NC + 1.8 g P* |
|---|---|---|---|---|
| Soybean Meal 48%CP | 300.5 | 300.5 | 300.5 | 300.5 |
| Rapeseed Meal | 38 | 38 | 38 | 38 |
| Maize/Wheat Starch | 2.5 | 2.5 | 2.5 | 2.5 |
| Soybean Oil | 21.1 | 21.1 | 21.1 | 21.1 |
| Pig/Poultry Fat | 20 | 20 | 20 | 20 |
| L-Lysine HCl | 1.69 | 1.69 | 1.69 | 1.69 |
| DL-methionine | 2.24 | 2.24 | 2.24 | 2.24 |
| L-threonine | 0.37 | 0.37 | 0.37 | 0.37 |
| Sodium Bicarbonate | 1.67 | 1.67 | 1.67 | 1.67 |
| Salt | 2.44 | 2.44 | 2.44 | 2.44 |
| Limestone | 13.68 | 14.63 | 13.55 | 12.47 |
| Monocalcium Phosphate | 1.001 | 3.668 | 6.334 | 9.001 |
| Poultry Vits/TE's | 5.0 | 5.0 | 5.0 | 5.0 |
| Diamol | 14.73 | 11.12 | 9.53 | 7.94 |
| BP17 (FTU/kg feed) | 0/250/500/75 0/1000 | - | - | - |
| *E. coli* Phytase (FTU/kg feed[T]) | 0/250/500/75 0/1000 | - | - | - |
|  |  |  |  |  |
| Crude Protein (%) | 21.54 | 21.54 | 21.54 | 21.54 |
| ME kcals/kg *(MJlkg)* | 3102 *(12.98)* | 3102 *(12.98)* | 3102 *(12.98)* | 3102 *(12.98)* |
| Calcium (%) | 0.70 | 0.79 | 0.79 | 0.79 |
| Total P (%) | 0.42 | 0.48 | 0.54 | 0.60 |
| Digestible P (%) | 0.16 | 0.22 | 0.27 | 0.32 |
| Phytate P (%) | 0.26 | 0.26 | 0.26 | 0.26 |
| Available P (%) | 0.15 | 0.21 | 0.27 | 0.33 |
| Lysine (%) | 1.25 | 1.25 | 1.25 | 1.25 |
| Digestible Lysine (%) | 1.08 | 1.08 | 1.08 | 1.08 |
| Methionine (%) | 0.57 | 0.57 | 0.57 | 0.57 |
| Digestible Methionine (%) | 0.55 | 0.55 | 0.55 | 0.55 |
| Methionine + cystine (%) | 0.94 | 0.94 | 0.94 | 0.94 |
| Digestible methionine + cystine (%) | 0.80 | 0.80 | 0.80 | 0.80 |
| *Supplied from MCP, Planned dose rates | | | | |

Design of the trial:

288 male Ross 308 broiler chicks were allocated to 12 treatments with 6 cage replicates per treatment (4 birds per cage). All birds received a standard diet for the first 5 days - Crude Protein 21.5%; AME 2900 kcal/kg (12.1 MJ/kg). The Negative Control (NC) diet was based on corn/soybean meal and was reduced in phosphorus content (0.42% total phosphorus, 0.15% available phosphorus) and was supplemented with either BP17 or an *E. coli* phytase at 250, 500, 750 or 1000 FTU/kg feed. The dosages in feed were checked at 2 laboratories following dosing and the actual analysed levels were then used for all subsequent modelling. The 3 Positive Control diets contained incremental additions of monocalcium phosphate (+0.6, +1.2 and +1.8 g/kg feed P) to the NC formulation. All diets were fed as mash from days 5-20. Excreta was collected from each cage days 17-20 for the determination of calcium digestibility and phosphorus retention. On day 20 all birds were euthanased and ileal contents taken for determination of ileal phosphorus, protein and amino acid digestbilities.

Dose response relationships were determined using an exponential model of the form: $Y = A + B*R^x$ where Y = response parameter, A = upper asymptote value, B = maximum response value, R = non-linear slope parameter, X = dosed phytase activity (FTU/kg feed).

Equivalencies of BP17 and the *E. coli* phytase (in FTU/kg feed) were calculated for both ileal digestible phosphorus and retained phosphorus using the data for the positive control diets containing incremental additions of MCP. As with dose response data, an exponential curve best described this relationship and was used to calculate the product equivalencies.

**Example 18: Performance in animal feed in broiler chicks**

Summary of results

BP17 showed superior performance, tibia ash and AMEn compared to an *E. coli* Phytase

- BP17 was on average, 96% more effective than the *E. coli* phytase based on the exponential curve for tibia ash content
- BP17 at 1000 FTU/kg feed was able to fully restore bodyweight gain and tibia ash to the level of the birds fed the diet with 1.8 g P from MCP
- BP17 at 691 FTU/kg feed was equivalent to 1.2 g/kg feed of P from MCP whereas, in contrast, 1372 FTU/kg feed of the *E. coli* phytase was equivalent to 1.2 g/kg feed of P from MCP.

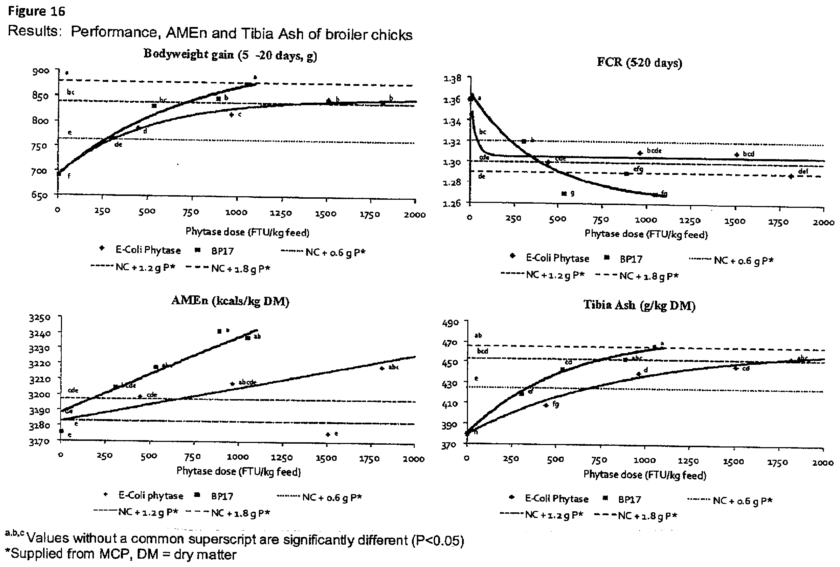

The results of this example are shown in Figure 16.

Table 18.1: Results: Feed intakes (g, 5-20 days)

| | Phytase (FTU/kg feed) | | | | | NC +0.6 gP | NC +1.2 gP | NC +1.8g P |
|---|---|---|---|---|---|---|---|---|
| | 0 | 250 | 500 | 750 | 1000 | | | |
| *E. coli* Phytase | 944[g] | 1026[ef] | 1066[cd] | 1111[ab] | 1089[bcd] | 1004[f] | 1089[b cd] | 1134[a] |
| BP17 | | 1012[f] | 1057[de] | 1093[bc] | 1113[ab] | | | |
| a,b,c Values without a common superscript are significantly different (P<0.05) | | | | | | | | |

Table 18.2: Equivalence of BP17 in FTU compared to 1 FTU of the *E. coli* phytase for bodyweight gain (5-20 days) and Tibia Ash (day 20), and relative bioefficacy of BP17 versus the *E. coli* phytase.

| | 250 FTU | 500 FTU | 750 FTU | 1000 FTU | Average |
|---|---|---|---|---|---|
| Bodyweight gain | 0.87 +15% | 0.78 +28% | 0.70 +42% | 0.62 +61% | 0.74 +35% |
| Tibia Ash | 0.53 +89% | 0.52 +92% | 0.51 +96% | 0.49 +104% | 0.51 +96% |

Table 18.3: Levels (FTU/kg feed) of BP17 and the *E. coli* Phytase equivalent to 0.6 g or 1.2 g/kg feed of P from MCP.

| | 0.6 g P from MCP | | | 1.2 g P from MCP | | |
|---|---|---|---|---|---|---|
| | Bodyweight gain (g) | Tibia Ash (g/kg DM) | Mean | Bodyweight gain (g) | Tibia Ash (g/kg DM) | Mean |
| *E. coli* Phytase | 355 | 682 | 519 (100) | 1156 | 1588 | 1372 (100) |
| BP17 | 296 | 350 | 323(62) | 659 | 722 | 691 (50) |

Table 18.4: Diets: (kg/tonne) as fed

| | Negative control | NC + 0.6g P* | NC + 1.2 g P* | NC + 1.8 g P* |
|---|---|---|---|---|
| Maize | 560.3 | 560.3 | 560.3 | 560.3 |
| Maize Gluten Meal 60 | 14.8 | 14.8 | 14.8 | 14.8 |
| Soybean Meal 48%CP | 300.5 | 300.5 | 300.5 | 300.5 |
| Rapeseed Meal | 38 | 38 | 38 | 38 |
| Maize/Wheat Starch | 2.5 | 2.5 | 2.5 | 2.5 |
| Soybean Oil | 21.1 | 21.1 | 21.1 | 21.1 |

(continued)

| | Negative control | NC + 0.6g P* | NC + 1.2 g P* | NC + 1.8 g P* |
|---|---|---|---|---|
| Pig/Poultry Fat | 20 | 20 | 20 | 20 |
| L-Lysine HCl | 1.69 | 1.69 | 1.69 | 1.69 |
| DL-methionine | 2.24 | 2.24 | 2.24 | 2.24 |
| L-threonine | 0.37 | 0.37 | 0.37 | 0.37 |
| Sodium Bicarbonate | 1.67 | 1.67 | 1.67 | 1.67 |
| Salt | 2.44 | 2.44 | 2.44 | 2.44 |
| Limestone | 13.68 | 14.63 | 13.55 | 12.47 |
| Monocalcium Phosphate | 1.001 | 3.668 | 6.334 | 9.001 |
| Poultry Vits/TE's | 5.0 | 5.0 | 5.0 | 5.0 |
| Diamol | 14.73 | 11.12 | 9.53 | 7.94 |
| BP17 (FTU/kg feed) | 0/250/500/750/1000 | - | - | - |
| *E. coli* Phytase (FTU/kg[T]) | 0/250/500/750/1000 | - | - | - |
| | | | | |
| Crude Protein (%) | 21.54 | 21.54 | 21.54 | 21.54 |
| ME kcals/kg *(MJlkg)* | 3102 *(12.98)* | 3102 *(12.98)* | 3102 *(12.98)* | 3102 *(12.98)* |
| Calcium (%) | 0.70 | 0.79 | 0.79 | 0.79 |
| Total P (%) | 0.42 | 0.48 | 0.54 | 0.60 |
| Digestible P (%) | 0.16 | 0.22 | 0.27 | 0.32 |
| Phytate P (%) | 0.26 | 0.26 | 0.26 | 0.26 |
| Available P (%) | 0.15 | 0.21 | 0.27 | 0.33 |
| Lysine (%) | 1.25 | 1.25 | 1.25 | 1.25 |
| Digestible Lysine (%) | 1.08 | 1.08 | 1.08 | 1.08 |
| Methionine (%) | 0.57 | 0.57 | 0.57 | 0.57 |
| Digestible Methionine (%) | 0.55 | 0.55 | 0.55 | 0.55 |
| Methionine + cystine (%) | 0.94 | 0.94 | 0.94 | 0.94 |
| Digestible methionine + cystine (%) | 0.80 | 0.80 | 0.80 | 0.80 |
| Supplied from MCP, [T]Planned levels to add | | | | |

Design of the trial

288 male Ross 308 broiler chicks were allocated to 12 treatments with 6 cage replicates per treatment (4 birds per cage). All birds received a standard diet for the first 5 days - Crude Protein 21.5%; AME 2900 kcal/kg (12.1 MJ/kg). The Negative Control (NC) diet was based on corn/soybean meal and was reduced in phosphorus content (0.42% total phosphorus, 0.15% available phosphorus) and was supplemented with either BP17 or an *E. coli* phytase at 250, 500, 750 or 1000 FTU/kg feed. The dosages in feed were checked at 2 laboratories following dosing and the actual analysed levels were then used for all subsequent modelling. The 3 Positive Control diets contained incremental additions of monocalcium phosphate (+0.6, +1.2 and +1.8 g/kg feed P) to the NC formulation. All diets were fed as mash from days 5-20. Birds were weighed days 5 and 20 and FCR calculated. Excreta was collected from each cage days 17-20 for the determination of AMEn. On day 20 all birds were euthanased and the left tibia dissected out for determination of bone ash.
Dose response relationships were determined using an exponential model of the form: $Y=A + B*R^x$ where Y = response parameter, A = upper asymptote value, B = maximum response value, R = non-linear slope parameter, X = dosed phytase activity (FTU/kg feed). Equivalencies of BP17 and the *E. coli* phytase (in FTU/kg feed) were calculated for both bodyweight gain and tibia ash (%) using the data for the positive control diets containing incremental additions of MCP. As with dose response data, an exponential curve best described this relationship and was used to calculate the product equivalencies.

**Example 19: Performance in animal feed in broiler chicks**

Summary of results:

BP17 showed superior performance, tibia ash and nutrient digestibility compared to an *E. coli* Phytase.

- BP17 was on average, 33-35% more effective than the *E. coli* phytase based on the exponential curves for tibia ash content and Retained Phosphorus
- BP17 at 1000 FTU/kg feed was able to fully restore bodyweight gain, FCR and tibia ash to the level of the birds fed the diet with 1.8 g P from MCP
- BP17 at 656 FTU/kg feed was equivalent to 1.2 g/kg feed of P from MCP based on the bodyweight gains, tibia ash and retained P, whereas, in contrast, 833 FTU/kg feed of the *E. coli* phytase was required based on the same parameters

Results:

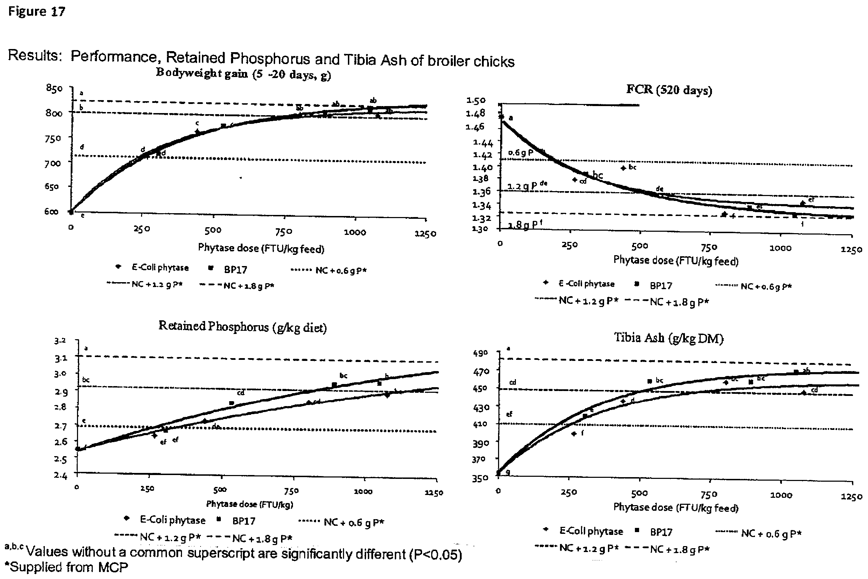

The results of this example are shown in Figure 17.

Table 19.1: Feed intakes (g, 5-20 days)

| | Phytase (FTU/kg feed) | | | | | NC +0.6 g P | NC +1.2 g P | NC +1.8g P |
|---|---|---|---|---|---|---|---|---|
| | **0** | **250** | **500** | **750** | **1000** | | | |
| *E. coli* Phytase | 885[d] | 985[c] | 1074[ab] | 1072[ab] | 1087[a] | 1002[c] | 1087[a] | 1092[a] |
| BP17 | | 1000[c] | 1059[b] | 1 082[ab] | 1087[a] | | | |
| a,b,c Values without a common superscript are significantly different (P<0.05) | | | | | | | | |

Equivalence of BP17 in FTU compared to 1 FTU of the *E. coli* phytase for bodyweight gain (5-20 days), Tibia Ash (day 20) and retained P. The table below shows the relative bioefficacy of BP17 versus the *E. coli* phytase.

Table 19.2: relative bioefficacy of phytases

| | **250 FTU** | **500 FTU** | **750 FTU** | **1000 FTU** | **Average** |
|---|---|---|---|---|---|
| Bodyweight gain | 1.08 -8% | 1.02 -2% | 0.95 +5% | 0.85 +18% | 0.98 +2% |
| Tibia Ash | 0.85+18% | 0.79+27% | 0.72+39% | 0.63+59% | 0.75+33% |
| Retained P | 0.75+33% | 0.74+35% | 0.73+37% | 0.72+39% | 0.74+35% |

Table 19.3: Levels (FTU/kg feed) of BP17 and the *E. coli* Phytase equivalent to 0.6 g or 1.2 g/kg feed of P from MCP for bodyweight, tibia ash and retained P.

| | 0.6 g P from MCP | | | | 1.2 g P from MCP | | | |
|---|---|---|---|---|---|---|---|---|
| | **Body weightgain (g)** | **Tibia Ash (g/kg DM)** | **Retained P** | **Mean** | **Body weightgain (g)** | **Tibia Ash (g/kg DM)** | **Retained P** | **Mean** |
| *E. coli* Phytase | 264 | 232 | 463 | 320 (100) | 666 | 690 | 1144 | 833 (100) |
| BP17 | 284 | 199 | 343 | 275 (86) | 648 | 508 | 813 | 656 (79) |

Table 19.4: Ileal Digestibility of nutrients (%, day 20)

| | Phytase (FTU/kg feed) | | | | | NC +0.6g P | NC +1.2g P | NC +1.8g P |
|---|---|---|---|---|---|---|---|---|
| | 0 | 250 | 500 | 750 | 1000 | | | |
| Crude protein | | | | | | | | |
| *E. coli* Phytase | 77.4[fg] | 78.4[def] | 79.3[cde] | 79.7[bc] | 80.7[sb] | 76.8[g] | 79.6[bcd] | 79.3[cde] |
| BP17 | | 78.2[ef] | 79.2[cde] | 81.4[a] | 81.1[a] | | | |
| Total Amino acids | | | | | | | | |
| *E. coli* Phytase | 80.0[ef] | 80.8[de] | 81.9[cd] | 82.3[bc] | 83.3[ab] | 79.3[f] | 82.1[c] | 81.9[cd] |
| BP17 | | 80.7[e] | 83.89[a] | 81.8[cd] | 83.5[a] | | | |
| Lysine (%) | | | | | | | | |
| *E. coli* Phytase | 84.4[de] | 84.8[cd] | 85.4[bc] | 85.4[bc] | 86.2[ab] | 83.8[e] | 85.3[c] | 84.7[cd] |
| BP17 | | 84.6[cd] | 86.6[a] | 82.3[c] | 86.4[a] | | | |
| Methionine (%) | | | | | | | | |
| *E. coli* Phytase | 89.0[g] | 90.1[de] | 90.7[bcde] | 90.8[bcd] | 91.5[ab] | 89.2[fg] | 91.5[abc] | 91.1[bc] |
| BP17 | | 89.9[ef] | 92.0[a] | 90.6[cde] | 91.5[ab] | | | |
| Threonine (%) | | | | | | | | |
| *E. coli* Phytase | 71.0[g] | 72.8[ef] | 74.1[de] | 74.7[bcd] | 76.1[ab] | 71.4[fg] | 74.7[bcd] | 74.2[cde] |
| BP17 | | 73.0[ef] | 76.6[a] | 73.8[de] | 75.8[abc] | | | |
| [a,b,c] Values without a common superscript are significantly different (P<0.05) | | | | | | | | |

Table 19.5: Total tract Calcium and Phosphorus digestibility (%)

| | Phytase (FTU/kg feed) | | | | | NC +0.6g P | NC +1.2g P | NC +1.8 g P |
|---|---|---|---|---|---|---|---|---|
| | 0 | 250 | 500 | 750 | 1000 | | | |
| Phosphorus | | | | | | | | |
| *E. coli* Phytase | 59.3[e] | 61.4[de] | 63.5[cd] | 66.3[b] | 67.5[ab] | 54.8[f] | 53.1[fg] | 50.9[g] |
| BP17 | | 62.0[d] | 66.2[bc] | 68.7[ab] | 69.1[a] | | | |
| Calcium | | | | | | | | |
| *E. coli* Phytase | 45.8[e] | 45.6[e] | 48.3[d] | 51.8[ab] | 52.5[a] | 44.5[e] | 48.3[d] | 51.9[ab] |
| BP17 | | 44.3[e] | 48.9[cd] | 50.5[bc] | 51.9[ab] | | | |
| [a,b,c] Values without a common superscript are significantly different (P<0.05 | | | | | | | | |

Table 19.6: Diets: (kg/tonne) as fed

| | Negative control | NC +0.6 g P* | NC +1.2 g P* | NC +1.8 g P* |
|---|---|---|---|---|
| Maize | 560.3 | 560.3 | 560.3 | 560.3 |
| Maize Gluten Meal 60 | 14.8 | 14.8 | 14.8 | 14.8 |
| Soybean Meal 48%CP | 300.5 | 300.5 | 300.5 | 300.5 |
| Rapeseed Meal | 38 | 38 | 38 | 38 |
| Maize/Wheat Starch | 2.5 | 2.5 | 2.5 | 2.5 |
| Soybean Oil | 21.1 | 21.1 | 21.1 | 21.1 |
| Pig/Poultry Fat | 20 | 20 | 20 | 20 |

(continued)

| | Negative control | NC +0.6 g P* | NC +1.2 g P* | NC +1.8 g P* |
|---|---|---|---|---|
| L-Lysine HCl | 1.69 | 1.69 | 1.69 | 1.69 |
| DL-methionine | 2.24 | 2.24 | 2.24 | 2.24 |
| L-threonine | 0.37 | 0.37 | 0.37 | 0.37 |
| Sodium Bicarbonate | 1.67 | 1.67 | 1.67 | 1.67 |
| Salt | 2.44 | 2.44 | 2.44 | 2.44 |
| Limestone | 13.68 | 14.63 | 13.55 | 12.47 |
| Monocalcium Phosphate | 1.001 | 3.668 | 6.334 | 9.001 |
| Poultry Vits/TE's | 5.0 | 5.0 | 5.0 | 5.0 |
| Diamol | 14.73 | 11.12 | 9.53 | 7.94 |
| BP17 (FTU/kg feed) | 0/250/500/750/1000 | - | - | - |
| *E. coli* Phytase (FTU/kg) | 0/250/500/750/1000 | - | - | - |
| | | | | |
| Crude Protein (%) | 21.54 | 21.54 | 21.54 | 21.54 |
| ME kcals/kg *(MJlkg)* | 3102 *(12.98)* | 3102 *(12.98)* | 3102 *(12.98)* | 3102 *(12.98)* |
| Calcium (%) | 0.70 | 0.79 | 0.79 | 0.79 |
| Total P (%) | 0.42 | 0.48 | 0.54 | 0.60 |
| Digestible P (%) | 0.16 | 0.22 | 0.27 | 0.32 |
| Phytate P (%) | 0.26 | 0.26 | 0.26 | 0.26 |
| Available P (%) | 0.15 | 0.21 | 0.27 | 0.33 |
| Lysine (%) | 1.25 | 1.25 | 1.25 | 1.25 |
| Digestible Lysine (%) | 1.08 | 1.08 | 1.08 | 1.08 |
| Methionine (%) | 0.57 | 0.57 | 0.57 | 0.57 |
| Digestible Methionine (%) | 0.55 | 0.55 | 0.55 | 0.55 |
| Methionine +cystine (%) | 0.94 | 0.94 | 0.94 | 0.94 |
| Digestible methionine + cystine (%) | 0.80 | 0.80 | 0.80 | 0.80 |
| *Supplied from MCP | | | | |

Design of the trial:

288 male Ross 308 broiler chicks were allocated to 12 treatments with 6 cage replicates per treatment (4 birds per cage). All birds received a standard diet for the first 5 days - Crude Protein 21.5%; AME 2900 kcal/kg (12.1 MJ/kg). The Negative Control (NC) diet was based on corn/soybean meal and was reduced in phosphorus content (0.42% total phosphorus, 0.15% available phosphorus) and was supplemented with either BP17 or an *E. coli* phytase at 250, 500, 750 or 1000 FTU/kg feed. The dosages in feed were checked at 2 laboratories following dosing and the actual analysed levels were then used for all subsequent modelling. The 3 Positive Control diets contained incremental additions of monocalcium phosphate (+0.6, +1.2 and +1.8 g/kg feed P) to the NC formulation. All diets were fed as mash from days 5-20. Birds were weighed days 5 and 20 and FCR calculated. Excreta was collected from each cage days 17-20 for the determination of P and Ca. On day 20 all birds were euthanased and the left tibia dissected out for determination of bone ash. The ileal contents were also taken for measurement of protein and amino acid digestibility.

Dose response relationships were determined using an exponential model of the form: $Y = A + B*R^X$ where $Y$ = response parameter, $A$ = upper asymptote value, $B$ = maximum response value, $R$ = non-linear slope parameter, $X$ = dosed phytase activity (FTU/kg feed).

Equivalencies of BP17 and the *E. coli* phytase (in FTU/kg feed) were calculated for both bodyweight gain and tibia ash (%0 using the data for the positive control diets containing incremental additions of MCP. As with dose response data, an exponential curve best described this relationship and was used to calculate the product equivalencies.

**Example 20: Performance in animal feed in broiler chicks**

Summary of results:

BP17 showed superior performance and tibia ash compared to Phyzyme® XP.

- BP17 was on average, 82% more effective than Phyzyme® XP based on the exponential curve for tibia ash content and by 85% based on bodyweight gain
- BP17 at 500 FTU/kg feed was able to fully restore bodyweight gain and FCR to the level of the birds fed the diet with 1.8 g P from MCP and 1000 FTU/kg feed was able to fully restore tibia ash
- BP17 at 468 FTU/kg feed was equivalent to 1.2 g/kg feed of P from MCP whereas, in contrast, 988 FTU/kg feed of Phyzyme® XP was required based on bodyweight gain
- BP17 at 680 FTU/kg feed was equivalent to 1.2 g/kg feed of P from MCP based on tibia ash, whereas Phyzyme® XP was unable to reach the response level of the 1.2 g/kg feed of P from MCP in the dose range tested

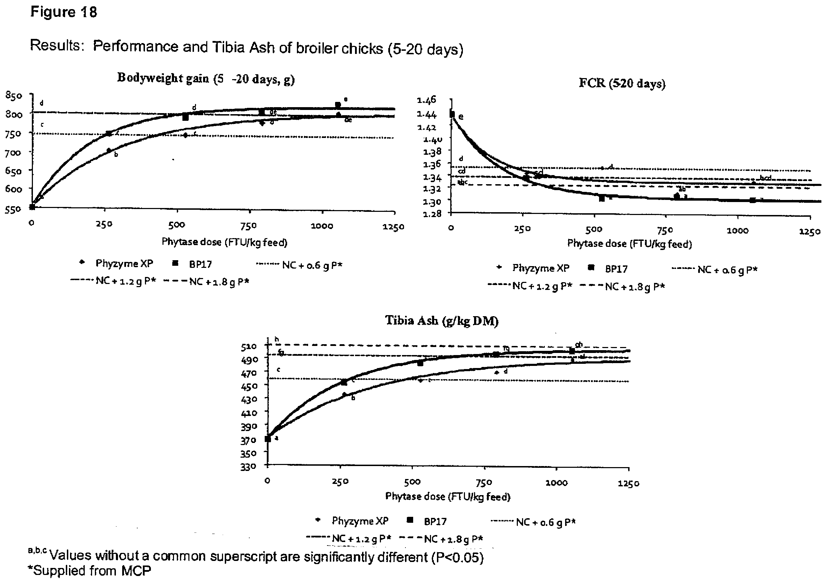

The results of this example are shown in Figure 18.

Table 20.1: Results: Feed intakes (g, 5-20 days)

| | Phytase (FTU/kg feed) | | | | | NC +0.6 g P | NC +1.2g P | NC +1.8 g P |
|---|---|---|---|---|---|---|---|---|
| | 0 | 250 | 500 | 750 | 1000 | | | |
| Phyzyme® XP | 792[a] | 948[b] | 1012[c] | 1029[cd] | 1077[e] | 1011[C] | 1075[e] | 1063[de] |
| BP17 | | 1003[c] | 1037[cd] | 1059[de] | 1086[e] | | | |

Equivalence of BP17 in FTU compared to 1 FTU of Phyzyme® XP for bodyweight gain (5-20 days) and Tibia Ash (day 20), and relative bioefficacy of BP17 versus Phyzyme® XP.

Table 20.2: relative bioefficacy of phytases

| | 250 FTU | 500 FTU | 750 FTU | 1000 FTU | Average |
|---|---|---|---|---|---|
| Bodyweight gain | 0.60 | 0.57 | 0.53 | 0.47 | 0.54 |
| | +67% | +75% | +89% | +113% | +85% |
| Tibia Ash | 0.61 | 0.57 | 0.53 | 0.48 | 0.55 |
| | +64% | +75% | +87% | +108% | +82% |

Table 20.3: Levels (FTU/kg feed) of BP17 and Phyzyme XP equivalent to 0.6 g or 1.2 g/kg feed of P from MCP.

| | 0.6 g P from MCP | | | 1.2 g P from MCP* | | |
|---|---|---|---|---|---|---|
| | Body weight gain(g) | Tibia Ash (g/kg DM) | Mean | Body weight gain (g) | Tibia Ash (g/kg DM) | Mean |
| Phyzyme® XP | 459 | 493 | 476 (100) | 988 (100) | - | - |
| BP17 | 264 | 283 | 274 (58) | 468 (47) | 680 | - |
| *If the performance level of the MCP was not reached an equivalency could not be calculated and therefore no value is shown. | | | | | | |

Table 20.4: Diets: (kg/tonne) as fed

| | Negative control | NC + 0.6 g P* | NC + 1.2 g P* | NC + 1.8 g P* |
|---|---|---|---|---|
| Maize | 584.2 | 584.2 | 584.2 | 584.2 |
| Maize Gluten Meal 60 | 10.0 | 10.0 | 10.0 | 10.0 |
| Soybean Meal 48%CP | 297.0 | 297.0 | 297.0 | 297.0 |
| Rapeseed Meal | 30 | 30 | 30 | 30 |
| Maize/Wheat Starch | 2.5 | 2.5 | 2.5 | 2.5 |
| Soybean Oil | 15.0 | 15.0 | 15.0 | 15.0 |
| Pig/Poultry Fat | 14.9 | 14.9 | 14.9 | 14.9 |
| L-Lysine HCl | 1.60 | 1.60 | 1.60 | 1.60 |
| DL-methionine | 2.30 | 2.30 | 2.30 | 2.30 |

(continued)

| | Negative control | NC + 0.6 g P* | NC + 1.2 g P* | NC + 1.8 g P* |
|---|---|---|---|---|
| L-threonine | 0.30 | 0.30 | 0.30 | 0.30 |
| Sodium Bicarbonate | 1.80 | 1.80 | 1.80 | 1.80 |
| Salt | 2.40 | 2.40 | 2.40 | 2.40 |
| Limestone | 15.29 | 14.18 | 13.07 | 11.97 |
| Monocalcium Phosphate | 3.555 | 6.198 | 8.841 | 11.49 |
| Poultry Vits/TE's | 5.0 | 5.0 | 5.0 | 5.0 |
| Diamol | 14.15 | 12.62 | 11.08 | 9.55 |
| BP17 (FTU/kg feed) | 0/250/500/750/1000 | - | - | - |
| *E. coli* Phytase (FTU/kg) | 0/250/500/750/1000 | - | - | - |
| | | | | |
| Crude Protein (%) | 21 | 21 | 21 | 21 |
| ME kcals/kg *(MJ/kg)* | 3040 *(12.72)* | 3040 *(12.72)* | 3040 *(12.72)* | 3040 *(12.72)* |
| Calcium (%) | 0.80 | 0.80 | 0.80 | 0.80 |
| Total P (%) | 0.47 | 0.53 | 0.59 | 0.65 |
| Digestible P (%) | 0.21 | 0.26 | 0.31 | 0.36 |
| Phytate P (%) | 0.26 | 0.26 | 0.26 | 0.26 |
| Available P (%) | 0.20 | 0.26 | 0.32 | 0.38 |
| Lysine (%) | 1.22 | 1.22 | 1.22 | 1.22 |
| Digestible Lysine (%) | 1.05 | 1.05 | 1.05 | 1.05 |
| Methionine (%) | 0.56 | 0.56 | 0.56 | 0.56 |
| Digestible Methionine (%) | 0.54 | 0.54 | 0.54 | 0.54 |
| Methionine + cystine (%) | 0.92 | 0.92 | 0.92 | 0.92 |
| Digestible methionine + cystine (%) | 0.79 | 0.79 | 0.79 | 0.79 |
| Supplied from MCP | | | | |

Design of the trial:

288 male Ross 308 broiler chicks were allocated to 12 treatments with 6 cage replicates per treatment (4 birds per cage). All birds received a standard diet for the first 5 days - Crude Protein 21.5%; AME 2900 kcal/kg (12.1 MJ/kg). The Negative Control (NC) diet was based on corn/soybean meal and was reduced in phosphorus content (0.47% total phosphorus, 0.20% available phosphorus) and was supplemented with either BP17 Phyzyme® XP at 250, 500, 750 or 1000 FTU/kg feed. The dosages in feed were checked at 2 laboratories following dosing and the actual analysed levels were then used for all subsequent modelling. The 3 Positive Control diets contained incremental additions of monocalcium phosphate (+0.6, +1.2 and +1.8 g/kg feed P) to the NC formulation. All diets were fed as mash from days 5-20. Birds were weighed days 5 and 20 and FCR calculated. On day 20 all birds were euthanased and the left tibia dissected out for determination of bone ash.

Dose response relationships were determined using an exponential model of the form: $Y = A + B \cdot R^x$ where Y = response parameter, A = upper asymptote value, B = maximum response value, R = non-linear slope parameter, X = dosed phytase activity (FTU/kg feed).

Equivalencies of BP17 and Phyzyme® XP (in FTU/kg feed) were calculated for both bodyweight gain and tibia ash (%) using the data for the positive control diets containing incremental additions of MCP. As with dose response data, an exponential curve best described this relationship and was used to calculate the product equivalencies.

**Example 21: Comparison of efficacy of BP17 compared to other phytases in piglets**

The objective was to assess the bio-efficacy of BP17 compared to two different commercially available phytase enzymes (one *E.coli* Phytase and one *P. lycii* -derived phytase in piglets fed maize based diets, deficient in phosphorus and calcium. The aim of the study was to assess nutrient digestibility and retention as well as monitoring and recording daily feed intake, daily liveweight gain and feed use efficiency of individually housed piglets from approximately 7 to 14 days post weaning (35-38 days old weighing 8-12 kg liveweight) for a period of 22 days.

21.1 Materials and methods

21.1.1 Test Articles

The test articles were supplied as a liquid and two powder enzyme products by Danisco UK Ltd. The enzyme products were sent to Target Feeds Ltd and applied to the mash study diets, shown in Table 21.1. The formulation of the diets was provided by Danisco UK Ltd.

21.1.2 Animals

A total of 66 weaned male Landroc X piglets (three feeding runs of 22 piglets) of between 28 and 32 days of age were selected. Their average start liveweight was 10.7kg. There were 121 treatment diets, and six piglets (two in each run) were allocated to each treatment. The piglets had a six day acclimatisation period before starting the study, during which time they were fed a commercial weaner diet. The piglets were then on test for 22 days. All piglets were supplied by Rattlerow Farms Ltd and were vaccinated against enzootic pneumonia prior to delivery. The week of birth of each piglet was recorded in the study records.

21.2. Experimental Design

21.2.1 Assignment of Treatment Groups

The experimental design was a complete randomised block with 6 replicates of 11 treatments, with two replicates per run and one animal per replicate per run. There was one piglet per crate, within a room of 22 individual piglet metabolism crates. There were three runs, therefore a total of 66 male piglets were used in this study.

21.2.2 Treatments

There were 11 treatment diets as shown in Table 21.1.

Table 21.1: Dietary treatments, enzyme identification and incorporation rates

| Treatment | Enzyme* | FTU/kg feed | Inclusion (g/tonne) |
|---|---|---|---|
| T1 | Positive Control (PC) | 0 | 0 |
| T2 | Negative Control (NC) | 0 | 0 |
| T3 | NC + liquid BP17 | 250 | 50 |
| T4 | NC + liquid BP17 | 1000 | 200 |
| T5 | NC + liquid BP17 | 2000 | 400 |
| T6 | NC + Liquid *E. coli* phytase I | 250 | 50 |
| T7 | NC + Liquid *E. coli* phytase I | 1000 | 200 |
| T8 | NC + Liquid *E. coli* phytase I | 2000 | 400 |
| T9 | NC + *P. lycii* phytase | 500 | 50 |
| T10 | NC + *P. lycii* phytase | 2000 | 200 |
| T11 | NC + *P. lycii* phytase | 4000 | 400 |

Table 21.2 Diet formulations of the finished feeds

| Phase I Diets 0 - 14days | Positive Control (PC) | Negative Control (NC) |
|---|---|---|
| Ingredients in % | | |
| Maize | 55.67 | 57.1 |
| SBM 48% | 26.7 | 26.6 |

(continued)

| Phase I Diets 0 - 14days | Positive Control (PC) | Negative Control (NC) |
|---|---|---|
| Whey powder | 10.0 | 10.0 |
| Soya protein conc. | 2.5 | 2.5 |
| Soybean oil | 1.7 | 1.2 |
| L-lysine HCl | 0.135 | 0.135 |
| DL-methionine | 0.11 | 0.11 |
| L-threonine | 0.06 | 0.06 |
| TiO2 | 0.40 | 0.40 |
| Salt | 0.12 | 0.12 |
| Limestone | 0.63 | 0.98 |
| DCP | 1.47 | 0.29 |
| Vitamins and Minerals | 0.50 | 0.50 |
| **TOTAL** | **100** | **100** |
| **Calculated Nutrients & Energy** | | |
| Protein, % | 20.5 | 20.5 |
| DE, Mj/kg | 14.5 | 14.5 |
| Ca, % | 0.80 | 0.65 |
| P, % | 0.68 | 0.47 |
| Dig. P % | 0.35 | 0.20 |
| **Digestible amino acids, %** | | |
| Lys | 1.09 | 1.09 |
| Met | 0.40 | 0.40 |
| Met+Cys | 0.65 | 0.65 |
| Thr | 0.71 | 0.71 |
| Trp | 0.195 | 0.195 |

21.2.3 Daily Feed Intake

Test diets were offered ad-libitum, to the piglets, in feeding bowls in each of the crates throughout the trial period from day 0 to day 22 of each run. Each of the 11 treatment diets were fed to two piglet replicates in individual crates. The total amount consumed per piglet from day 0 to 22 was recorded. Weighed feed was added to the feed bowls daily and any food not consumed since the previous day was removed weighed and discarded.

21.2.4 Body Weight

All piglets were weighed on days 0 and 22 of the study for each of the three feeding runs. All animal weights were recorded on the Animal Weight Form. On day 0 weighing took place prior to the feeding of the test diets.

21.2.5 FCR and FCE

Feed conversion ratio (FCR) (feed intake/weight gain) and feed conversion efficiency (FCE) (weight gain/feed intake) were calculated using total feed consumed and total weight gained per piglet and per treatment over each of the three 22 day feeding periods.

21.2.6 Digestibility and Retention

Urinary and faecal production were recorded twice daily (am and pm) for each of the 22 crates during days 18 to 22 for each of the three feeding runs.

The fresh faeces were collected from each crate at least twice daily, and stored refrigerated at approximately 4°C. At the end of the collection period the total four day collection for each animal was weighed, and thoroughly mixed.

Due to small amounts of faecal material collected only one set of samples from each animal was weighed and dried at 55°C to determine individual sample dry matter (DM). After drying the individual animal samples of dried faecal material were sent to Eurofins Ltd and analysed for total phosphorus, calcium, total nitrogen, ash, and gross energy.

Urine was collected from days 18 to 22 of each of the three feeding runs. Prior to the start of urine collection on the first day, 25 ml of 25% v/v sulphuric acid was placed into the air tight container (carboy). One carboy was used for urine collection from each of the piglet crates. A further 25ml of sulphuric acid was added to each carboy containing urine each morning, to prevent volatilisation of the nitrogen fraction. At the end of the collection period the total four day collection for each animal was weighed.

At the end of the collection period two representative duplicate samples of urine (each of approximately 100g) were taken from each carboy. The first sample was retained frozen at ADAS Drayton and the second sample was sent chilled on ice packs to Eurofins Ltd and analysed for dry matter, total nitrogen, total phosphorus and calcium at the end of each run.

## 21.3 Results

Table 21.3 :Effect of increasing dose of different phytases fed at 1,000 FTU, except *P. lycii* phytase that was fed at 2,000 FTU, on performance of weaned piglets

| Item | Diet 1 | Diet 2 | Diet 4 | Diet 7 | Diet 10 | P value |
|---|---|---|---|---|---|---|
| Phytase source | PC | NC | BP17 | *E. coli* | *P. lycii* | |
| Planned level | 0 | 0 | 1,000 | 1,000 | 2,000 | |
| ADFI, g | 443 | 470 | 484 | 485 | 480 | 0.968 |
| ADG, g | 267 | 293 | 329 | 323 | 311 | 0.488 |
| FCR | 1.67 | 1.6 | 1.47 | 1.5 | 1.56 | 0.062 |
| GF | 0.602 | 0.628 | 0.68 | 0.671 | 0.645 | 0.064 |

Table 21.4 Effect of different phytases in comparison to PC and NC on digestibility of P and Ca and retention of P and Ca in weaned piglets

| BP17 phytase | | | | | | | | |
|---|---|---|---|---|---|---|---|---|
| | Diet 2 | Diet 3 | Diet 4 | Diet 5 | | P value | | |
| | NC | Phy B | Phy B | Phy B | | Anova | Linear | Quadratic |
| Phytase dose | 0 | 250 | 1000 | 2000 | | | | |
| P digestibility, % | 72.1$^X$ | 75.5$^x$ | 85.3$^y$ | 87.4$^y$ | | 0.001 | 0.001 | 0.105 |
| Ca digestibility, % | 80.9$^x$ | 83.3$^{xy}$ | 89.0$^{yz}$ | 90.9$^z$ | | 0.011 | 0.002 | 0.594 |
| P retention, % | 69.8$^X$ | 74.4$^X$ | 81.8$^y$ | 86.5$^y$ | | 0.001 | 0.001 | 0.119 |
| Ca retention, % | 65.0$^X$ | 71.7$^{xy}$ | 76.1$^{yz}$ | 82.1$^z$ | | 0.006 | 0.001 | 0.462 |

(continued)

| *E. coli* phytase | | | | | | | | |
|---|---|---|---|---|---|---|---|---|
| | Diet 2 | Diet 6 | Diet 7 | Diet 8 | | P value | | |
| | NC | PXP | PXP | PXP | | Anova | Linear | Quadratic |
| Phytase dose | 0 | 250 | 1000 | 2000 | | | | |
| P digestibility, % | 72.1xy | 71.6x | 77.6y | 84.0y | | 0.001 | 0.001 | 0.841 |
| Ca digestibility, % | 80.9x | 81.4x | 82.8x | 91.9y | | 0.002 | 0.008 | 0.124 |
| P retention, % | 69.8x | 70.5xy | 76.5y | 83.1z | | 0.001 | 0.001 | 0.991 |
| Ca retention, % | 65.0x | 70.0x | 69.0x | 86.8y | | 0.001 | 0.004 | 0.083 |
| *P. lycii* phytase | | | | | | | | |
| | Diet 2 | Diet 9 | Diet 10 | Diet 11 | | P value | | |
| | NC | *P. lycii* | *P. lycii* | *P. lycii* | | Anova | Linear | Quadratic |
| Phytase dose | 0 | 500 | 2000 | 4000 | | | | |
| P digestibility, % | 72.1 | 72.6 | 72.6 | 77.9 | | 0.333 | 0.334 | 0.467 |
| Ca digestibility, % | 80.9 | 85.4 | 82.3 | 82.6 | | 0.533 | 0.951 | 0.755 |
| P retention, % | 69.8 | 71.3 | 70 | 76.4 | | 0.265 | 0.368 | 0.383 |
| Ca retention, % | 65 | 72.9 | 68.4 | 70.7 | | 0.388 | 0.581 | 0.769 |
| Statistical analysis to compare phytase sources | | | | | | | | |
| | | | ANOVA | | | Contrasts[1] | | |
| Item | | | P values | | | P values | | |
| | | Phytase | FTU | Phytase x FTU | | BP17 vs. PXP | BP17 vs. *P. lycii* | PXP vs. *P. lycii* |
| P digestibility , % | | 0.001 | 0.001 | 0.188 | | <0.05 | <0.05 | <0.05 |
| Ca digestibility, % | | 0.047 | 0.002 | 0.002 | | Ns | <0.05 | Ns |
| P retention, % | | 0.001 | 0.001 | 0.195 | | <0.05 | <0.05 | <0.05 |
| Ca retention, % | | 0.039 | 0.001 | 0.009 | | Ns | <0.05 | Ns |
| x,y,z, Means within each row with different supersccripts are significantly different (P<0.05)<br>1. Contrasts used to determine significant differences in the mean response between Phytase sources at all 3 dose levels | | | | | | | | |

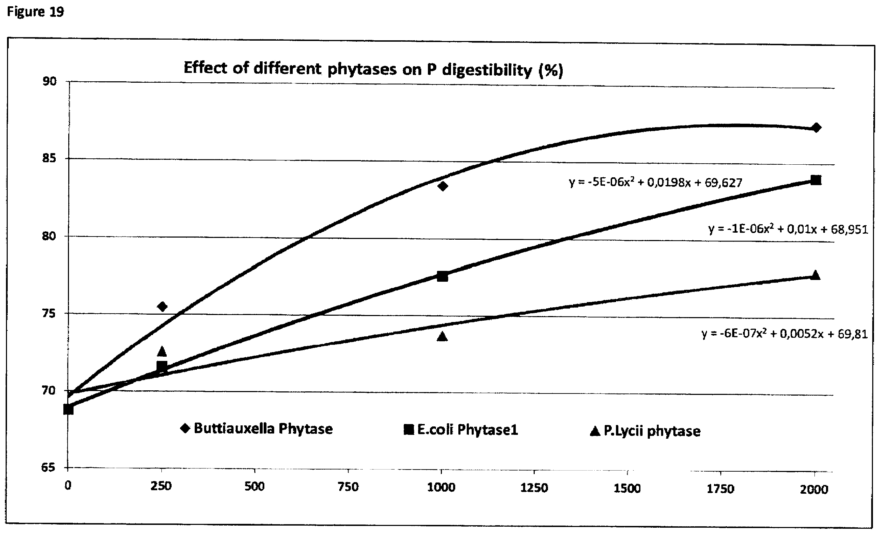

The above tables show both BP17 and *E. coli* phytase were able to significantly increase P and Ca digestibility and P and Ca retention in a linear manner, while *P. lycii* phytase only numerically (P>0.1) increased these variables. Using contrast statements to statistically compare the mean response to BP17 phytase with either *E. coli* or *P. lycii* phytase sources showed BP17 phytase to be significantly better than either *E. coli* or *P. lycii* phytase at improving P digestibility and P retention. These results are illustrated by Figure 19.

**Example 22: The efficacy of BP17 phytase on amino acid digestibility in weaned piglets fed a corn-soy based diet**

In order to optimize phytase efficacy, it is important to develop phytase that would be able to improve not only phosphorus but also amino acid digestibility. This would not only reduce feed cost and increase growth uniformity but also reduce nutrient (P and N) excretion. In this example, the efficacy supplementing graded level of BP17 phytase on ileal amino acid digestibility was investigated using weanling cannulated pigs.

**MATERIALS AND METHODS**

A total of 16 weanling pigs that are cannulated using the simple T cannula fitted at about 6 cm anterior to the ileo-cecal-colonic junction were used in this study as an incomplete Latin Square design. There were 2 periods of 4 blocks of pigs with 4 pigs/block. Each diet was represented in each block and each pig received a different diet in each of the 2 periods. Feed intake was restricted to 4.5% of the weight of the lightest pig within each block. At the end of period I all pigs were fed a positive control diet (rest diet) for 5 days after which the second period was commenced. Each diet was fed for 9 continuous days. Fresh fecal samples were randomly collected the mornings and evenings of day 5 and 6 while ileal digesta was collected for 12 hours/day on days 7, 8, and 9. Each pig was weighed individually at the start and end of each period.

**Dietary Treatments**

One basal diet that is corn-soybean meal-corn DDGS-wheat middlings-soybean meal based was made (NC diet). To the NC diet, BP17 phytase was added in increasing order of 500, 1000, and 2000 FTU phytase/kg diet to make diets 2, 3, and 4, respectively. The analyzed energy, mineral, and amino acid compositions of the 4 diets are shown in Tables 22.2 and 22.3.

Mineral, nitrogen, and energy contents of the experimental diets (Table 22.1 and 22.2) and amino acids (Table 22.3) showed that minerals, amino acids, and energy contents are similar across the four diets.

Table 22.1: Diet composition

| Description of diets | 1 | 2 | 3 | 4 | 5 |
|---|---|---|---|---|---|
| BP17 | 0 | 500 | 1000 | 2000 | Rest diet |
| Ingredients, g/kg | NC (T1) | T2 | T3 | T4 | PC |
| Corn | 506.3 | 496.3 | 496.3 | 496.3 | 533.8 |
| Wheat midds | 71.9 | 71.9 | 71.9 | 71.9 | 73 |
| Corn DDGS | 72.4 | 72.4 | 72.4 | 72.4 | 72.4 |
| Soybean Meal, 48%CP | 271 | 271 | 271 | 271 | 258 |
| L-Lysine HCl | 2 | 2 | 2 | 2 | 2 |
| DL-Met | 0.6 | 0.6 | 0.6 | 0.6 | 0.1 |
| L-Threonine | 1.5 | 1.5 | 1.5 | 1.5 | 0.9 |
| Soy oil | 28 | 28 | 28 | 28 | 28 |
| Monocalcium Phosphate | 0 | 0 | 0 | 0 | 11.3 |
| Salt | 4 | 4 | 4 | 4 | 4 |
| Limestone (A) | 13 | 13 | 13 | 13 | 12.2 |
| Titanium Oxide (B) | 25 | 25 | 25 | 25 | 0 |
| Vitamin Premix (C) | 2.5 | 2.5 | 2.5 | 2.5 | 2.5 |
| Mineral Premix (D) | 1.3 | 1.3 | 1.3 | 1.3 | 1.3 |
| Selenium Premix (E) | 0.5 | 0.5 | 0.5 | 0.5 | 0.5 |
| T2 (phytase at 500 units/kg) (F) | 0 | 10 | 0 | 0 | 0 |
| T3 (phytase at 1000 units/kg) (G) | 0 | 0 | 10 | 0 | 0 |
| T4 (phytase at 2000 units/kg) (H) | 0 | 0 | 0 | 10 | 0 |
| Ground corn | 0 | 0 | 0 | 0 | 0 |
| Total | 1000.0 | 1000.0 | 1000.0 | 1000.0 | 1000.0 |

(continued)

| Calculated Nutrients & Energy | | | | | |
|---|---|---|---|---|---|
| Ditets | 1798 | 1799 | 1800 | 1801 | 1802 |
| Protein, g/kg | 189 | 189 | 189 | 189 | 183 |
| DE, kcal /kg | 3307 | 3306 | 3306 | 3306 | 3289 |
| ME, k cal /kg | 3138 | 3137 | 3137 | 3137 | 3123 |
| Ca, g/kg | 6.25 | 6.25 | 6.25 | 6.25 | 5.91 |
| P, g/kg | 4.57 | 4.57 | 4.57 | 4.57 | 4.51 |
| Non-phytate P, g / kg | 1.34 | 1.34 | 1.34 | 1.34 | 1.33 |
| Ca:P | 1.4 | 1.4 | 1.4 | 1.4 | 1.3 |
| Ca:NPP | 4.7 | 4.7 | 4.7 | 4.7 | 4.5 |
| Apparent Ileal Dig. AA, g/kg | | | | | |
| Arg | 11.3 | 11.3 | 11.3 | 11.3 | 10.9 |
| His | 4.5 | 4.5 | 4.5 | 4.5 | 4.4 |
| Ile | 6.9 | 6.9 | 6.9 | 6.9 | 6.7 |
| Leu | 14.9 | 14.9 | 14.9 | 14.9 | 14.6 |
| Lys | 9.9 | 9.9 | 9.9 | 9.9 | 9.6 |
| Met | 3.3 | 3.3 | 3.3 | 3.3 | 2.8 |
| Met + Cys | 6.1 | 6.1 | 6.1 | 6.1 | 5.5 |
| Phe | 8.3 | 8.3 | 8.3 | 8.3 | 8.1 |
| Phe + Tyr | 14.2 | 14.2 | 14.2 | 14.2 | 13.8 |
| Thr | 6.9 | 6.9 | 6.9 | 6.9 | 6.2 |
| Trp | 1.8 | 1.8 | 1.8 | 1.8 | 1.8 |
| Val | 7.6 | 7.6 | 7.6 | 7.6 | 7.4 |

Table 22.2: Analyzed mineral (g/100 g DM) and gross energy (kcal/kg DM) contents of the experimental diets

| Diets | 1 | 2 | 3 | 4 |
|---|---|---|---|---|
| | NC | NC+500 | NC+1000 | NC+2000 |
| Nitrogen | 3.74 | 3.85 | 3.67 | 3.74 |
| Calcium | 1.14 | 1.14 | 1.08 | 1.01 |
| Phosphorus | 0.62 | 0.62 | 0.62 | 0.62 |
| Sodium | 0.22 | 0.22 | 0.19 | 0.18 |
| Magnesium | 0.24 | 0.25 | 0.25 | 0.24 |
| Potassium | 1.32 | 1.28 | 1.29 | 1.26 |
| Copper | 0.00 | 0.00 | 0.00 | 0.00 |
| Iron | 0.05 | 0.03 | 0.03 | 0.03 |
| Zinc | 0.01 | 0.02 | 0.01 | 0.01 |
| Chloride | 0.38 | 0.38 | 0.33 | 0.32 |
| Phytic Acid | 0.15 | 0.21 | 0.24 | 0.28 |

(continued)

| Diets | 1 | 2 | 3 | 4 |
|---|---|---|---|---|
| Gross energy | 4687 | 4679 | 4726 | 4695 |

Table 22.3 Amino acid contents of the experimental diets

| Diets | 1 | 2 | 3 | 4 |
|---|---|---|---|---|
| | NC | NC+500 | NC+1000 | NC+2000 |
| *Essential amino acid, %* | | | | |
| Arg | 1.58 | 1.56 | 1.50 | 1.56 |
| His | 0.63 | 0.63 | 0.61 | 0.63 |
| Ile | 1.01 | 1.01 | 0.99 | 1.04 |
| Leu | 2.15 | 2.13 | 2.06 | 2.14 |
| Lys | 1.53 | 1.50 | 1.46 | 1.49 |
| Met | 0.46 | 0.44 | 0.41 | 0.45 |
| Phe | 1.20 | 1.19 | 1.15 | 1.19 |
| Thr | 1.06 | 1.05 | 0.99 | 1.01 |
| Trp | 0.26 | 0.28 | 0.26 | 0.25 |
| Val | 1.16 | 1.16 | 1.14 | 1.19 |
| *Nonessential amino acid, %* | | | | |
| Ala | 1.25 | 1.24 | 1.20 | 1.24 |
| Asp | 2.32 | 2.30 | 2.19 | 2.31 |
| Cys | 0.46 | 0.47 | 0.46 | 0.49 |
| Glu | 3.95 | 3.91 | 3.73 | 3.96 |
| Gly | 1.00 | 1.00 | 0.97 | 1.00 |
| Pro | 1.46 | 1.48 | 1.42 | 1.47 |
| Ser | 1.09 | 1.07 | 1.00 | 1.03 |
| Tyr | 0.85 | 0.84 | 0.81 | 0.85 |
| Total | 23.67 | 23.49 | 22.57 | 23.52 |

**Sample Processing and Chemical Analysis**

Prior to analysis, diets were ground to pass through 0.5 mm screen. Then all the diet samples (4) were analyzed for dry matter, nitrogen, phosphorus, sodium, magnesium, potassium, copper, iron, zinc, chloride, phytic acid, and gross energy. Dry matter in diets was determined by drying the samples in drying oven for 24 hours. Gross energy content was determined in a Parr adiabatic calorimeter using Benzoic acid as a calibration standard. Crude protein was determined using the combustion method with EDTA as calibration standard; the crude protein content was then calculated as N multiplied by a factor of 6.25. Analysis for P and Ca was preceded by nitric and perchloric acid digestion of samples. The digest was subsequently used for P and Ca analyses using spectrophotometric and flame atomic absorption procedures, respectively.

**Statistics**

Data were analyzed as a replicated Latin rectangle design using SAS. Means were separated by linear and quadratic contrasts. Contrast coefficients for unequal spacing were generated using Proc IML of SAS. Least squares means were presented, P-values $\leq$ 0.05 were considered significant.

**RESULTS**

Contrasts showed that supplementation of the NC diet with 500 units of BP17 phytase resulted in higher (trends for Ala, Gly, Glu, and Lys) ileal AA digestibility except for methionine (Table 22.4). Increasing level (0, 500, 1,000, and 2,000) of BP17 phytase supplementation resulted in linear increase in ileal AA digestibility (7 AAs, Table 22.4). There was no quadratic effect of phytase supplementation on ileal AA digestibility.

Table 22.4: Ileal amino acid digestibility

| Diet | 1 | 2 | 3 | 4 | | | | | Probability of contrasts | | |
|---|---|---|---|---|---|---|---|---|---|---|---|
| | NC+0 | NC+500 | NC+1000 | NC+2000 | | | | | 0 vs. 500 | Linear | Quadratic |
| | | | | | | SE | Diet | | | | |
| Indispensable amino acid, % | | | | | | | | | | | |
| Arg | 87.3 | 89.6 | 88.8 | 90.3 | | 0.359 | 0.00 | | 0.001 | 0.001 | 0.265 |
| His | 80.9 | 84.8 | 82.4 | 84.8 | | 1.165 | 0.04 | | 0.037 | 0.122 | 0.635 |
| Ile | 80.0 | 83.4 | 82.3 | 84.6 | | 1.043 | 0.02 | | 0.041 | 0.026 | 0.585 |
| Leu | 80.9 | 84.4 | 83.2 | 84.9 | | 0.911 | 0.02 | | 0.019 | 0.034 | 0.375 |
| Lys | 83.3 | 86.8 | 84.8 | 87.1 | | 1.167 | 0.06 | | 0.055 | 0.122 | 0.672 |
| Met | 85.6 | 87.1 | 85.9 | 88.1 | | 1.122 | 0.26 | | 0.363 | 0.227 | 0.784 |
| Phe | 81.1 | 84.8 | 83.2 | 85.1 | | 0.878 | 0.01 | | 0.012 | 0.030 | 0.381 |
| Thr | 74.9 | 79.3 | 76.0 | 77.7 | | 1.200 | 0.05 | | 0.023 | 0.408 | 0.474 |
| Try | 76.4 | 81.4 | 77.8 | 77.8 | | 1.350 | 0.08 | | 0.021 | 0.960 | 0.237 |
| Val | 76.4 | 80.8 | 78.9 | 81.3 | | 1.197 | 0.02 | | 0.024 | 0.050 | 0.458 |
| | | | | | | | | | | | |
| Dispensable amino acid, % | | | | | | | | | | | |
| Ala | 76.3 | 80.3 | 78.2 | 80.4 | | 1.363 | 0.09 | | 0.064 | 0.152 | 0.602 |
| Asp | 77.1 | 80.8 | 78.6 | 81.3 | | 0.948 | 0.01 | | 0.016 | 0.036 | 0.679 |
| Cys | 69.7 | 76.9 | 74.1 | 76.3 | | 2.024 | 0.06 | | 0.027 | 0.131 | 0.306 |
| Glu | 82.7 | 86.1 | 84.1 | 86.0 | | 0.751 | 0.01 | | 0.007 | 0.050 | 0.461 |
| Gly | 66.0 | 72.4 | 69.6 | 71.1 | | 2.176 | 0.16 | | 0.058 | 0.303 | 0.363 |
| Pro | 78.8 | 82.2 | 80.4 | 81.6 | | 1.201 | 0.15 | | 0.066 | 0.299 | 0.475 |
| Ser | 78.9 | 83.8 | 80.7 | 81.3 | | 0.882 | 0.01 | | 0.002 | 0.464 | 0.112 |
| Tyr | 82.1 | 85.2 | 83.3 | 85.2 | | 0.899 | 0.03 | | 0.030 | 0.107 | 0.603 |
| **Total** | **79.4** | **83.1** | **81.1** | **83.3** | | **1.078** | **0.03** | | **0.032** | **0.096** | **0.584** |
| n | 8 | 8 | 8 | 8 | | | | | | | |

**Fish Examples**

**Example 23: Effect of graded supplementation levels of BP17 phytase on the apparent digestibility of nutrients and energy in *Nile tilapia* fed a fishmeal free diet**

The objective of this experiment was to investigate the effect of graded supplemental doses of BP17 phytase on the apparent digestibility of nutrients, energy and amino acids in Nile Tilapia fed a fishmeal free diet.

## MATERIALS AND METHODS

23.1 Rearing conditions

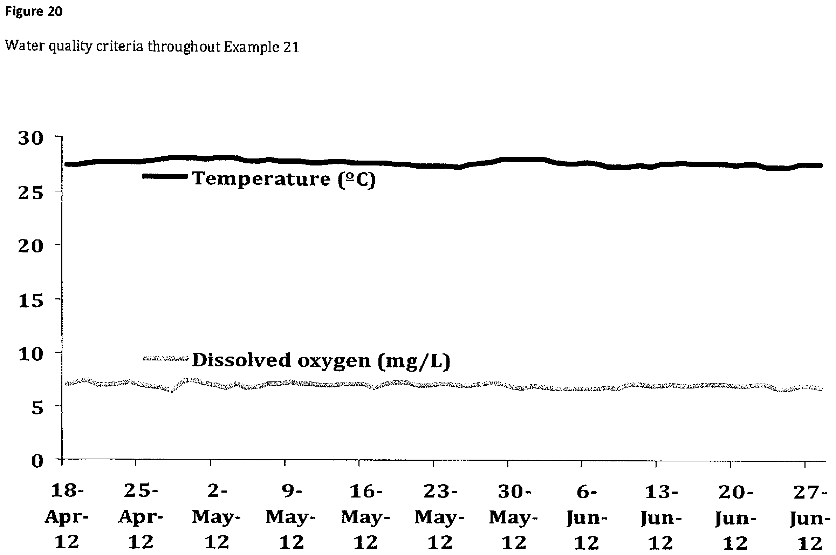

For experimental purposes, fish were subjected to moderate anaesthesia (20 μl/L of AQUI-S™, New Zealand), individually weighed and selected according to body weight range. Homogenous groups of 12 tilapia juveniles, with a mean initial body weight (IBW) of 56 ± 3 g were stocked in each tank. The experiment was conducted in cylindroconical fiberglass tanks (60 L) at indoor facilities, supplied with recirculated fresh water (water-flow rate: 3.5 L/min) at a constant water temperature of 27.8 ± 0.2°C. A light:dark photoperiod cycle of 14:10 h was adopted. Water quality parameters including dissolved oxygen, temperature (daily), ammonia and pH (weekly) were monitored, recorded and kept within the comfort range for the species (see Figure 20). Prior to initiation of the experimental rearing phase (faeces collection), fish were subjected to a one week conditioning period during which they were adapted to each experimental diet and overall experimental conditions.

23.2 Dietary treatments

The dietary treatments used in the present experiments are shown in Table 23.1.

Table 23.1: Dietary treatments

|   | Code | Treatments | Inclusion Product (per kg feed) |
|---|------|-----------|-------------------------------|
| 1 | PCC | Positive control with DCP supplementation | |
| 2 | NCC | Negative control - no DCP supplementation | |
| 3 | NCC 500 | NCC + 500 FTU phytase/kg | 0.05 g BP17 phytase/kg |
| 4 | NCC 750 | NCC + 750 FTU phytase/kg | 0.075 g BP17 phytase /kg |
| 5 | NCC 1000 | NCC + 1000 FTU phytase/kg | 0.1 g BP17 phytase /kg |
| 6 | NCC 2000 | NCC + 2000 FTU phytase/kg | 0.2 g BP17 phytase /kg |

23.3 Diet preparation

All ingredients were finely ground, mixed and extruded (3 mm) by means of pilot-scale twinscrew extruder CLEXTRAL BC45 with a screw diameter of 55.5 mm and temperature ranging 111-116°C. Upon extrusion, all batches of extruded feeds were dried in a convection oven (LTE OP 750-UF) for 4 hours at 45°C. Following drying, pellets were allowed to cool at room temperature, and subsequently phytase was top-dressed onto the post-extruded pellets prior to oil coating under vacuum in a DINNISEN Pegasus vacuum mixer (PG-10VCLAB). Throughout the duration of the trial, the experimental feeds were stored at 4°C.

Table 23.2: Nile Tilapia feed formulation and Nutrient composition

| Ingredients (%) | PCT | NCT | NCT500 | NCT600 | NCT1000 | NCT1500 |
|-----------------|-----|-----|--------|--------|---------|---------|
| Soybean meal 48 | 24.0 | 24.0 | 24.0 | 24.0 | 24.0 | 24.0 |
| Soybean meal 44 | 15.0 | 15.0 | 15.0 | 15.0 | 15.0 | 15.0 |
| Full-fat soybean meal | 5.0 | 5.0 | 5.0 | 5.0 | 5.0 | 5.0 |
| Whole wheat | 3.7 | 5.9 | 5.9 | 5.9 | 5.9 | 5.9 |
| Wheat bran | 20.0 | 20.0 | 20.0 | 20.0 | 20.0 | 20.0 |
| Rice bran full fat | 23.0 | 23.0 | 23.0 | 23.0 | 23.0 | 23.0 |
| Rapeseed oil | 3.0 | 3.0 | 3.0 | 3.0 | 3.0 | 3.0 |
| Vit & Min Premix | 1.0 | 1.0 | 1.0 | 1.0 | 1.0 | 1.0 |
| Binder (guar gum) | 0.5 | 0.5 | 0.5 | 0.5 | 0.5 | 0.5 |
| Dicalcium phosphate | 2.2 | 0.0 | 0.0 | 0.0 | 0.0 | 0.0 |
| L-Lysine | 0.2 | 0.2 | 0.2 | 0.2 | 0.2 | 0.2 |

(continued)

| Ingredients (%) | PCT | NCT | NCT500 | NCT600 | NCT1000 | NCT1500 |
|---|---|---|---|---|---|---|
| DL-Methionine | 1.4 | 1.4 | 1.4 | 1.4 | 1.4 | 1.4 |
| Chromic oxide | 1.0 | 1.0 | 1.0 | 1.0 | 1.0 | 1.0 |
| BP17 phytase (U/kg) | | | 500.0 | 750.0 | 1000.0 | 2000.0 |
| **Composition** | | | | | | |
| Dry matter (DM), % | 93.50 | 92.81 | 93.50 | 93.40 | 92.59 | 92.28 |
| Crude protein, % DM | 30.46 | 30.16 | 30.37 | 30.24 | 30.24 | 30.46 |
| Crude lipid, % DM | 8.38 | 8.34 | 8.41 | 8.28 | 8.35 | 8.33 |
| Ash, % DM | 10.23 | 8.33 | 8.51 | 8.20 | 8.26 | 8.36 |
| Total phosphorus, % DM | 1.39 | 0.93 | 0.93 | 0.94 | 0.93 | 1.00 |
| Gross energy, kJ/g DM | 19.17 | 19.42 | 19.38 | 19.31 | 19.28 | 19.32 |
| Chromic oxide, % DM | 1.01 | 1.01 | 0.99 | 0.99 | 1.01 | 0.99 |
| Phytase analytics (U/kg) | 151 | 212 | 488 | 590 | 1092 | 1521 |

The trial comprised 6 experimental fishmeal-free diets (Tables 23.2 and 23.8). A positive control diet (PCT) was formulated with practical ingredients to contain 32.5% DM crude protein, 8.4% DM crude fat and 19.2 MJ/kg DM gross energy. This diet contained dicalcium-phosphate in order to attain a total phosphorus level (1.4% DM) sufficient to cover the requirement of the species. A negative control diet (NCT) was not supplemented with dicalcium phosphate and formulated to contain a total P level of 0.9%, but in which about 0.6% is found in the form of phytate-bound P. This diet had therefore a putative phosphorus deficiency. Four other diets were based on the NCT formulation but supplemented with graded doses (500, 750, 1000 and 2000 U/kg feed) of test phytase (diets NCT500, NCT600, NCT1000 and NCT1500). Diets were supplemented with crystalline amino acids (Lys and Met) to cover the nutritional requirements of the species. Diets were isonitrogenous, isolipidic and isoenergetic. Chromic oxide ($Cr_2O_3$) was incorporated at 1% in all diets, as an inert marker for apparent digestibility measurements.

Compared with National Research Council of the National Academies values (Nutrient requirements of Fish and Shrimp, NRC 2010), nutrient formulation was above NRC requirements.

The requirements for protein, energy, amino acids, fatty acids, vitamins, and minerals were determined with diets containing purified and chemically defined ingredients that are highly digestible to fish; therefore, the values in the table represent near 100 percent bioavailability to the fish. This fact should be considered when formulating diets from natural feedstuffs in which the bioavailability of the nutrients is markedly less than that in the laboratory diets.

23.4 Administration of test product and duration of treatment

Throughout the trial, fish were fed once a day in slight excess. For each experimental replicate, total feeding duration varied in between 12-15 days, depending on the amount of faeces required for analytical purposes. As mentioned before each experimental replicate was tested on three separate runs. At each run, prior to the initiation of faeces collection fish were adapted to the experimental diets for 8 days.

23.5 Enzyme analysis

The enzyme recoveries were acceptable for doses at 500 and 1,000 FTU/kg but a little bit low for 750 and 2,000 FTU/kg.

Table 23.3: Enzyme Guaranteed minimum activity in product (10,000 FTU/g)

| Gp No | Dose (kg/t) | Treatment* | Result (FTU/kg) | Expected Result (FTU/kg)** | Result (%) |
|---|---|---|---|---|---|
| 1 | 0 | PCC | 151 | <100 | - |
| 2 | 0 | NCC | 212 | <100 | - |
| 3 | 0.05 | NCC 500 | 488 | 500 | 97.6 |

(continued)

| Gp No | Dose (kg/t) | Treatment* | Result (FTU/kg) | Expected Result (FTU/kg)** | Result (%) |
|---|---|---|---|---|---|
| 4 | 0.075 | NCC 750 | 590 | 750 | 78.6 |
| 5 | 0.1 | NCC 1000 | 1092 | 1000 | >100 |
| 6 | 0.2 | NCC 2000 | 1521 | 2000 | 76.0 |
| * All analyses performed at Danisco Enzyme Assay Laboratory, Brabrand, Denmark. ** Based on product guaranteed minimum activity | | | | | |

23.6 Methods of analysis

23.6.1. Proximate composition of diets and faeces

Diets and freeze-dried faeces were homogenized with a laboratory mill prior to analysis. The chemical composition analysis of diets and faeces was made using the following procedures: dry matter after drying at 105°C for 24 h; ash by combustion at 550°C for 12 h; crude protein (N×6.25) by a flash combustion technique followed by a gas chromatographic separation and thermal conductivity detection (LECO FP428); fat by dichloromethane extraction (Soxhlet); gross energy in an adiabatic bomb calorimeter (IKA). Analysis of total phosphorus was done according to the ISO/DIS 6491 method using the vanado-molybdate reagent. Chromic oxide in the diets and faeces was determined according to Bolin, D. W., R. P. King and E. W. Klosterman. 1952 (A simplified method for the determination of chromic oxide (Cr:O~) when used as an index substance. Science 116:634, after perchloric acid digestion).

23.6.2. Amino acid composition of diets and faeces

Amino acids profile of diets and faeces was obtained after hydrolysis in 6M HCL at 108°C over 24h in nitrogen-flushed glass vials. A Waters Pico-Tag reversed-phase HPLC system, using norleucine as an internal standard, was used. The resulting chromatograms were analysed with Breeze software (Waters, USA). Tryptophan was not analysed since it is destroyed by acid hydrolysis.

23.7 Observations during the study

23.7.1 Environmental factors

Environmental rearing parameters observed throughout the experimental feeding period are reported in Figure 20. The trial was conducted at indoor facilities, supplied with recirculated thermo-regulated freshwater. A light:dark photoperiod cycle of 14:10 h was adopted. Water temperature remained within the established limits ($27.8 \pm 0.2$°C). Dissolved oxygen levels were maintained above 6.5 mg/L. Moreover, ammonia levels were low throughout the trial.

23.7.2 Assessment criteria

Apparent digestibility coefficients (ADC) of the dietary nutrients and energy were calculated according to the formula:

$$ADC(\%) = 100 - \left[ \frac{\% \text{ dietary } Y_2O_3 \text{ level}}{\% \text{ feacal } Y_2O_3 \text{ level}} \times \frac{\% \text{ faecal nutrient or energy level}}{\% \text{ dietary nutrient or energy level}} \right]$$

23.7 Analysis of data

Data are presented as mean of triplicates $\pm$ standard deviation. Data were subjected to a one-way analysis of variance, and when appropriate, means were compared by the Newman-Keuls test. Parameters expressed as percentages were subjected to arcsin square root transformation. Statistical significance was tested at 0.05 probability level.

Results

23.8 Nutrient digestibility

In *Nile Tilapia*, supplementation of phytase (500 to 2,000 FTU/kg) in NC groups numerically improved the digestibility of protein, lipid and energy compared with NC.

PC levels were restored and even higher for CP, lipid and energy (Table 23.3).

In the phytase trial groups, it was significantly higher than the digestibility of NC group with all phytase doses higher than 750 FTU/kg (real = 590 FTU/kg).

PC levels were statistically restored and even higher for phytase used at doses up to 750 FTU/kg (real = 590 FTU/kg).

About P digestibility, the dose response when increasing phytase doses were used was clear.

Optimal dose seemed to be between 750 and 1,000 FTU/kg. However, given that the targeted phytase dose of 750 U/kg feed was not correctly achieved, doubts exists on whether such beneficial effects could also be found at a dose between 590 and 1000 U/kg.

Table 23.4: Nutrient digestibility of Nile Tilapia fed graded levels of Phytase over the whole study

|  | PC | | NC | | NC + 500 FTU/kg | | NC + 750 FTU/kg | | NC+ 1000 FTU/kg | | NC+ 2000 FTU/kg | |
|---|---|---|---|---|---|---|---|---|---|---|---|---|
|  | mean | SD | mean | SD | mean | SD | mean | SD | mean | SD | mean | SD |
| Dry matter (%) | 68.0 | 2.7 | 68.2 | 0.2 | 68.1 | 1.7 | 68.5 | 0.7 | 66.5 | 1.2 | 67.3 | 1.9 |
| Protein (%) | 82.6 | 2.0 | 83.3 | 1.1 | 84.6 | 2.0 | 84.2 | 1.0 | 82.8 | 2.5 | 84.1 | 2.8 |
| Lipid (%) | 89.6 | 1.5 | 90.6 | 0.8 | 91.7 | 1.3 | 91.9 | 0.1 | 91.4 | 0.5 | 90.5 | 1.3 |
| Energy (%) | 71.4 | 2.2 | 71.6 | 0.6 | 73.3 | 1.6 | 72.2 | 1.7 | 70.0 | 1.4 | 70.4 | 1.7 |
|  | | | | | | | | | | | | |
| P (%) | 42.8 b | 4.8 | 30.7 a | 3.5 | 29.5 a | 4.5 | 31.6 a | 2.4 | 51.8 c | 6.6 | 56.9 c | 1.4 |
| ANOVA P<0.05 and a>b>c | | | | | | | | | | | | |

Table 23.5: Nutrient digestibility of Nile Tilapia: % Improvement over positive control

|  | NC | NC + 500 FTU/kg | NC + 750 FTU/kg | NC+ 1000 FTU/kg | NC+ 2000 FTU/kg |
|---|---|---|---|---|---|
| Dry matter (%) | 0.29 | +0.15 | +0.74 | -2.21 | -1.03 |
| Protein (%) | 0.85 | +2.42 | +1.94 | +0.24 | +1.82 |
| Lipid (%) | 1.12 | +2.34 | +2.57 | +2.01 | +1.00 |
| Energy (%) | 0.28 | +2.66 | +1.12 | -1.96 | -1.40 |
| P (%) | -28.27 | -31.07 | -26.17 | +21.03 | +32.94 |
| Id = identical to positive control | | | | | |

23.9. Assessment of Nutrients release values

The phytase supplementation (especially doses between 500 and 750 FTU/kg) tended to improve global nutrients release.

However, except the response observed with P, none of the nutrient releases curves gave clear linear and consistent response (Table 23.6).

About Phosphorus, phytase supplementation clearly and linearly improves P release.

Table 23.6: Nutrients release values of the dietary treatments with graded levels of phytase supplementation fed to Nile Tilapia

| | NC + 500 | NC + 750 | NC + 1000 | NC + 2000 |
|---|---|---|---|---|
| DE release (cal/kg, as fed) | 87 | 27 | -103 | -92 |
| Lipid release (%, as fed) | 0,68 | 0,46 | -0,16 | 0,29 |
| CP release (%, as fed) | 0,14 | 0,17 | -0,02 | -0,06 |
| EAA release (%, as fed) | 0,20 | 0,10 | 0,05 | -0,06 |
| | | | | |
| P release (%, as fed) | -0,01 | 0,01 | 0,18 | 0,25 |

23.10 Reduction of Phosphorous release in water

The decrease on phosphorus content of faeces affected by dietary phytase supplementation levels is presented in Table 23.7.

Table 23.7: Fecal phosphorus (P) levels in tilapia juveniles fed the experimental diets

| Phytase dose (FTU/kg) | 0 | 500 | 750 | 1000 | 2000 |
|---|---|---|---|---|---|
| Faecal P | 2,02 | 2,06 | 2,03 | 1,34 | 1,32 |
| SD | 0,09 | 0,02 | 0,05 | 0,17 | 0,12 |

1,000 U/kg of Phytase potentially decreases P release in water by 0.6%

Discussion

In conclusion, use of BP17 phytase show good results in fish apparent nutrient digestibility and reduction of phosphorous release in water.

Supplementation of BP17 phytase (up to 750 FTU/kg) significantly improves the digestibility of Phosphorous. The digestibility of P in the phytase trial groups is significantly higher than the digestibility of NC group with all phytase doses higher than 500 FTU/kg.

PC levels are statistically restored for phytase used at doses up to 750 FTU/kg but optimal dose seems to be 1,000 FTU/kg.

Total DCP in the diet can be reduced by at least 2.0%
Phosphorous release in the water can be reduced by 0.6%

**Example 24 - comparative Example**

The pH of the acidic portion of the digestive tract of poultry (Gizzard/proventriculus) has been reported to range from 3.3 to 3.5 with the pH in the stomach of pigs being 2.5 to 4.5 and in fish pH 3.3 to 3.8. Due to the low pH in the Gizzard /Proventriculus area of poultry and the acid stomach of other species, this is also the main region where interactions of phytate with proteins are expected to occur, possibly resulting in inefficient protein digestion (as in 002). Consequently, it has been thought that the pH optimum of phytase enzymes is important, and that phytase enzymes that have a pH optimum closer to the gastric portion of the digestive tract can be expected to exert greater beneficial effects on the animals biophysical characteristics than phytases that have higher pH optima.

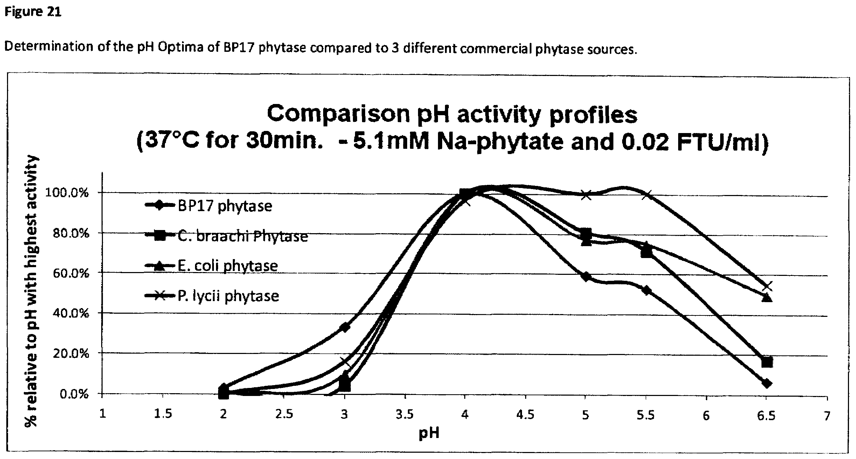

The pH optimum of 3 different commercial sources of phytase and BP17 phytase is shown in Figure 21. Here it can be seen that the pH optimum of BP17 phytase of pH 4.0 is similar to the pH optimum of *Escherichia coli* phytase and *Citrobacter braachi* phytase, while the *Peniophora lycii* phytase also reaches the greatest activity at ~4.0, but has a broader optimum pH range of 4.0 to 5.5.

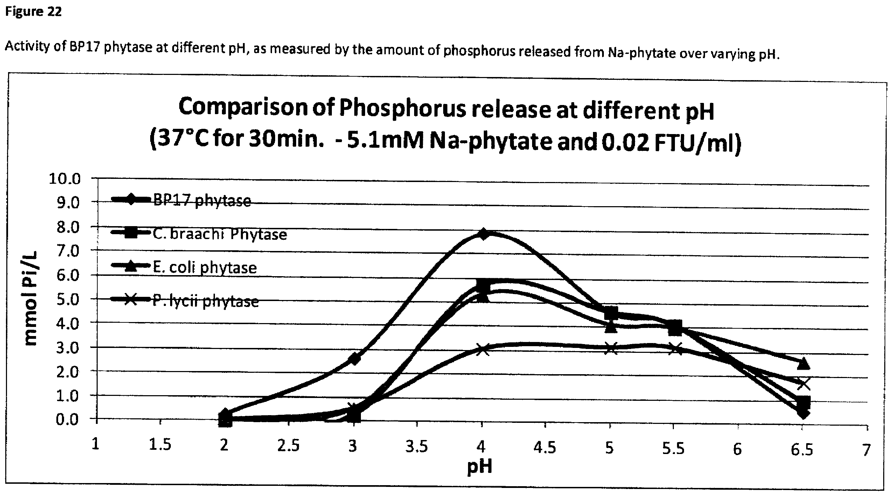

However, what was surprising was that the relative activity of the BP17 phytase at pH 4.0 as measured by the amount of Phosphorus release *in-vitro* under standard conditions, was 37%, 48%, and 156% greater than that of *Citrobacter* phytase, *E.coli* phytase, and *P. lycii* phytase, respectively (see Figure 22).

Phytase inclusion in feed is standardized based on including a defined amount of phytase units (FTU/kg feed).

Typically this would be 500 units/kg phytase / kg feed, or 1000 units/kg feed, but can also be in the range of 250 FTU/kg to as high as 5000, or 10,000 units/kg feed. Importantly, the inclusion in feed is standardized based on the relative activity of the phytase at pH 5.5.

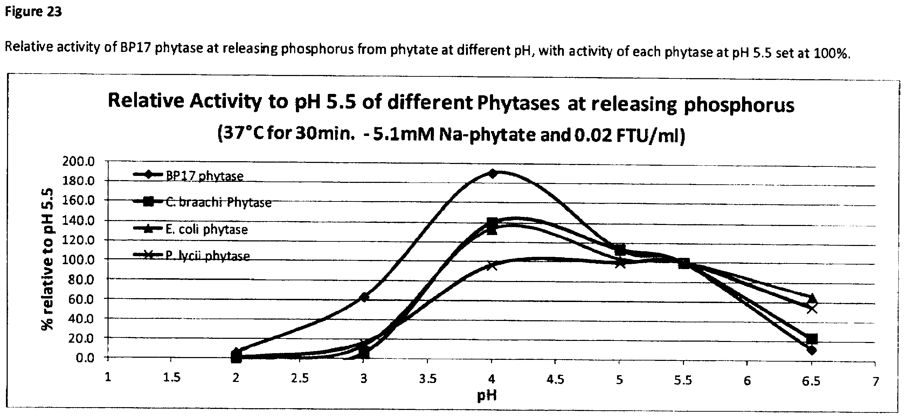

As phytase inclusion in feed is based on the phytase activity at pH 5.5, the relative activity of phytase at lower pH compared to pH 5.5 is important. This is shown in Figure 23.

It was further surprising that the phytase activity of BP17 phytase expressed as a percentage of activity at pH 5.5, was 10.8, 4.9, and 3.9 times greater at pH 3.0 and 1.36:1, 1.42:1, and 1.97 times greater at pH 4.0 compared to *Citrobacter*, *E.coli*, and *P. lycii* phytases.

## SUMMARY PARAGRAPHS

**[0424]** Further described is:

1. A method of feeding an animal with a feed, wherein said feed comprises a phytase, wherein said phytase results in an improvement in one or more of said animal's biophysical characteristics when compared to the equivalent use of *Peniophora lycii* phytase and/or an *E. coli phytase.*

2. A method according to paragraph 1 wherein said phytase results in an improvement in said animal's biophysical characteristics as a food source.

3. A method according to paragraph 1 or paragraph 2 wherein the improvement in said animal's biophysical characteristics comprises an increase in weight gain.

4. A method according to paragraph 3 wherein the improvement in said animal's biophysical characteristics comprises an increase in weight gain of at least 2% over a period of at least 21 days when the phytase is dosed at an amount of at least 500 FTU/kg feed.

5. A method according to any preceding paragraph wherein the improvement in said animal's biophysical characteristics comprises an increase in feed conversion ratio.

6. A method according to paragraph 5 wherein the improvement in said animal's biophysical characteristics comprises an increase in feed conversion ratio of at least 5% over a period of at least 21 days when the phytase is dosed at an amount of at least 500 FTU/kg feed.

7. A method according to any preceding paragraph wherein the improvement in said animal's biophysical characteristics comprises an increase in bone density and/or bone strength and/or calcium deposition and/or Phosphorus deposition.

8. A method according to paragraph 7 wherein the improvement in said animal's biophysical characteristics comprises an increase in bone density and/or bone strength and/or calcium deposition and/or Phosphorus deposition of at least 10% over a period of at least 21 days when the phytase is dosed at an amount of at least 250 FTU/kg feed.

9. A method according to any preceding paragraph wherein the improvement in said animal's biophysical characteristics comprises an increase in the retention of a mineral and/or a decrease in secretion of a mineral.

10. A method according to paragraph 9 wherein the improvement in said animal's biophysical characteristics comprises an increase in the retention of a mineral and/or a decrease in secretion of a mineral of at least 10% over a period of at least 14 days when the phytase is dosed at an amount of at least 250 FTU/kg feed.

11. A method according to any preceding paragraph wherein the improvement in said animal's biophysical characteristics comprises an increase in retention and/or a decrease in secretion of any one or more of copper, sodium, phosphorous, nitrogen and calcium.

12. A method according to paragraph 11 wherein the improvement in said animal's biophysical characteristics comprises an increase in retention and/or a decrease in secretion of any one or more of copper, sodium, phosphorous, nitrogen and calcium of at least 10% over a period of at least four weeks when the phytase is dosed at an amount of at least 250 FTU/kg feed.

13. A method according to any preceding paragraph wherein the improvement in said animal's biophysical characteristics comprises an increase in amino acid retention.

14. A method according to paragraph 13 wherein the improvement in said animal's biophysical characteristics comprises an increase in amino acid retention of on average at least 10% over a period of at least 21 days when the phytase is dosed at an amount of at least 500 FTU/kg feed.

15. A method according to any preceding paragraph wherein the improvement in said animal's biophysical characteristics comprises an increase in mineralisation.

16. A method according to paragraph 15 wherein the improvement in said animal's biophysical characteristics comprises an increase in mineralisation of at least 10% over a period of at least 14 days when the phytase is dosed at an amount of at least 250 FTU/kg feed.

17. A method according to any preceding paragraph wherein the improvement in said animal's biophysical characteristics comprises an increase in growth.

18. A method according to paragraph 17 wherein the improvement in said animal's biophysical characteristics comprises an increase in growth of at least 20% over a period of at least 28 days when the phytase is dosed at an amount of at least 250 FTU/kg feed.

19. A method according to any preceding paragraph wherein the improvement in said animal's biophysical characteristics comprises an increase in egg laying rate and/or egg weight and/or egg mass.

20. A method according to paragraph 19 wherein the improvement in said animal's biophysical characteristics comprises an increase in egg laying rate of around at least 2% and/or egg weight of around at least 2% and/or egg mass of around at least 4% over a period of at least 23 weeks when the phytase is dosed at an amount of at least 250 FTU/kg feed.

21. A method of any preceding paragraph wherein said animal is a monogastric farm animal

22. A method of any preceding paragraph wherein said animal is a monogastric animal.

23. A method of any preceding paragraph wherein said animal is a bird or poultry.

24. A method of any preceding paragraph wherein said animal is a chicken or duck.

25. A method of any preceding paragraph wherein said animal is a turkey.

26. A method of any of paragraphs 1 to 22 wherein said animal is a pig, piglet, swine, hog, grower-finisher or sow

27. A method of any of paragraphs 1-20 wherein said animal is a non mono-gastric animal or ruminant animal.

28. A method of any of paragraphs 1-20 or 27 wherein said animal is a non-monogastric farm animal.

29. A method of any of paragraphs 1-20 or 27-28 wherein said animal is a beef producing animal.

30. A method of any of paragraphs 1-20 or 27-29 wherein said animal is a dairy producing animal.

31. A method of any of paragraphs 1-20 or 27-30 wherein said animal is an Alpaca, Bison, Bovine, Camel, Cattle, Cow, Deer, Donkey, Equine, Equus, Goat, Horse, Lamb, Livestock, Llama, Mule, Ox, Reindeer, Sheep, Steer, Yak, Buffalo, Giraffe, Moose, Elk, Llama, Antelope, Pronghorn, or Nilgai or beef or dairy producing animal, or any ruminant, equine, bovine, cervidae, caprinae or camelidae animal.

32. A method of any preceding paragraph wherein said animal is a domesticated animal.

33. A method of any of paragraphs 1-20 or 32 wherein said animal is a fish.

34. A method of any of paragraphs 1-20 or 32-33 wherein said animal is a gastric fish.

35. A method of any of paragraphs 1-20 or 32-34 wherein said animal is an agastric fish.

36. A method of any of paragraphs 1-20 or 32-35 wherein said animal is a shrimp or other crustacean.

37. A method of any of paragraphs 1-20 or 32-36 wherein said animal is a marine fish or freshwater fish.

38. A method according to any preceding paragraph wherein said feed is in pellet, granule, meal, mash, liquid, wet capsule or spray form.

39. A method according to any preceding paragraph wherein said phytase is selected from a naturally occurring phytase, a non-naturally occurring phytase or variant thereof.

40. A method according to any preceding paragraph wherein said phytase has been prior isolated from a source.

41. A method according to any preceding paragraph wherein said phytase has been prepared by use of recombinant DNA techniques.

42. A method according to any preceding paragraph wherein said phytase is or is obtainable from or is derivable from a bacterial origin.

43. A method according to any preceding paragraph wherein said phytase is or is obtainable from or is derivable from a *Buttiaxuella* species.

44. A method according to any preceding paragraph wherein said phytase has at least 75% identity to BP17 as shown in SEQ ID NO:1.

45. A method according to any preceding paragraph wherein said phytase has at least 85% identity to BP17 as shown in SEQ ID NO:1.

46. A method according to any of paragraphs 1 to 45 wherein said phytase is BP17 as shown in SEQ ID NO:1.

47. A method according to any one of paragraphs 1 to 42 wherein said phytase is or is obtainable from or is derivable from a *Citrobacter* species.

48. A method according to any preceding paragraph in which said phytase is low pH tolerant.

49. Use of BP17 phytase of SEQ ID NO:1 in a method of feeding an animal with a feed, wherein said BP17 phytase results in an improvement in one or more of said animal's biophysical characteristics when compared to the equivalent use of *Peniophora lycii* phytase.

50. A use according to paragraph 49 wherein said phytase results in an improvement in said animal's biophysical characteristics as a food source.

51. A use according to any of paragraphs 49-50 wherein the improvement in said animal's biophysical characteristics comprises an increase in weight gain.

52. A use according to paragraph 51 wherein the improvement in said animal's biophysical characteristics comprises an increase in weight gain of at least 2% over a period of at least 21 days when the phytase is dosed at an amount of at least 500 FTU/kg feed.

53. A use according to any of paragraphs 49-52 wherein the improvement in said animal's biophysical characteristics comprises an increase in feed conversion ratio.

54. A use according to paragraph 53 wherein the improvement in said animal's biophysical characteristics comprises an increase in feed conversion ratio of at least 5% over a period of at least 21 days when the phytase is dosed at an amount of at least 500 FTU/kg feed.

55. A use according to any of paragraphs 49-54 wherein the improvement in said animal's biophysical characteristics comprises an increase in bone density and/or bone strength and/or calcium deposition and/or Phosphorus deposition.

56. A use according to paragraph 55 wherein the improvement in said animal's biophysical characteristics comprises an increase in bone density and/or bone strength and/or calcium deposition and/or Phosphorus deposition of at least 10% over a period of at least 21 days when the phytase is dosed at an amount of at least 250 FTU/kg feed.

57. A use according to any of paragraphs 49-56 wherein the improvement in said animal's biophysical characteristics comprises an increase in the retention of a mineral and/or a decrease in secretion of a mineral.

58. A use according to paragraph 57 wherein the improvement in said animal's biophysical characteristics comprises an increase in the retention of a mineral and/or a decrease in secretion of a mineral of at least 10% over a period of at least 14 days when the phytase is dosed at an amount of at least 250 FTU/kg feed.

59. A use according to any of paragraphs 49-58 wherein the improvement in said animal's biophysical characteristics comprises an increase in retention and/or a decrease in secretion of any one or more of copper, sodium, phosphorous, nitrogen and calcium.

60. A use according to paragraph 59 wherein the improvement in said animal's biophysical characteristics comprises an increase in retention and/or a decrease in secretion of any one or more of copper, sodium, phosphorous, nitrogen and calcium of at least 10% over a period of at least six weeks when the phytase is dosed at an amount of at least 250 FTU/kg feed.

61. A use according to any of paragraphs 49-60 wherein the improvement in said animal's biophysical characteristics comprises an increase in amino acid retention.

62. A use according to paragraph 61 wherein the improvement in said animal's biophysical characteristics comprises an increase in amino acid retention of on average at least 10% over a period of at least 21 days when the phytase is dosed at an amount of at least 500 FTU/kg feed.

63. A use according to any of paragraphs 49-62 wherein the improvement in said animal's biophysical characteristics comprises an increase in mineralisation.

64. A use according to paragraph 63 wherein the improvement in said animal's biophysical characteristics comprises an increase in mineralisation of at least 10% over a period of at least 14 days when the phytase is dosed at an amount of at least 250 FTU/kg feed.

65. A use according to any of paragraphs 49-64 wherein the improvement in said animal's biophysical characteristics comprises an increase in growth.

66. A use according to paragraph 65 wherein the improvement in said animal's biophysical characteristics comprises an increase in growth of at least 20% over a period of at least 28 days when the phytase is dosed at an amount of at least 250 FTU/kg feed.

67. A use according to any of paragraphs 49-66 wherein the improvement in said animal's biophysical characteristics comprises an increase in egg laying rate and/or egg weight and/or egg mass.

68. A use according to paragraph 67 wherein the improvement in said animal's biophysical characteristics comprises an increase in egg laying rate of around at least 2% and/or egg weight of around at least 2% and/or egg mass of around 4% over a period of at least 23 weeks when the phytase is dosed at an amount of at least 250 FTU/kg feed.

69. A use according to any of paragraphs 49-68 wherein said animal is a mono-gastric farm animal.

70. A use according to any of paragraphs 49-69 wherein said animal is a monogastric animal.

71. A use according to any of paragraphs 49-70 wherein said animal is a bird or poultry.

72. A use according to any of paragraphs 49-71 wherein said animal is a chicken or duck.

73. A use according to any of paragraphs 49-71 wherein said animal is a turkey.

74. A use according to any of paragraphs 49-70 wherein said animal is a pig, piglet, swine, hog, grower finisher or sow.

75. A use of any of paragraphs 49-68 wherein said animal is a non mono-gastric animal or ruminant animal.

76. A use of any of paragraphs 49-68 or 75 wherein said animal is a non-monogastric farm animal.

77. A use of any of paragraphs 49-68 or 75-76 wherein said animal is a beef producing animal.

78. A use of any of paragraphs 49-68 or 75-77 wherein said animal is a dairy producing animal.

79. A use of any of paragraphs 49-68 or 75-78 wherein said animal is an Alpaca, Bison, Bovine, Camel, Cattle, Cow, Deer, Donkey, Equine, Equus, Goat, Horse, Lamb, Livestock, Llama, Mule, Ox, Reindeer, Sheep, Steer, Yak, Buffalo, Giraffe, Moose, Elk, Llama, Antelope, Pronghorn, or Nilgai or beef or dairy producing animal, or any ruminant, equine, bovine, cervidae, caprinae or camelidae animal.

80. A use of any of paragraphs 49-79 wherein said animal is a domesticated animal.

81. A use of any of paragraphs 49-68 or 80 wherein said animal is a fish.

82. A use of any of paragraphs 49-68 or 80-81 wherein said animal is a gastric fish.

83. A use of any of paragraphs 49-68 or 80-81 wherein said animal is an agastric fish.

84. A use of any of paragraphs 49-68 or 80-83 wherein said animal is a shrimp or other crustacean.

85. A use of any of paragraphs 49-68 or 80-84 wherein said animal is a marine fish or freshwater fish.

86. A use according to any of paragraphs 49-85 wherein said feed is in pellet, granule, meal, mash, liquid, wet, capsule or spray form.

87. A method for producing a feed for use in the methods of any of paragraphs 1-48 involving the step of adding a phytase to an animal feed.

88. A method according to paragraph 87 wherein said animal is a mono-gastric farm animal.

89. A method according to paragraph 87 or 88 wherein said animal is a monogastric animal.

90. A method according to any of paragraphs 87-89 wherein said animal is a bird or poultry.

91. A method according to any of paragraphs 87-90 wherein said animal is a chicken or a duck.

92. A method according to any of paragraphs 87-90 wherein said animal is a turkey.

93. A method according to any of paragraphs 87-89wherein said animal is a pig, piglet, swine, hog, grower finisher or sow.

94. A method of paragraphs 87 wherein said animal is a non mono-gastric animal or ruminant animal.

95. A method of any of paragraphs 87 or 94 wherein said animal is a non-monogastric farm animal.

96. A method of any of paragraphs 87 or 94-95 wherein said animal is a beef producing animal.

97. A method of any of paragraphs 87 or 94-96 wherein said animal is a dairy producing animal.

98. A method of any of paragraphs 87 or 94-97 wherein said animal is an Alpaca, Bison, Bovine, Camel, Cattle, Cow, Deer, Donkey, Equine, Equus, Goat, Horse, Lamb, Livestock, Llama, Mule, Ox, Reindeer, Sheep, Steer, Yak, Buffalo, Giraffe, Moose, Elk, Llama, Antelope, Pronghorn, or Nilgai or beef or dairy producing animal, or any ruminant, equine, bovine, cervidae, caprinae or camelidae animal.

99. A method of any of paragraphs 87-98 wherein said animal is a domesticated animal.

100. A method of any of paragraphs 87 or 99 wherein said animal is a fish.

101. A method of any of paragraphs 87 or 99-100 wherein said animal is a gastric fish.

102. A method of any of paragraphs 87 or 99-100 wherein said animal is an agastric fish.

103. A method of any of paragraphs 87 or 99-102 wherein said animal is a shrimp or other crustacean.

104. A method of any of paragraphs 87 or 99-103 wherein said animal is a marine fish or freshwater fish.

105. A method according to any of paragraphs 87-104 wherein said feed is in pellet, granule, meal, mash, liquid, wet, capsule or spray form.

106. A method according to any of paragraphs 87-105wherein said phytase is selected from a naturally occurring phytase, a non-naturally occurring phytase or variant thereof.

107. A method according to any of paragraphs 87-106wherein said phytase has been prior isolated from a source.

108. A method according to of paragraphs 87-107wherein said phytase has been prepared by use of recombinant DNA techniques.

109. A method according to any of paragraphs 87-108 wherein said phytase is or is obtainable from or is derivable from a bacterial origin.

110. A method according to any of paragraphs 87-109 wherein said phytase is or is obtainable from or is derivable from a *Buttiaxuella* species.

111. A method according to any of paragraphs 87-109 wherein said phytase has at least 75% identity to BP17 as shown in SEQ ID NO:1.

112. A method according to any of paragraphs 87-110 wherein said phytase has at least 85% identity to BP17 as shown in SEQ ID NO:1.

113. A method according to any of paragraphs 87-110 wherein said phytase is BP17 as shown in SEQ ID NO:1.

114. A method according to any of paragraphs 87-110 wherein said phytase is or is obtainable from or is derivable from a *Citrobacter* species.

115. A method according to any of paragraphs 87-114 in which said phytase is low pH tolerant.

116. A method of feeding an animal with a feed, wherein said feed comprises a phytase, wherein said phytase results in an improvement in one or more of said animal's biophysical characteristics; wherein said improvement in said animal's biophysical characteristics comprises an increase in weight gain.

117. A method according to paragraph 116 wherein the improvement in said animal's biophysical characteristics comprises an increase in weight gain of at least 2% over a period of at least 21 days when the phytase is dosed at an amount of at least 500 FTU/kg feed.

118. A method of feeding an animal with a feed, wherein said feed comprises a phytase, wherein said phytase results in an improvement in one or more of said animal's biophysical characteristics; wherein said improvement in said animal's biophysical characteristics comprises an increase in feed conversion ratio.

119. A method according to paragraph 118 wherein the improvement in said animal's biophysical characteristics comprises an increase in feed conversion ratio of at least 5% over a period of at least 21 days when the phytase is dosed at an amount of at least 500 FTU/kg feed.

120. A method of feeding an animal with a feed, wherein said feed comprises a phytase, wherein said phytase results in an improvement in one or more of said animal's biophysical characteristics; wherein said improvement in said animal's biophysical characteristics comprises an increase in bone density and/or bone strength and/or calcium deposition and/or Phosphorus deposition.

121. A method according to paragraph 120 wherein the improvement in said animal's biophysical characteristics comprises an increase in bone density and/or bone strength and/or calcium deposition and/or Phosphorus deposition of at least 10% over a period of at least 21 days when the phytase is dosed at an amount of at least 250 FTU/kg feed.

122. A method of feeding an animal with a feed, wherein said feed comprises a phytase, wherein said phytase results in an improvement in one or more of said animal's biophysical characteristics; wherein said improvement in said animal's biophysical characteristics comprises an increase in the retention of a mineral and/or a decrease in secretion of a mineral.

123. A method according to paragraph 122 wherein the improvement in said animal's biophysical characteristics comprises an increase in the retention of a mineral and/or a decrease in secretion of a mineral of at least 10% over a period of at least 14 days when the phytase is dosed at an amount of at least 250 FTU/kg feed.

124. A method of feeding an animal with a feed, wherein said feed comprises a phytase, wherein said phytase results in an improvement in one or more of said animal's biophysical characteristics; wherein said improvement in said animal's biophysical characteristics comprises an increase in retention and/or a decrease in secretion of any one or more of copper, sodium, phosphorous, nitrogen and calcium.

125. A method according to paragraph 124 wherein the improvement in said animal's biophysical characteristics comprises an increase in retention and/or a decrease in secretion of any one or more of copper, sodium, phosphorous, nitrogen and calcium of at least 10% over a period of at least four weeks when the phytase is dosed at an amount of at least 250 FTU/kg feed.

126. A method of feeding an animal with a feed, wherein said feed comprises a phytase, wherein said phytase results in an improvement in one or more of said animal's biophysical characteristics; wherein said improvement in said animal's biophysical characteristics comprises an increase in amino acid retention.

127. A method according to paragraph 126 wherein the improvement in said animal's biophysical characteristics comprises an increase in amino acid retention of on average at least 10% over a period of at least 21 days when the phytase is dosed at an amount of at least 500 FTU/kg feed.

128. A method of feeding an animal with a feed, wherein said feed comprises a phytase, wherein said phytase results in an improvement in one or more of said animal's biophysical characteristics; wherein said improvement in said animal's biophysical characteristics comprises an increase in mineralisation.

129. A method according to paragraph 128 wherein the improvement in said animal's biophysical characteristics comprises an increase in mineralisation of at least 10% over a period of at least 14 days when the phytase is dosed at an amount of at least 250 FTU/kg feed.

130. A method of feeding an animal with a feed, wherein said feed comprises a phytase, wherein said phytase results in an improvement in one or more of said animal's biophysical characteristics; wherein said improvement in said animal's biophysical characteristics comprises an increase in growth.

131. A method according to paragraph 130 wherein the improvement in said animal's biophysical characteristics comprises an increase in growth of at least 20% over a period of at least 28 days when the phytase is dosed at an amount of at least 250 FTU/kg feed.

132. A method according to any of paragraphs 116-131 wherein the improvement in said animal's biophysical characteristics comprises an increase in egg laying rate and/or egg weight and/or egg mass.

133. A method according to paragraph 132 wherein the improvement in said animal's biophysical characteristics comprises an increase in egg laying rate of around at least 2% and/or egg weight of around at least 2% and/or egg mass of around 4% over a period of at least 23 weeks when the phytase is dosed at an amount of at least 250 FTU/kg feed.

134. A method according to any one of paragraphs 116-133wherein said animal is a mono-gastric farm animal.

135. A method according to any one of paragraphs 116-134wherein said animal is a monogastric animal.

136. A method according to any of paragraphs 116-135paragraph wherein said animal is a bird or poultry.

137. A method according to any of paragraphs 116-136wherein said animal is a chicken or duck.

138. A method according to any of paragraphs 116-136wherein said animal is a turkey.

139. A method according to any of paragraphs 116-135wherein said animal is a pig, piglet, swine, hog, grower finisher or sow.

140. A method of paragraphs 116-133 wherein said animal is a non mono-gastric animal or ruminant animal.

141. A method of any of paragraphs 116-133 or 140 wherein said animal is a non-monogastric farm animal.

142. A method of any of paragraphs 116-133 or 140-141 wherein said animal is a beef producing animal.

143. A method of any of paragraphs 116-133 or 140-142 wherein said animal is a dairy producing animal.

144. A method of any of paragraphs 116-133 or 14-143 wherein said animal is an Alpaca, Bison, Bovine, Camel, Cattle, Cow, Deer, Donkey, Equine, Equus, Goat, Horse, Lamb, Livestock, Llama, Mule, Ox, Reindeer, Sheep, Steer, Yak, Buffalo, Giraffe, Moose, Elk, Llama, Antelope, Pronghorn, or Nilgai or beef or dairy producing animal, or any ruminant, equine, bovine, cervidae, caprinae or camelidae animal.

145. A method of any of paragraphs 116-144 wherein said animal is a domesticated animal.

146. A method of any of paragraphs 116-133 or 144 wherein said animal is a fish.

147. A method of any of paragraphs 116-133 or 145-146 wherein said animal is a gastric fish.

148. A method of any of paragraphs 116-133 or 145-146 wherein said animal is an agastric fish.

149. A method of any of paragraphs 116-133 or 145-148 wherein said animal is a shrimp or other crustacean.

150. A method of any of paragraphs 116-133 or 145-149 wherein said animal is a marine fish or freshwater fish.

151. A method according to any of paragraphs 116-150 wherein said feed is in pellet, granule, meal, mash, liquid, wet, capsule or spray form.

152. A method according to any of paragraphs 116-151 wherein said phytase is selected from a naturally occurring phytase, a non-naturally occurring phytase or variant thereof.

153. A method according to any of paragraphs 116-152 wherein said phytase has been prior isolated from a source.

154. A method according to of paragraphs 116-153 wherein said phytase has been prepared by use of recombinant DNA techniques.

155. A method according to any of paragraphs 116-154 wherein said phytase is or is obtainable from or is derivable from a bacterial origin.

156. A method according to any of paragraphs 116-155 wherein said phytase is or is obtainable from or is derivable from a *Buttiaxuella* species.

157. A method according to any of paragraphs 116-156 wherein said phytase has at least 75% identity to BP17 as shown in SEQ ID NO:1.

158. A method according to any of paragraphs 116-156 wherein said phytase has at least 85% identity to BP17 as shown in SEQ ID NO:1.

159. A method according to any of paragraphs 116-156 wherein said phytase is BP17 as shown in SEQ ID NO:1.

160. A method according to any of paragraphs 116-155wherein said phytase is or is obtainable from or is derivable from a *Citrobacter* species.

161. A method according to any of paragraphs 116-160in which said phytase is low pH tolerant.

162. A method of feeding an animal with a feed as described in the description, paragraphs or figures.

163. Use of BP17 phytase of SEQ ID NO:1 in a method of feeding an animal with a feed as described in the description, paragraphs or figures.

164. A method for producing a feed for use in a method of feeding an animal with a feed as described in the description, paragraphs or figures.

165. A method of feeding an animal with a feed, wherein said feed comprises a phytase, wherein said phytase results in an improvement in one or more of said animal's biophysical characteristics when compared to the equivalent use of *Peniophora lycii* phytase and/or an *E. coli phytase;* wherein said phytase is BP17 phytase SEQ ID NO:1.

166. A method of feeding an animal with a feed, wherein said feed comprises a phytase, wherein said phytase results in an improvement in one or more of said animal's biophysical characteristics when compared to the equivalent use of *Peniophora lycii* phytase and/or an *E. coli phytase*; wherein said phytase is BP17 phytase SEQ ID NO:1; wherein said improvement in said animal's biophysical characteristics comprises one or more of: an increase in weight gain, preferably an increase in weight gain of at least 2% over a period of at least 21 days when the phytase is dosed at an amount of at least 500 FTU/kg feed; an increase in feed conversion ratio, preferably an increase in feed conversion ratio of at least 5% over a period of at least 21 days when the phytase is dosed at an amount of at least 500 FTU/kg feed; an increase in bone density and/or bone strength and/or calcium deposition and/or Phosphorus deposition, preferably an increase in bone density and/or bone strength and/or calcium deposition and/or Phosphorus deposition of at least 10% over a period of at least 21 days when the phytase is dosed at an amount of at least 250 FTU/kg feed; an increase in the retention of a mineral and/or a decrease in secretion of a mineral, preferably an increase in the retention of a mineral and/or a decrease in secretion of a mineral of at least 10% over a period of at least 14 days when the phytase is dosed at an amount of at least 250 FTU/kg feed; an increase in retention and/or a decrease in secretion of any one or more of copper, sodium, phosphorous, nitrogen and calcium, preferably an increase in retention and/or a decrease in secretion of any one or more of copper, sodium, phosphorous, nitrogen and calcium of at least 10% over a period of at least four weeks when the phytase is dosed at an amount of at least 250 FTU/kg feed; an increase in amino acid retention, preferably an increase in amino acid retention of on average at least 10% over a period of at least 21 days when the phytase is dosed at an amount of at least 500 FTU/kg feed; an increase in mineralization, preferably an increase in mineralisation of at least 10% over a period of at least 14 days when the phytase is dosed at an amount of at least 250 FTU/kg feed; an increase in growth, preferably an increase in growth of at least 20% over a period of at least 28 days when the phytase is dosed at an amount of at least 250 FTU/kg feed; an increase in egg laying rate and/or egg weight and/or egg mass, preferably an increase in egg laying rate of around at least 2% and/or egg weight of around at least 2% and/or egg mass of around 4% over a period of at least 23 weeks when the phytase is dosed at an amount of at least 250 FTU/kg feed.

167. A method according to any of paragraphs 1-20 wherein the improvement in said animal's biophysical characteristics comprises an increase in digestion of any one or more of copper, sodium, phosphorous, nitrogen and calcium of at least 50% over a period of at least four weeks when the phytase is dosed at an amount of at least 1000FTU/kg feed.

**SEQUENCE LISTINGS**

**SEQ ID NO: 1**

```
NDTPASGYQVEKVVILSRHGVRAPTKMTQTMRDVTPNTWPEWPVKLGYITPRGEHLISLMGGFYRQKFQQQGILSQGSCPTPNSI
YVWTDVAQRTLKTGEAFLAGLAPQCGLTIHHQQNLEKADPLFHPVKAGICSMDKTQVQQAVEKEAQTPIDNLNQHYIPSLALMNT
TLNFSKSPWCQKHSADKSCDLGLSMPSKLSIKDNGNEVSLDGAIGLSSTLAEIFLLEYAQGMPQAAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPAGSVQLKIPGCNDQTAEGYCPLSTFTRVVSQSVEPGCQLQ
```

**SEQ ID NO: 2**

```
NDTPASGYQVEKVVILSRHGVRAPTKMTQTMRDVTPNTWPEWPVKLGYITPRGEHLISLMGGFYRQKFQQQGILSQGSCPTPNSI
YVWTDVDQRTLKTGEAFLAGLAPQCGLTIHHQQNLEKADPLFHPVKAGICSMDKTQVQQAVEKEAQTPIDNLNQHYIPSLALMNT
TLNFSKSPWCQKHSADKSCDLGLSMPSKLSIKDNGNEVSLDGAIGLSSTLAEIFLLEYAQGMPQAAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPAGSVQLKIPGCNDQTAEGYCPLSTFTRVVSQSVEPGCQLQ
```

**SEQ ID NO: 3**

```
NDTPASGYQVEKVVILSRHGVRAPTKMTQTMRDVTPYTWPEWPVKLGYITPRGEHLISLMGGFYRQKFQQQGILPRGSCPTPNSI
YVWTDVAQRTLKTGEAFLAGLAPQCGLTIHHQQNLEKADPLFHPVKAGICSMDKTQVQQAVEKEAQTPIDNLNQRYIPELALMNT
ILNFSKSPWCQKHSADKPCDLALSMPSKLSIKDNGNEVSLDGAIGLSSTLAEIFLLEYAQGMPQVAWGNIHSEQEWALLLKLHNV
YFDLMERTPYIARHKGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPPGSVQLKIPGCNDQTAEGYCPLSTFTRVVSQSVEPGCQLQ
```

**SEQ ID NO: 4**

```
NDTPASGYQVEKVVILSRHGVRAPTKMTQTMRDVTPNTWPEWPVKLGYITPRGEHLISLMGGFYRQKFQQQGILSQGSCPTPNSI
YVWADVDQRTLKTGEAFLAGLAPQCGLTIHHQQNLEKADPLFHPVKAGTCSMDKTQVQQAVEKEAQTPIDNLNQHYIPFLALMNT
TLNFSTSAWCQKHSADKSCDLGLSMPSKLSIKDNGNKVALDGAIGLSSTLAEIFLLEYAQGMPQAAWGNIHSEQEWASLLKLHNV
QFDLMARTPYIARHNGTPLLQAISNALNPNATESKLPDISPDNKILFIAGHDTNIANIAGMLNMRWTLPGQPDNTPPGGALVFER
LADKSGKQYVSVSMVYQTLEQLRSQTPLSLNQPAGSVQLKIPGCNDQTAEGYCPLSTFTRVVSQSVEPGCQLQ
```

**SEQ ID NO: 5**
MTISAFNRKKLTLHPGLFVALSAIFSLGSTAYA

**SEQ ID NO:6**

```
MKAILIPFLSLLIPLTPQSAFAQSEPELKLESVVIVSRHGVRAPTKATQLMQDVTPDAWPTWPVKLGWLTPRGGELIAYLGHYQRQRLVADGLLAKK
GCPQSGQVAIIADVDERTRKTGEAFAAGLAPDCAITVHTQADTSSPDPLFNPLKTGVCQLDNANVTDAILSRAGGSIADFTGHRQTAFRELERVLNF
PQSNLCLKREKQDESCSLTQALPSELKVSADNVSLTGAVSLASMLTEIFLLQQAQGMPEPGWGRITDSHQWNTLLSLHNAQFYLLQRTPEVARSRAT
PLLDLIMAALTPHPPQKQAYGVTLPTSVLFIAGHDTNLANLGGALELNWTLPGQPDNTPPGGELVFERWRRLSDNSQWIQVSLVFQTLQQMRDKTPL
SLNTPPGEVKLTLAGCEERNAQGMCSLAGFTQIVNEARIPACSLRSHHHHHH
```

## Claims

1. A method of feeding an animal with a feed, wherein said feed comprises a phytase, wherein said phytase results in an improvement in one or more of said animal's biophysical characteristics when compared to the equivalent use of *Peniophora lycii* phytase and/or an *E. coli* phytase; wherein said improvement of said animal's biophysical characteristics comprises an increase in weight gain, wherein the increase in weight gain is at least 2% over a period of at least 21 days when the phytase is dosed at an amount of at least 500 FTU/kg feed, further wherein said phytase is or is obtainable from or is derivable from a *Buttiauxella* species.

2. A method according to claim 1, further wherein said improvement of said animal's biophysical characteristics is selected from a list consisting of:

    a) an increase in feed conversion ratio;
    b) an increase in bone density and/or bone strength and/or calcium deposition and/or phosphorus deposition;
    c) an increase in the retention of a mineral and/or a decrease in secretion of a mineral;
    d) an increase in retention and/or a decrease in secretion of any one or more of copper, sodium, phosphorous, nitrogen and calcium;
    e) an increase in amino acid retention;

f) an increase in growth.

3. A method according to claim 1, wherein said improvement in said animal's biophysical characteristics comprises an increase in feed conversion ratio when compared to the equivalent use of *Peniophora lycii* phytase and/or an *E. coli* phytase wherein the increase in feed conversion ratio is at least 5% over a period of at least 21 days when the phytase is dosed at an amount of at least 500 FTU/kg feed.

4. A method according to claim 1, wherein said improvement in said animal's biophysical characteristics comprises an increase in bone density and/or bone strength and/or calcium deposition and/or phosphorus deposition when compared to the equivalent use of *Peniophora lycii* phytase and/or an *E. coli* phytase, wherein the increase in bone density and/or bone strength and/or calcium deposition and/or phosphorus deposition is at least 10% over a period of at least 21 days when the phytase is dosed at an amount of at least 250 FTU/kg feed .

5. A method according to claim 1, wherein said improvement in said animal's biophysical characteristics comprises an increase in the retention of a mineral and/or a decrease in secretion of a mineral when compared to the equivalent use of *Peniophora lycii* phytase and/or an *E. coli* phytase wherein the increase in the retention of a mineral and/or a decrease in secretion of a mineral is at least 10% over a period of at least 14 days when the phytase is dosed at an amount of at least 250 FTU/kg feed .

6. A method according to claim 1, wherein said improvement in said animal's biophysical characteristics comprises an increase in retention and/or a decrease in secretion of any one or more of copper, sodium, phosphorous, nitrogen and calcium when compared to the equivalent use of *Peniophora lycii* phytase and/or an *E. coli* phytase wherein the increase in retention and/or a decrease in secretion of any one or more of copper, sodium, phosphorous, nitrogen and calcium is at least 10% over a period of at least four weeks when the phytase is dosed at an amount of at least 250 FTU/kg feed.

7. A method according to claim 1, wherein said improvement in said animal's biophysical characteristics comprises an increase in amino acid retention when compared to the equivalent use of *Peniophora lycii* phytase and/or an *E. coli* phytase wherein the increase in amino acid retention is on average at least 10% over a period of at least 21 days when the phytase is dosed at an amount of at least 500 FTU/kg feed.

8. A method according to claim 1, wherein said improvement in said animal's biophysical characteristics comprises an increase in growth when compared to the equivalent use of *Peniophora lycii* phytase and/or an *E. coli* phytase wherein the increase in growth is at least 20% over a period of at least 28 days when the phytase is dosed at an amount of at least 250 FTU/kg feed .

9. A method of any preceding claim wherein said animal is selected from the following:-a monogastric farm animal; a monogastric animal; a bird; a poultry bird; a chicken; a duck; a turkey; a pig; a piglet; a swine; a hog; a grower-finisher; a sow; a non-monogastric animal; a ruminant animal; a non-monogastric farm animal; a beef producing animal; a dairy producing animal; an Alpaca; a Bison; a Bovine animal; a Camel; an animal of the Cattle family; a Cow; a Deer; a Donkey; an Equus animal; a Goat; a Horse; a Lamb; any animal considered to be Livestock; a Llama; a Mule; an Ox; a Reindeer; a Sheep; a Steer; a Yak; a Buffalo; a Giraffe; a Moose; a Elk; a Llama; an Antelope; a Pronghorn; a Nilgai; an equine, bovine, cervidae, caprinae, camelidae animal; a fish; a gastric fish; an agastric fish; a shrimp or other crustacean; a marine fish or a freshwater fish, preferably wherein said animal is a domesticated animal.

10. A method according to any one of claims 1-9 wherein said phytase has at least 75, 80, 85, 90, or 95% identity to BP17 as shown in SEQ ID NO:1, preferably wherein said phytase is BP17 as shown in SEQ ID NO:1.

11. A method according to any one of claims 1-10 in which said phytase is low pH tolerant.

12. Use of a phytase in a method of feeding an animal with a feed, wherein said phytase results in an improvement in one or more of said animal's biophysical characteristics when compared to the equivalent use of *Peniophora lycii* phytase and/or an *E. coli* phytase, wherein said phytase is BP17 phytase of SEQ ID NO:1 or a phytase having at least 75%, 80%, 85%, 90% or 95% identity thereto; wherein said improvement of said animal's biophysical characteristics comprises an increase in weight gain, wherein the increase in weight gain is at least 2% over a period of at least 21 days when the phytase is dosed at an amount of at least 500 FTU/kg feed.

13. Use according to claim 12, further wherein said improvement of said animal's biophysical characteristics is selected from a list consisting of:

> a) an increase in feed conversion ratio;
> b) an increase in bone density and/or bone strength and/or calcium deposition and/or phosphorus deposition;
> c) an increase in the retention of a mineral and/or a decrease in secretion of a mineral;
> d) an increase in retention and/or a decrease in secretion of any one or more of copper, sodium, phosphorous, nitrogen and calcium;
> e) an increase in amino acid retention;
> f) an increase in growth;

**Patentansprüche**

1. Verfahren zum Füttern eines Tieres mit einem Futtermittel, wobei das Futtermittel eine Phytase umfasst, wobei die Phytase zu einer Verbesserung einer oder mehrerer biophysikalischer Eigenschaften des Tieres führt, verglichen mit der äquivalenten Verwendung von *Peniophora lycii*-Phytase und/oder einer E. coli-Phytase; wobei die Verbesserung der biophysikalischen Eigenschaften des Tieres eine Erhöhung der Massezunahme umfasst, wobei die Erhöhung der Massezunahme über eine Zeitdauer von mindestens 21 Tagen mindestens 2% beträgt, wenn die Phytase mit einer Menge von mindestens 500 FTU/kg Futtermittel dosiert wird, wobei die Phytase ferner eine *Buttiauxella*-Spezies ist, oder von ihr erhalten oder von ihr deriviert werden kann.

2. Verfahren nach Anspruch 1, wobei die Verbesserung der biophysikalischen Eigenschaften des Tieres ferner ausgewählt ist aus einer Liste bestehend aus:

> a) einer Zunahme der Futterverwertungsrate;
> b) einer Zunahme der Knochendichte und/oder Knochenfestigkeit und/oder Calcium-Ausscheidung und/oder Phosphor-Ausscheidung;
> c) einer Zunahme in der Rückhaltung eines Minerals und/oder einer Verringerung der Sekretion eines Minerals;
> d) einer Zunahme in der Rückhaltung und/oder einer Verringerung der Sekretion eines oder mehrerer von Kupfer, Natrium, Phosphor, Stickstoff und Calcium;
> e) einer Zunahme in der Aminosäure-Retention;
> f) einer Zunahme des Wachstums.

3. Verfahren nach Anspruch 1, wobei die Verbesserung der biophysikalischen Eigenschaften des Tieres eine Zunahme der Futterverwertungsrate im Vergleich zu der äquivalenten Verwendung von *Peniophora lycii*-Phytase und/oder einer *E. coli*-Phytase umfasst, wobei die Zunahme der Futterverwertungsrate über eine Zeitdauer von mindestens 21 Tagen mindestens 5% beträgt, wenn die Phytase mit mindestens 500 FTU/kg Futtermittel dosiert wird.

4. Verfahren nach Anspruch 1, wobei die Verbesserung der biophysikalischen Eigenschaften des Tieres eine Zunahme der Knochendichte und/oder Knochenfestigkeit und/oder Calcium-Ausscheidung und/oder Phosphor-Ausscheidung im Vergleich zu der äquivalenten Verwendung von *Peniophora lycii*-Phytase und/oder einer *E. coli*-Phytase umfasst, wobei die Zunahme der Knochendichte und/oder Knochenfestigkeit und/oder Calcium-Ausscheidung und/oder Phosphor-Ausscheidung über eine Zeitdauer von mindestens 21 Tagen mindestens 10% beträgt, wenn die Phytase in einer Menge von mindestens 250 FTU/kg Futtermittel dosiert wird.

5. Verfahren nach Anspruch 1, wobei die Verbesserung der biophysikalischen Eigenschaften des Tieres eine Zunahme in der Rückhaltung eines Minerals und/oder einer Verringerung der Sekretion eines Minerals im Vergleich zu der äquivalenten Verwendung von *Peniophora lycii*-Phytase und/oder einer *E. coli*-Phytase umfasst, wobei die Zunahme in der Rückhaltung eines Minerals und/oder einer Verringerung der Sekretion eines Minerals über eine Zeitdauer von mindestens 14 Tagen mindestens 10% beträgt, wenn die Phytase in einer Menge von mindestens 250 FTU/kg Futtermittel dosiert wird.

6. Verfahren nach Anspruch 1, wobei die Verbesserung der biophysikalischen Eigenschaften des Tieres eine Zunahme in der Rückhaltung und/oder einer Verringerung der Sekretion eines oder mehrerer von Kupfer, Natrium, Phosphor, Stickstoff und Calcium im Vergleich zu der äquivalenten Verwendung von *Peniophora lycii*-Phytase und/oder einer *E. coli*-Phytase umfasst, wobei die Zunahme in der Rückhaltung und/oder einer Verringerung der Sekretion eines oder mehrerer von Kupfer, Natrium, Phosphor, Stickstoff und Calcium über eine Zeitdauer von mindestens 4 Wochen mindestens 10% beträgt, wenn die Phytase in einer Menge von mindestens 250 FTU/kg Futtermittel dosiert wird.

7. Verfahren nach Anspruch 1, wobei die Verbesserung der biophysikalischen Eigenschaften des Tieres eine Zunahme der Aminosäure-Retention im Vergleich zu der äquivalenten Verwendung von *Peniophora lycii*-Phytase und/oder einer *E. coli*-Phytase umfasst, wobei die Zunahme der Aminosäure-Retention über eine Zeitdauer von mindestens 21 Tagen mindestens 10% beträgt, wenn die Phytase in einer Menge von mindestens 500 FTU/kg Futtermittel dosiert wird.

8. Verfahren nach Anspruch 1, wobei die Verbesserung der biophysikalischen Eigenschaften des Tieres eine Zunahme des Wachstums im Vergleich zu der äquivalenten Verwendung von *Peniophora lycii*-Phytase und/oder einer *E. coli*-Phytase umfasst, wobei die Zunahme des Wachstums über eine Zeitdauer von mindestens 28 Tagen mindestens 20% beträgt, wenn die Phytase in einer Menge von mindestens 250 FTU/kg Futtermittel dosiert wird.

9. Verfahren nach einem der vorhergehenden Ansprüche, wobei das Tier aus den Folgenden ausgewählt ist: einem monogastrischen Nutztier; einem monogastrischen Tier; einem Vogel; einem Geflügelvogel; einem Huhn; einer Ente; einem Truthahn; einem Schwein; einem Ferkel; einem Borstenvieh; einem Hausschwein, einem Mastschwein; einem Mutterschwein, einem nicht monogastrischen Tier; einem Wiederkäuer-Tier; einem nicht monogastrischen Nutztier; einem Rindfleisch produzierenden Tier; einem milchproduzierenden Tier; einem Alpaka; einem Bison; einem Hornvieh; einem Kamel; einem Tier aus der Rinderfamilie; einer Kuh; einem Reh; einem Esel; einem Pferdetier; einer Ziege; einem Pferd; einem Lamm; jedem Tier, das als Nutzviehbestand zu betrachten ist; einem Lama; einem Maultier; einem Ochsen; einem Rentier; einem Schaf; einem Mastochsen; einem Yak; einem Büffel; einer Giraffe; einem Alaskaelch; einem Elch; einem Lama; einer Antilope; einem Gabelbock; einer Nilgauantilope; einem Pferd-, Rind-, Cervidae-, Caprinae-, Camelidae-Tier; einem Fisch; einem gastrischen Fisch; einem agastrischen Fisch; einer Garnele oder anderen Krebstieren; einem Meeresfisch oder einem Süßwasserfisch, wobei das Tier bevorzugt ein domestiziertes Tier ist.

10. Verfahren nach einem der Ansprüche 1 bis 9, wobei die Phytase mindestens 75, 80, 85, 90 oder 95% Identität zu BP17 aufweist, wie in SEQ ID NO: 1 gezeigt ist, bevorzugt wobei die Phytase BP17 ist, wie in SEQ ID NO: 1 gezeigt ist.

11. Verfahren nach einem der Ansprüche 1 bis 10, wobei die Phytase geringe pH-Wert-Toleranz aufweist.

12. Verwendung einer Phytase in einem Verfahren zum Füttern eines Tieres mit einem Futtermittel, wobei die Phytase Verbesserung einer oder mehrerer biophysikalischer Eigenschaften des Tieres führt, verglichen mit der äquivalenten Verwendung von *Peniophora lycii*-Phytase und/oder einer E. coli-Phytase; wobei die Phytase BP17-Phytase von SEQ ID No: 1 oder eine Phytase ist, die mindestens 75, 80, 85, 90 oder 95% Identität zu ihr aufweist; wobei die Verbesserung der biophysikalischen Eigenschaften des Tieres eine Erhöhung der Massezunahme umfasst, wobei die Erhöhung der Massezunahme über eine Zeitdauer von mindestens 21 Tagen mindestens 2% beträgt, wenn die Phytase mit einer Menge von mindestens 500 FTU/kg dosiert wird.

13. Verwendung nach Anspruch 12, ferner wobei die Verbesserung der biophysikalischen Eigenschaften des Tieres ferner ausgewählt ist aus einer Liste bestehend aus:

> a) einer Zunahme der Futterverwertungsrate;
> b) einer Zunahme der Knochendichte und/oder Knochenfestigkeit und/oder Calcium-Ausscheidung und/oder Phosphor-Ausscheidung;
> c) einer Zunahme in der Rückhaltung eines Minerals und/oder einer Verringerung der Sekretion eines Minerals;
> d) einer Zunahme in der Rückhaltung und/oder einer Verringerung der Sekretion eines oder mehrerer von Kupfer, Natrium, Phosphor, Stickstoff und Calcium;
> e) einer Zunahme in der Aminosäure-Retention;
> f) einer Zunahme des Wachstums.

**Revendications**

1. Mode d'alimentation d'un animal avec un aliment, dans lequel ledit aliment comprend une phytase, ladite phytase conduisant à une amélioration d'une ou plusieurs des caractéristiques biophysiques dudit animal par rapport à une utilisation équivalente d'une phytase de *Peniophora lycii* et/ou d'une phytase de *E. coli;* dans lequel ladite amélioration des caractéristiques biophysiques dudit animal comprend une augmentation du gain de poids, laquelle augmentation du gain de poids est d'au moins 2% sur une période d'au moins 21 jours lorsque la phytase est administrée en une quantité d'au moins 500 FTU/kg d'aliment, en outre dans lequel ladite phytase est ou est susceptible d'être obtenue ou est dérivable d'une espèce de *Buttiauxella*.

2. Procédé selon la revendication 1, dans lequel en outre ladite amélioration des caractéristiques biophysiques dudit animal est choisie dans une liste constituée par:

> a) une augmentation du taux de conversion alimentaire;
> b) une augmentation de densité osseuse et/ou de résistance osseuse et/ou de dépôt de calcium et/ou de dépôt de phosphore;
> c) une augmentation de rétention d'un minéral et/ou une diminution de sécrétion d'un minéral;
> d) une augmentation de rétention et/ou une diminution de sécrétion de l'un quelconque ou plusieurs parmi le cuivre, le sodium, le phosphore, l'azote et le calcium;
> e) une augmentation de rétention d'acides aminés;
> f) une augmentation de la croissance.

3. Procédé selon la revendication 1, dans lequel ladite amélioration des caractéristiques biophysiques dudit animal comprend une augmentation du taux de conversion alimentaire par rapport à l'utilisation équivalente d'une phytase de *Peniophora lycii* et/ou d'une phytase de *E. coli*, laquelle augmentation du taux de conversion alimentaire est d'au moins 5% sur une période d'au moins 21 jours lorsque la phytase est administrée en une quantité d'au moins 500 FTU/kg d'aliment.

4. Procédé selon la revendication 1, dans lequel ladite amélioration des caractéristiques biophysiques dudit animal comprend une augmentation de densité osseuse et/ou de résistance osseuse et/ou de dépôt de calcium et/ou de dépôt de phosphore par rapport à l'utilisation équivalente d'une phytase de *Peniophora lycii* et/ou d'une phytase de *E. coli*, laquelle augmentation de densité osseuse et/ou de résistance osseuse et/ou de dépôt de calcium et/ou de dépôt de phosphore est d'au moins 10% sur une période d'au moins 21 jours lorsque la phytase est administrée en une quantité d'au moins 250 FTU/kg d'aliment.

5. Procédé selon la revendication 1, dans lequel ladite amélioration des caractéristiques biophysiques dudit animal comprend une augmentation de rétention d'un minéral et/ou une diminution de sécrétion d'un minéral par rapport à l'utilisation équivalente d'une phytase de *Peniophora lycii* et/ou d'une phytase de *E. coli*, laquelle augmentation de rétention d'un minéral et/ou diminution de sécrétion d'un minéral est d'au moins 10% sur une période d'au moins 14 jours lorsque la phytase est administrée en une quantité d'au moins 250 FTU/kg d'aliment.

6. Procédé selon la revendication 1, dans lequel ladite amélioration des caractéristiques biophysiques dudit animal comprend une augmentation de rétention et/ou une diminution de sécrétion de l'un quelconque ou plusieurs parmi le cuivre, le sodium, le phosphore, l'azote et le calcium par rapport à l'utilisation équivalente d'une phytase de *Peniophora lycii* et/ou d'une phytase de *E. coli*, laquelle augmentation de rétention et/ou diminution de sécrétion de l'un quelconque ou plusieurs parmi le cuivre, le sodium, le phosphore, l'azote et le calcium est d'au moins 10% sur une période d'au moins 4 semaines lorsque la phytase est administrée en une quantité d'au moins 250 FTU/kg d'aliment.

7. Procédé selon la revendication 1, dans lequel ladite amélioration des caractéristiques biophysiques dudit animal comprend une augmentation de rétention d'acides aminés par rapport à l'utilisation équivalente d'une phytase de *Peniophora lycii* et/ou d'une phytase de *E. coli*, laquelle augmentation de rétention d'acides aminés est en moyenne d'au moins 10% sur une période d'au moins 21 jours lorsque la phytase est administrée en une quantité d'au moins 500 FTU/kg d'aliment.

8. Procédé selon la revendication 1, dans lequel ladite amélioration des caractéristiques biophysiques dudit animal comprend une augmentation de la croissance par rapport à l'utilisation équivalente d'une phytase de *Peniophora lycii* et/ou d'une phytase de *E. coli*, laquelle augmentation de croissance est d'au moins 20% sur une période d'au moins 28 jours lorsque la phytase est administrée en une quantité d'au moins 250 FTU/kg d'aliment.

9. Procédé selon l'une quelconque des revendications précédentes, dans lequel ledit animal est choisi parmi les suivants: un animal de ferme monogastrique; un animal monogastrique; un oiseau; une volaille; un poulet; un canard; une dinde; un cochon; un porcelet; un porc; un porcin; un porc d'engraissement; une truie; un animal non monogastrique; un animal ruminant; un animal de ferme non monogastrique; un animal producteur de boeuf; un animal laitier; un alpaca; un bison; un animal bovin; un chameau; un animal de la famille du bétail; une vache; un cerf; un âne; un animal Equus; une chèvre; un cheval; un agneau; tout animal considéré comme étant cheptel; un lama; une mule; un boeuf; un renne; un mouton; un bouvillon; un yack; un buffle; une girafe; un élan; un wapiti; un lama; une antilope; une antilocapre; une antilope Nilgaut; un animal équin, bovin, cervidé, caprin, camélidé; un poisson; un poisson gastrique; un poisson agastrique; une crevette ou autre crustacé; un poisson marin ou un poisson d'eau douce, de préférence dans lequel ledit animal est un animal domestiqué.

10. Procédé selon l'une quelconque des revendications 1 à 9, dans lequel ladite phytase a au moins 75, 80, 85, 90 ou 95% d'identité avec BP17 comme le montre la SEQ ID NO:1, de préférence dans lequel ladite phytase est BP17 comme le montre la SEQ ID NO:1.

11. Procédé selon l'une quelconque des revendications 1 à 10, dans lequel ladite phytase tolère un pH bas.

12. Utilisation d'une phytase dans un procédé d'alimentation d'un animal avec un aliment, dans laquelle ladite phytase conduit à une amélioration d'une ou plusieurs des caractéristiques biophysiques dudit animal par rapport à l'utilisation équivalente d'une phytase de *Peniophora lycii* et/ou d'une phytase de *E. coli*, dans laquelle ladite phytase est la phytase BP17 de SEQ ID NO:1 ou une phytase ayant au moins 75%, 80%, 85%, 90% ou 95% d'identité avec celle-ci; dans laquelle ladite amélioration des caractéristiques biophysiques dudit animal comprend une augmentation du gain de poids, laquelle augmentation du gain de poids est d'au moins 2% sur une période d'au moins 21 jours lorsque la phytase est administrée en une quantité d'au moins 500 FTU/kg d'aliment.

13. Utilisation selon la revendication 12, dans laquelle en outre ladite amélioration des caractéristiques physiques dudit animal est choisie dans une liste constituée par:

a) une augmentation du taux de conversion alimentaire;
b) une augmentation de densité osseuse et/ou de résistance osseuse et/ou de dépôt de calcium et/ou de dépôt de phosphore;
c) une augmentation de rétention d'un minéral et/ou une diminution de sécrétion d'un minéral;
d) une augmentation de rétention et/ou une diminution de sécrétion de l'un quelconque ou plusieurs parmi le cuivre, le sodium, le phosphore, l'azote et le calcium;
e) une augmentation de rétention d'acides aminés;
f) une augmentation de la croissance.

Figure 1

**Amino acid digestibility improvement (% of Control)**

Lysine    Methionine    Cystine    Threonine    All amino acids

■ BP17 500 FTU   ■ BP17 1,000 FTU   Phytase R 1,850 FTU
(left bar)    (middle bar)    (right bar)

˙Values are significantly different to the NC (P<0.05)

**Figure 2**

*abc* *Values without a common superscript are significantly different (P<0.05)*

P digestibility (% of intake)
+10%

AME (kcal/kg DM)

Ca digestibilty (% of intake)
+9%

Phytate degradation (%)

Figure 3

**Calorie Conversion**
**(kcal/kg bodyweight gain, 5 -21 days)**

**Figure 4**

$^{(abc)}$ *Values without a common superscript are significantly different (P<0.05)*

## Fecal Na

70.49a

63.18b

56.09c

62.83b

61.94b

| | | | | |
|---|---|---|---|---|
| PC | NC | NC + 250 FTU BP17 | NC + 1000 FTU BP17 | NC + 2000 FTU BP17 |

## Fecal Cu

48.31(a)

47.07(ab)

44.29(ab)

44.68(ab)

43.39(b)

| | | | | |
|---|---|---|---|---|
| PC | NC | NC + 250 FTU BP17 | NC + 1000 FTU BP17 | NC + 2000 FTU BP17 |

## Fecal Zn

Figure 5

$^{abc}$ Values without a common superscript are significantly different (P<0.05)

**Figure 6**

# Bone Ash

[abc] *Values without a common superscript are significantly different (P<0.05)*

Figure 7

**Feed Intake (g, 7 -29 days)**

Phytase dose (FTU/kg feed)

● BP17    ○ E.Coli Phytase    ▬▬BP17    ▬▬E.Coli Phytase

**BWG (g, 7 -29 days)**

Phytase dose (FTU/kg feed)

● BP17    ● E.Coli Phytase    ▬▬BP17    ▬▬E.Coli Phytase

**FCR**

$y = 4E\,{-}08x^2 - 0.0001x + 1.38$

$y = 7E\,{-}08x^2 - 0.0002x + 1.38$

Phytase dose (FTU/kg feed)

● BP17    ○ E.Coli Phytase    ▬▬BP17    ▬▬E.Coli Phytase

**Calorie Conversion (kcal/kg bodyweight gain)**

$y = 0.0001x^2 - 0.3435x + 4018$

$y = 0.0002x^2 - 0.5012x + 4018$

Phytase dose (FTU/kg feed)

● BP17    ○ E.Coli Phytase    ▬▬BP17    ▬▬E.Coli Phytase

Figure 8

**P Digestibility (% of intake)**

$y = -1E-05x^2 + 0.0346x + 47.8$

$y = -6E-06x^2 + 0.0215x + 47.8$

positive controle

Phytase dose (FTU/T Feed)

● BP17　○ E.Coli Phytase　——BP17　—— E.Coli Phytase

**Ca Digestibility (% of intake)**

$y = -1E-05x^2 + 0.0337x + 40.89$

$y = -5E-06x^2 + 0.02x + 40.9$

positive controle

Phytase dose (FTU/kg feed)

● BP17　○ E.Coli Phytase　——BP17　—— E.Coll Phytase

**Tibia Ash (% of DM)**

$y = -6E-06x^2 + 0.0166x + 33.5$

$y = 0.0056x + 33.5$

positive controlb

Phytase dose (FTU/kg Feed)

● BP17　○ E.Coli Phytase　——BP17　—— E.Coli Phytase

**Tibia P (g/kg of DM)**

$y = -1E-05x^2 + 0.0325x + 58.8$

$y = 0.0108x + 58.8$

positive controlb

Phytase dose (FTU/kg Feed)

● BP17　○ E.Coli Phytase　——BP17　—— E.Coli Phytase

EP 2 800 476 B1

Figure 9

# Cu digestibility %

Figure 10

**AME**

**Excreta N Digestibility Coefficient**

**Excreta Ca Digestibility Coefficient**

**Excreta P Digestibility Coefficient**

Figure 11

**Excreta Na Digestibility Coefficient**

0.562    0.486    0.594    0.562

PC    NC    BP17 250FTU    BP17 2000FTU

**N Retention**

1.69a    1.62a    1.88b    1.77ab

PC    NC    BP17 250FTU    BP17 2000FTU

**P Retention**

0.20b    0.12a    0.18b    0.21b

PC    NC    BP17 250FTU    BP17 2000FTU

**Ca Retention**

2.09a    2.14a    3.24b    2.40a

PC    NC    BP17 250FTU    BP17 2000FTU

Figure 12

**Laying rate (%, week 23)**

**Laying rate (%, week 24-26)**

**Laying rate difference (%, week 24-26)**

**Egg Weight (%, week 23-26)**

Figure 13

## Feed Intake (%, week 23-26)

NC  PC  BP17 250FTU  BP17 500FTU  BP17 1000FTU  BP17 2000FTU

## FCR (%, week 23-26)

NC  PC  BP17 250FTU  BP17 500FTU  BP17 1000FTU  BP17 2000FTU

Figure 14

BP17 phytase

**Digestible energy (kcals)**

- 3766 — NC
- 3844 — BP17 PHY 250 FTU/kg
- 3867 — BP17 PHY 500 FTU/kg
- 3874 — BP17 PHY 1000 FTU/kg
- 3919 — BP17 PHY 2000 FTU/kg

## Figure 15

Results: Ileal digestibility of broilers (20 days).

**Ileal Digestible Phosphorus (g/kg diet)**

♦ E-Coli phytase  ■ BP17  —— NC + 0.6 g P*
—— NC + 1.2 g P*  — — NC + 1.8 g P*

**Ileal Digestible Protein (g/kg diet)**

♦ E-Coli phytase  ■ BP17  —— NC + 0.6 g P*
—— NC + 1.2 g P*  — — NC + 1.8 g P*

**Ileal Digestible Amino Acid (g/kg diet)**

♦ E-Coli phytase  ■ BP17  —— NC + 0.6 g P*
—— NC + 1.2 g P*  — — NC + 1.8 g P*

**Ileal Digestible Methionine (g/kg diet)**

♦ E-Coli phytase  ■ BP17  —— NC + 0.6 g P*
—— NC + 1.2 g P*  — — NC + 1.8 g P*

[a,b,c] Values without a common superscript are significantly different (P<0.05)
*Supplied from MCP

Results: Ileal digestible lysine, total tract digestible Ca and P retention of broilers (day 20)

**Ileal Digestible Lysine (g/kg diet)**

Phytase dose (FTU/kg feed)

♦ E-Coli phytase ■ BP17 ⋯⋯ NC + 0.6 g P*

⸺ NC + 1.2 g P* ⸻ NC + 1.8 g P*

**P retention (g/kg diet)**

Phytase dose (FTU/kg)

♦ E-Coli phytase ■ BP17 ⋯⋯ NC + 0.6 g P*

⸺ NC + 1.2 g P* ⸻ NC + 1.8 g P*

**Digestible Ca (g/kg diet)**

Phytase dose (FTU/kg)

♦ E-Coli phytase ■ BP17 ⋯⋯ NC + 0.6 g P*

⸺ NC + 1.2 g P* ⸻ NC + 1.8 g P* ⸻ Series6

[a,b,c] Values without a common superscript are significantly different (P<0.05)

*supplied from MCP

EP 2 800 476 B1

**Figure 16**

Results: Performance, AMEn and Tibia Ash of broiler chicks

Bodyweight gain (5 -20 days, g)

FCR (5-20 days)

AMEn (kcals/kg DM)

Tibia Ash (g/kg DM)

[a,b,c]Values without a common superscript are significantly different (P<0.05)
*Supplied from MCP, DM = dry matter

Figure 17

Results: Performance, Retained Phosphorus and Tibia Ash of broiler chicks

a,b,c Values without a common superscript are significantly different (P<0.05)
*Supplied from MCP

124

Figure 18

Results: Performance and Tibia Ash of broiler chicks (5-20 days)

**Bodyweight gain (5 –20 days, g)**

Phytase dose (FTU/kg feed)

♦ Phyzyme XP   ■ BP17   ⋯⋯ NC + 0.6 g P*
⋯ NC + 1.2 g P*   ⎯ ⎯ NC + 1.8 g P*

**FCR (520 days)**

Phytase dose (FTU/kg feed)

♦ Phyzyme XP   ■ BP17   ⋯⋯ NC + 0.6 g P*
⋯ NC + 1.2 g P*   ⎯ ⎯ NC + 1.8 g P*

**Tibia Ash (g/kg DM)**

Phytase dose (FTU/kg feed)

♦ Phyzyme XP   ■ BP17   ⋯⋯ NC + 0.6 g P*
⋯ NC + 1.2 g P*   ⎯ ⎯ NC + 1.8 g P*

a,b,c Values without a common superscript are significantly different ($P<0.05$)
*Supplied from MCP

Figure 19

Effect of different phytases on P digestibility (%)

$y = -5E\text{-}06x^2 + 0,0198x + 69,627$

$y = -1E\text{-}06x^2 + 0,01x + 68,951$

$y = -6E\text{-}07x^2 + 0,0052x + 69,81$

◆ Buttiauxella Phytase    ■ E.coli Phytase1    ▲ P.Lycii phytase

EP 2 800 476 B1

Figure 20

Water quality criteria throughout Example 21

EP 2 800 476 B1

**Figure 21**

Determination of the pH Optima of BP17 phytase compared to 3 different commercial phytase sources.

Comparison pH activity profiles
(37°C for 30min. - 5.1mM Na-phytate and 0.02 FTU/ml)

Figure 22

Activity of BP17 phytase at different pH, as measured by the amount of phosphorus released from Na-phytate over varying pH.

## Comparison of Phosphorus release at different pH (37°C for 30min. - 5.1mM Na-phytate and 0.02 FTU/ml)

Legend:
- BP17 phytase
- C. braachi Phytase
- E. coli phytase
- P. lycii phytase

EP 2 800 476 B1

Figure 23

Relative activity of BP17 phytase at releasing phosphorus from phytate at different pH, with activity of each phytase at pH 5.5 set at 100%.

Relative Activity to pH 5.5 of different Phytases at releasing phosphorus (37°C for 30min. - 5.1mM Na-phytate and 0.02 FTU/ml)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010122532 A
- EP 0619369 A
- US 5554399 A
- WO 2004071218 A
- WO 2006043178 A
- WO 2008097619 A
- WO 2009129489 A
- WO 2006038128 A
- WO 2008092901 A
- US 200941011 W
- WO 2010051804 A
- WO 06043178 A
- WO 2006038062 A
- WO 2006037328 A
- WO 2007112739 A
- WO 2006037327 A
- US 2008263688 A

- WO 2006034710 A
- WO 0001793 A
- WO 9932595 A
- WO 2007044968 A
- WO 0047060 A
- WO 03059086 A
- WO 03059087 A
- WO 2006053564 A
- US 20030054511 A
- GB 1200132 A
- US 61596944 B
- GB 1203868 A
- GB 1211170 A
- GB 1211168 A
- GB 1211167 A
- GB 1211169 A
- GB 1211166 A

**Non-patent literature cited in the description**

- **ZHOU, J. R. ; ERDMAN, J. W.** *Critical Reviews in Food Science and Nutrition.,* 1995, vol. 35 (6), 495-508
- **ZHOU ; ERDMAN ; SEBASTIAN, S. et al.** *World's Poultry Science Journal.,* 1998, vol. 54, 27-47
- **KNUCKLES, B. E.** Effect of phytate and partially hydrolyzed phytate on in vitro protein digestibility. *J. Food Sci.,* 1985, vol. 50, 1080-1082
- Phytic acid and nutritional impact. **KONIETZNY, U. ; GREINER, R.** In Encyclopedia of Food Sciences and Nutrition. Elsevier Science, 2003, vol. 7, 4546-4563
- **KIES, A. K. ; DE JONGE, L. H. ; KEMME, P. A. ; JONGBLOED, A. W.** Interaction between protein, phytate, and microbial phytase. *In vitro studies. J. Agric. Food Chem.,* 2006, vol. 54, 1753-1758
- **VAINTRAUB, I. A. ; BULMAGA, V. P.** Effect of phytate on the in vitro activity of digestive proteinases. *J. Agric. Food Chem.,* 1991, vol. 39, 859-861
- **CARNOVALE, E. ; LUGARO, E. ; LOMBARDI-BOCCIA, G.** Phytic acid in Faba bean and pea: effect on protein availability. *Cereal Chem.,* 1988, vol. 65, 114-117
- **A. J. COWIESON ; V. RAVINDRANAND ; P. H. SELLE.** Influence of Dietary Phytic Acid and Source of Microbial Phytase on Ileal Endogenous Amino Acid Flows in Broiler Chickens. *Poult Sci.,* 2008, vol. 87, 2287-2299

- **RAVINDRAN et al.** *J. Poult. Sci.,* 1999, vol. 78, 699-706
- **OH, B. C. et al.** *Appl. Microbiol Biotechnol,* 2004, vol. 63, 362-372
- **PALLAUF, J. ; RIMBACH, G.** *Arch. Anim. Nutr.,* 1997, vol. 50, 301-319
- Pelleting Cost Center. **FAIRFIELD, D.** In Feed Manufacturing Technology IV. American Feed Industry Association, 1994, vol. IV, 110-139
- **SAMBROOK et al.** Molecular Cloning: A Laboratory Manual. 1989
- Oligonucleotide Synthesis. 1984
- Current Protocols in Molecular Biology. 1994
- PCR: The Polymerase Chain Reaction. 1994
- **KRIEGLER.** Gene Transfer and Expression: A Laboratory Manual. 1990
- **SINGLETON et al.** Dictionary of Microbiology and Molecular Biology. John Wiley and Sons, 1994
- **HALE ; MARKHAM.** The Harper Collins Dictionary of Biology. Harper Perennial, 1991
- **AUSUBEL et al.** Short Protocols in Molecular Biology. 1999
- *FEMS Microbiol Lett,* 1999, vol. 174 (2), 247-50
- *FEMS Microbiol Lett,* 1999, vol. 177 (1), 187-8, tatiana@ncbi.nlm.nih.gov
- **ALTSCHUL et al.** *J. Mol. Biol.,* 1990, 403-410

- **HIGGINS DG ; SHARP PM.** *Gene,* 1988, vol. 73 (1), 237-244
- **DEVEREUX et al.** *Nuc. Acids Research,* 1984, vol. 12, 387
- **AUSUBEL et al.** *Short Protocols in Molecular Biology,* 1999, 7-58, 7-60
- *FEMS Microbiol Lett 1999,* vol. 174 (2), 247-50
- **SIMON RJ et al.** *PNAS,* 1992, vol. 89 (20), 9367-9371
- **HORWELL DC.** *Trends Biotechnol.,* 1995, vol. 13 (4), 132-134

- **DENBOW, L. M. ; V. RAVINDRAN ; E. T. KORNEGAY ; Z. YI ; R. M. HULET.** Improving phosphorus availability in soybean meal for broilers by supplemental phytase. *Poult. Sci.,* 1995, vol. 74, 1831-1842
- **SIBBALD, I.R.** *1980. BioScience,* November 1980, vol. 30 (11
- **BOLIN, D. W. ; R. P. KING ; E. W. KLOSTERMAN.** A simplified method for the determination of chromic oxide (Cr:O~) when used as an index substance. *Science,* 1952, vol. 116, 634